(19) Europäisches Patentamt
European Patent Office
Office européen des brevets

(11) EP 3 693 023 A1

(12) # EUROPEAN PATENT APPLICATION

(43) Date of publication:
**12.08.2020 Bulletin 2020/33**

(21) Application number: **19305173.7**

(22) Date of filing: **11.02.2019**

(51) Int Cl.:
***A61K 47/68*** *(2017.01)* ***A61P 35/00*** *(2006.01)*
***C07K 16/30*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Sanofi**
**75008 Paris (FR)**

(72) Inventor: **The designation of the inventor has not yet been filed**

(74) Representative: **Sanofi**
**54, rue La Boétie**
**75008 Paris (FR)**

(54) **USE OF ANTI-CEACAM5 IMMUNOCONJUGATES FOR TREATING LUNG CANCER**

(57) The present disclosure provides methods of treating high CEACAM5 expressing cancers including lung cancers such NSQ-NSCLC using immunoconjugates comprising an antibody that specifically binds human CEACAM5.

EP 3 693 023 A1

## Description

## FIELD

The present disclosure relates to the field of therapeutic treatment of cancers, such as non squamous non-small cell lung cancer (NSQ-NSCLC), which express CEACAM5. Certain aspects of the invention relate to the use of CEACAM5 antagonists, such as anti-CEACAM5 antibodies and immunoconjugates, to treat lung cancer.

## BACKGROUND

The mechanism of action of antibody drug conjugates (ADCs) begins with its binding to a specific antigen, sufficiently expressed on the tumor cells in order to achieve a selective and efficient internalization of the drug. Selectively targeting potent cytotoxics to tumor cells using ADCs has now been shown to be an effective strategy for the treatment of cancer, as demonstrated by the recent approvals of brentuximab vedotin for the treatment of Hodgkin lymphoma and trastuzumab emtansine (T-DM1) for the treatment of relapsed metastatic HER2+ breast cancer (Younes A, Gopal AK, Smith SE, Ansell SM, Rosenblatt JD, Savage KJ, et al. Results of a pivotal phase II study of brentuximab vedotin for patients with relapsed or refractory Hodgkin's lymphoma. J Clin Oncol. 2012;30(18):2183-9; Verma S, Miles D, Gianni L, Krop IE, Welslau M, Baselga J, et al. Trastuzumab emtansine for HER2-positive advanced breast cancer. N Engl J Med. 2012;19:1783-91). Many other malignant diseases with unmet medical needs, such as solid tumor cancers, could benefit from such therapeutic options.

Lung cancer, for example, is an aggressive form of cancer that is accounts for hundreds of thousands of deaths in the United States. Unfortunately, it tends to recur after initial treatment and become more resistant to subsequent treatment. While multiple treatments have been utilized for the treatment of individuals with lung cancer, more effective treatments are needed.

## SUMMARY

This disclosure provides, *inter alia,* methods for treating lung cancer (e.g., NSQ-NSCLC) in a subject in need thereof comprising administering an effective amount of an antibody or immunoconjugate (comprising the antibody) that specifically binds CEACAM5.

This disclosure provides, *inter alia,* antibodies and methods for treating a cancer that express CEACAM5 in a subject in need thereof comprising administering an effective amount of an antibody or immunoconjugate or that specifically binds CEACAM5. For example, the cancer expresses human carcinoembryonic antigen-related cell adhesion molecule 5 (hCEACAM5). In various embodiments, the cancer highly expresses hCEACAM5. For example, the cancer expresses the A3-B3 domain of hCEACAM5 comprising SEQ ID NOs: 10 and 11, such that the antibody or the immunoconjugate binds the domain.

The disclosure provides an antibody for use in treating high carcinoembryonic antigen-related cell adhesion molecule 5 cancer in a subject in need thereof. In various embodiments, the antibody specifically binds human carcinoembryonic antigen-related cell adhesion molecule 5 (hCEACAM5) wherein the antibody comprises a heavy chain variable region (VH) and a light chain variable region (VL), wherein the VH comprises the three complementarity determining regions HCDR1, HCDR2 and HCDR3 and wherein the VL comprises the three CDRs LCDR1, LCDR2 and LCDR3, wherein the HCDR1 comprises the amino acid sequence of SEQ ID NO: 3 (GFVFSSYD); the HCDR2 comprises the amino acid sequence of SEQ ID NO: 4 (ISSGGGIT); the HCDR3 comprises the amino acid sequence of SEQ ID NO: 5 (AAHYFGSSGPFAY); the LCDR1 comprises the amino acid sequence of SEQ ID NO: 6 (ENIFSY); the LCDR2 comprises the amino acid sequence of NTR; and the LCDR3 comprises the amino acid sequence of SEQ ID NO: 7 (QHHYGTPFT).

The disclosure provides an antibody for use in treating non squamous non-small cell lung cancer (NSQ-NSCLC) in a subject in need thereof, wherein the antibody specifically binds hCEACAM5 wherein the antibody comprises a VH and a VL, wherein the VH comprises the three complementarity determining regions HCDR1, HCDR2 and HCDR3 and wherein the VL comprises the three CDRs LCDR1, LCDR2 and LCDR3, wherein the HCDR1 comprises the amino acid sequence of SEQ ID NO:3; the HCDR2 comprises the amino acid sequence of SEQ ID NO: 4; the HCDR3 comprises the amino acid sequence of SEQ ID NO: 5; the LCDR1 comprises the amino acid sequence of SEQ ID NO: 6; the LCDR2 comprises the amino acid sequence of NTR; and the LCDR3 comprises the amino acid sequence of SEQ ID NO: 7.

The disclosure provides an antibody for use in treating a subject that has been pre-treated with a cancer therapeutic, wherein the antibody specifically binds hCEACAM5 wherein the antibody comprises a heavy chain variable region (VH) and a light chain variable region (VL), wherein the VH comprises the three complementarity determining regions HCDR1, HCDR2 and HCDR3 and wherein the VL comprises the three CDRs LCDR1, LCDR2 and LCDR3, wherein the HCDR1 comprises the amino acid sequence of SEQ ID NO: 3; the HCDR2 comprises the amino acid

sequence of SEQ ID NO: 4; the HCDR3 comprises the amino acid sequence of SEQ ID NO: 5; the LCDR1 comprises the amino acid sequence of SEQ ID NO: 6; the LCDR2 comprises the amino acid sequence of NTR; and the LCDR3 comprises the amino acid sequence of SEQ ID NO: 7. In certain embodiments, the subject is a high carcinoembryonic antigen-related cell adhesion molecule expresser. In other embodiments, the subject was pre-treated with an agent or drug for treatment of non-small cell lung cancer. In other embodiments, the agent or drug is selected from the group consisting of: a chemotherapy agent, an angiogenesis inhibitor, an epidermal growth factor receptor (EGFR) inhibitor, an anaplastic lymphoma kinase (ALK) inhibitor, a receptor tyrosine kinase (ROS1) inhibitor, and an immune checkpoint inhibitor. In certain aspects of these embodiments, the immune checkpoint inhibitor is a PD-1 inhibitor and/or a PD-L1 inhibitor.

In various embodiments, the cancer is NSQ-NSCLC.

In various embodiments, the VH comprises SEQ ID NO: 1

(EVQLQESGPGLVKPGGSLSLSCAAS**GFVFSSYD**MSWVRQTPERGLEWVAY**ISSGGGIT**
YAPSTVKGRFTVSRDNAKNTLYLQMNSLTSEDTAVYYC**AAHYFGSSGPFAY**WGQGTL
VTVSS).

In various embodiments, the heavy chain comprises SEQ ID NO: 8

(EVQLQESGPGLVKPGGSLSLSCAASGFVFSSYDMSWVRQTPERGLEWVAYISSGGGITY
APSTVKGRFTVSRDNAKNTLYLQMNSLTSEDTAVYYCAAHYFGSSGPFAYWGQGTLVT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLP
PSRDELTKNQVSLTCLVKGFYPSDIAVEWESNGQPENNYKTTPPVLDSDGSFFLYSKLTV
DKSRWQQGNVFSCSVMHEALHNHYTQKSLSLSPG).

In various embodiments, the VL comprises SEQ ID NO: 2

(DIQMTQSPASLSASVGDRVTITCRAS**ENIFSY**LAWYQQKPGKSPKLLVY**NTR**TLAEGVP
SRFSGSGSGTDFSLTISSLQPEDFATYYC**QHHYGTPFT**FGSGTKLEIK).

In various embodiments, the light chain comprises SEQ ID NO: 9

(DIQMTQSPASLSASVGDRVTITCRASENIFSYLAWYQQKPGKSPKLLVYNTRTLAEGVP
SRFSGSGSGTDFSLTISSLQPEDFATYYCQHHYGTPFTFGSGTKLEIKRTVAAPSVFIFPPS
DEQLKSGTASVVCLLNNFYPREAKVQWKVDNALQSGNSQESVTEQDSKDSTYSLSSTL
TLSKADYEKHKVYACEVTHQGLSSPVTKSFNRGEC).

In various embodiments, the antibody is conjugated or linked to at least one growth inhibitory agent. In an embodiment, the growth inhibitory agent is a cytotoxic agent. In various embodiments of the antibody, the growth inhibitory agent is selected from the group consisting of chemotherapeutic agents, enzymes, antibiotics, and toxins such as small

molecule toxins or enzymatically active toxins, taxoids, vincas, taxanes, maytansinoid or maytansinoid analogs, tomaymycin or pyrrolobenzodiazepine derivatives, cryptophycin derivatives, leptomycin derivatives, auristatin or dolastatin analogs, prodrugs, topoisomerase II inhibitors, DNA alkylating agents, anti-tubulin agents, and CC-1065 or CC-1065 analogs. In various embodiments of the antibody, the growth inhibitory agent is N2'-deacetyl-N2'-(3-mercapto-1-oxopropyl)-maytansine (DM1) or N2'-deacetyl-N-2'(4-methyl-4-mercapto-1-oxopentyl)-maytansine (DM4). For example, the growth inhibitory agent is DM4.

In various embodiments, the antibody is covalently attached via a cleavable or non-cleavable linker to the at least one growth inhibitory agent. In various embodiments of the antibody, the linker is selected from the group consisting ofN-succinimidyl pyridyldithiobutyrate (SPDB), 4-(Pyridin-2-yldisulfanyl)-2-sulfo-butyric acid (sulfo-SPDB), and succinimidyl (N-maleimidomethyl) cyclohexane-1-carboxylate (SMCC). For example, the linker is SPDB.

In various embodiments, wherein the antibody is huMAb2-3.

In various embodiments of the antibody, the subject has a hCEACAM5 expression of a percent score greater than or equal to 50 (consisting of 2+ and 3+ intensities) in the tumor cell populations, for example, the hCEACAM5 expression of a percent score greater than or equal to at least about 50, at least about 50 to about 80, at least about 80, or about 100 (consisting of 2+ and 3+ intensities). In various embodiments, the percentage of hCEACAM5 expression is at least about 50% to about 80% of the tumor cells populations, at least about 80%, at least about 90%, at least about 95%, or about 100% of the tumor cells populations. In various embodiments, the cancer is a non-squamous non-small cell lung carcinoma that highly expresses hCEACAM5 in at least about 50%, at least about 50% to about 80%, at least about 80%, at least about 90%, at least about 95%, or about 100% of the tumor cell population.

In various embodiments, the antibody is administered intravenously, for example by intravenous infusion.

In various embodiments, the antibody is administered at a rate of 2.5 mg/min for the first 30 minutes. In various embodiments, after about 30 minutes, the rate of administration of the antibody is increased to 5 mg/min.

In various embodiments, antibody is administered at a dose level of 5, 10, 20, 30, 40, 60, 80, 100, 120, 150, 180, or 210 mg/$m^2$ based on the body surface area of the subject. In various embodiments, the antibody is administered at a dose of about 2.5 mg/$m^2$ to about 5 mg/$m^2$. For example, the antibody is administered for one hour at a dose of about 2.5 mg/$m^2$ to about 5 mg/$m^2$. The dose includes 2.5 mg/$m^2$ of the antibody, 5 mg/$m^2$ of the antibody and all doses in between 2.5 mg/$m^2$ and 5 mg/$m^2$, for example 2.75, 3, 3.25, 3.5, 3.75, 4, 4.25, 4.5, and 4.75 mg/$m^2$. In various embodiments, the body surface area is calculated using the height and actual body of the subject.

In various embodiments, antibody is administered at every 14 days. In various embodiments, the antibody is administered every three weeks.

In various embodiments, wherein, prior to administering the antibody, the subject is administered a pre-medication. For example, the pre-medication is a histamine H1 antagonist. In various embodiments, the histamine H1 antagonist is diphenylhydramine or dexchlorpheniramine.

In various embodiments of the antibody, the subject was previously treated with an agent or drug for treatment of non-small cell lung cancer. For example, the subject was heavily pre-treated and/or ineffectively treated with the agent or drug. In various embodiments of the antibody, the agent or drug is selected from the group consisting of: a chemotherapy agent, an angiogenesis inhibitor, an epidermal growth factor receptor (EGFR) inhibitor, anaplastic lymphoma kinase (ALK) inhibitor, a receptor tyrosine kinase (ROS1) inhibitor, and an immune checkpoint inhibitor. In various embodiments of the antibody, the immune checkpoint inhibitor is a PD-1 inhibitor and/or a PD-L1 inhibitor.

In some embodiments of the antibody for use described above, progression of at least one symptom of cancer is reduced, slowed, halted, or otherwise ameliorated. In certain aspects of these embodiments, the tumor growth rate or tumor size is reduced after treatment with the antibody. In other aspects of these embodiments, the expression pattern, intensity and proportion of expressing cells indicates a reduction, slowing or halting of the cancer.

The disclosure provides a pharmaceutical composition comprising the antibody described herein and a pharmaceutically acceptable carrier.

The disclosure provides an immunoconjugate for use in treating high carcinoembryonic antigen-related cell adhesion molecule cancer in a subject in need thereof, wherein the immunoconjugate comprises an antibody drug conjugate (ADC) that specifically binds hCEACAM5 and comprises the antibody of any of the preceding claims, wherein progression of at least one symptom of cancer is reduced, slowed, halted, or otherwise ameliorated.

The disclosure provides an immunoconjugate for use in treating NSQ NSCLC in a subject in need thereof, wherein the immunoconjugate comprises an ADC that specifically binds hCEACAM5 and comprises the antibody of any of the preceding claims, wherein progression of at least one symptom of cancer is reduced, slowed, halted, or otherwise ameliorated.

The disclosure provides an immunoconjugate for use in treating a subject that is a heavily pre-treated high carcinoembryonic antigen-related cell adhesion molecule expresser, wherein the immunoconjugate comprises an ADC that specifically binds hCEACAM5 and comprises the antibody of any of the preceding claims, wherein progression of at least one symptom of cancer is reduced, slowed, halted, or otherwise ameliorated.

In various embodiments immunoconjugate comprises the antibody huMAb2-3.

wherein the growth inhibitory agent comprises DM4; and wherein the linker comprises SPDB. In various embodiments, the ADC comprises huMAb2-3-SPDB-DM4.

In various embodiments, the tumor growth rate or tumor size is reduced after treatment with the immunoconjugate.

In various embodiments expression pattern, intensity and proportion of expressing cells indicates a reduction, slowing or halting of the cancer.

The disclosure provides a pharmaceutical composition comprising the antibody described herein, or the immunoconjugate described, and a pharmaceutically acceptable carrier.

The disclosure provides a method for treating high carcinoembryonic antigen-related cell adhesion molecule 5 cancer in a subject in need thereof, the method comprising administering an antibody that specifically binds hCEACAM5 wherein the antibody comprises a VH and a VL, wherein the VH comprises the three complementarity determining regions HCDR1, HCDR2 and HCDR3) and wherein the VL comprises the three CDRs LCDR1, LCDR2 and LCDR3, wherein the HCDR1 comprises the amino acid sequence of SEQ ID NO: 3; the HCDR2 comprises the amino acid sequence of SEQ ID NO: 4; the HCDR3 comprises the amino acid sequence of SEQ ID NO: 5; the LCDR1 comprises the amino acid sequence of SEQ ID NO: 6; the LCDR2 comprises the amino acid sequence of NTR; and the LCDR3 comprises the amino acid sequence of SEQ ID NO: 7.

The disclosure provides a method for treating NSQ NSCLC in a subject in need thereof, the method comprising administering an antibody specifically binds hCEACAM5 wherein the antibody comprises a VH and a VL, wherein the VH comprises the three complementarity determining regions HCDR1, HCDR2 and HCDR3 and wherein the VL comprises the three CDRs LCDR1, LCDR2 and LCDR3, wherein the HCDR1 comprises the amino acid sequence of SEQ ID NO: 3; the HCDR2 comprises the amino acid sequence of SEQ ID NO: 4; the HCDR3 comprises the amino acid sequence of SEQ ID NO: 5; the LCDR1 comprises the amino acid sequence of SEQ ID NO: 6; the LCDR2 comprises the amino acid sequence of NTR; and the LCDR3 comprises the amino acid sequence of SEQ ID NO: 7.

The disclosure provides a method for treating a subject that has been pre-treated with a cancer therapeutic, wherein the antibody specifically binds hCEACAM5 wherein the antibody comprises a VH and a VL, wherein the VH comprises the three complementarity determining regions HCDR1, HCDR2 and HCDR3and wherein the VL comprises the three CDRs LCDR1, LCDR2 and LCDR3, wherein the HCDR1 comprises the amino acid sequence of SEQ ID NO: 3; the HCDR2 comprises the amino acid sequence of SEQ ID NO: 4; the HCDR3 comprises the amino acid sequence of SEQ ID NO: 5; the LCDR1 comprises the amino acid sequence of SEQ ID NO: 6; the LCDR2 comprises the amino acid sequence of NTR; and the LCDR3 comprises the amino acid sequence of SEQ ID NO: 7. In certain embodiments, the subject is a high carcinoembryonic antigen-related cell adhesion molecule expresser. In other embodiments, the subject was pre-treated with an agent or drug for treatment of non-small cell lung cancer. In other embodiments, the agent or drug is selected from the group consisting of: an chemotherapy agent, an angiogenesis inhibitor, an epidermal growth factor receptor (EGFR) inhibitor, an anaplastic lymphoma kinase (ALK) inhibitor, a receptor tyrosine kinase (ROS1) inhibitor, and an immune checkpoint inhibitor. In certain aspects of these embodiments, the immune checkpoint inhibitor is a PD-1 inhibitor and/or a PD-L1 inhibitor.

In various embodiments of the methods described above, the cancer is NSQ NSCLC.

In various embodiments of the method, the VH comprises SEQ ID NO: 1. In various embodiments of the method, the heavy chain comprises SEQ ID NO: 8.

In various embodiments of the method, the VL comprises SEQ ID NO: 2. In various embodiments of the method, the light chain comprises SEQ ID NO: 9.

In various embodiments of the method, the antibody is conjugated or linked to at least one growth inhibitory agent. For example, the growth inhibitory agent is a cytotoxic agent. In various embodiments of the method, the growth inhibitory agent is selected from the group consisting of chemotherapeutic agents, enzymes, antibiotics, and toxins such as small molecule toxins or enzymatically active toxins, taxoids, vincas, taxanes, maytansinoid or maytansinoid analogs, tomaymycin or pyrrolobenzodiazepine derivatives, cryptophycin derivatives, leptomycin derivatives, auristatin or dolastatin analogs, prodrugs, topoisomerase II inhibitors, DNA alkylating agents, anti-tubulin agents, and CC-1065 or CC-1065 analogs. In various embodiments of the method, the growth inhibitory agent is DM1 or DM4. For example, the growth inhibitory agent is DM4.

In various embodiments of the method, the antibody is covalently attached via a cleavable or non-cleavable linker to the at least one growth inhibitory agent. In various embodiments of the method, the linker is selected from the group consisting of SPDB, sulfo-SPDB, and SMCC. In one embodiment, the linker is SPDB.

In various embodiments of the method, the antibody is huMAb2-3. In various embodiments of the method, the subject patients has a hCEACAM5 expression of a percent score greater than or equal to 50 (consisting of 2+ and 3+ intensities) in the tumor cell populations. For example, the hCEACAM5 expression of a percent score greater than or equal to at least about 50, at least about 50 to about 80, at least about 80, or about 100 (consisting of 2+ and 3+ intensities). In various embodiments, the percentage of hCEACAM5 expression is at least about 50% to about 80% of the tumor cell populations, at least about 80% or about 100% of the tumor cell populations. In various embodiments of

the method, the cancer is a non-squamous non-small cell lung carcinoma that highly expresses hCEACAM5 in at least about 50%, at least about 50% to about 80%, at least about 80%, or about 100% of the tumor cell population.

In various embodiments of the method, the antibody is administered intravenously, for example by intravenous infusion.

In various embodiments of the method, the antibody is administered at a rate of 2.5 mg/min for the first 30 minutes. In various embodiments of the method, after 30 minutes, the rate of administration of the antibody is increased to 5 mg/min.

In various embodiments of the method, the antibody is administered at a dose level of 5, 10, 20, 30, 40, 60, 80, 100, 120, 150, 180, or 210 $mg/m^2$ based on the body surface area of the subject. In various embodiments of the method, the antibody is administered at a dose of about 2.5 mg/m2 to about 5 mg/m2. For example, the antibody is administered for one hour at a dose of about 2.5 mg/m2 to about 5 mg/m2. The dose includes 2.5 $mg/m^2$ of the antibody, 5 $mg/m^2$ of the antibody and all doses in between 2.5 $mg/m^2$ and 5 $mg/m^2$, for example 2.75, 3, 3.25, 3.5, 3.75, 4, 4.25, 4.5, and 4.75 $mg/m^2$. In various embodiments, the body surface area is calculated using the height and actual body of the subject.

In various embodiments of the method, the antibody is administered at every 14 days. In various embodiments, the antibody is administered every three weeks.

In various embodiments of the method, prior to administering the antibody, the subject is administered a pre-medication, for example the subject is pre-medication is a histamine H1 antagonist. In various embodiments of the method, the histamine H1 antagonist is diphenylhydramine or dexchlorpheniramine.

In various embodiments of the method, the subject was previously treated with an agent or drug for treatment of non-small cell lung cancer. For example, agent or drug is selected from the group consisting of: a chemotherapy agent, an angiogenesis inhibitor, an EGFR inhibitor, anaplastic lymphoma kinase (ALK) inhibitor, a receptor tyrosine kinase (ROS1) inhibitor, and an immune checkpoint inhibitor. For example, the immune checkpoint inhibitor is a PD-1 inhibitor and/or a PD-L1 inhibitor.

In some embodiments of the method described above, progression of at least one symptom of cancer is reduced, slowed, halted, or otherwise ameliorated. In certain aspects of these embodiments, the tumor growth rate or tumor size is reduced after treatment with the antibody. In other aspects of these embodiments, the expression pattern, intensity and proportion of expressing cells indicates a reduction, slowing or halting of the cancer.

The disclosure provides a pharmaceutical composition comprising the antibody described herein and a pharmaceutically acceptable carrier.

In various embodiments of the method, the antibody is administered as an ADC that specifically binds hCEACAM5 and comprises any antibody described herein, wherein progression of at least one symptom of cancer is reduced, slowed, halted, or otherwise ameliorated after administration/treatment with the ADC.

In various embodiments of the method, the ADC comprises the antibody huMAb2-3; wherein the growth inhibitory agent comprises DM4; and wherein the linker comprises SPDB. In various embodiments, the ADC comprises huMAb2-3-SPDB-DM4.

In various embodiments of the method, the tumor growth rate and/or tumor size is reduced after treatment with the antibody, the immunoconjugate and/or the ADC.

In various embodiments of the method, the expression pattern, intensity and proportion of expressing cells indicates a reduction, slowing or halting of the cancer after treatment with the antibody, the immunoconjugate and/or the ADC.

In various embodiments of the method, the antibody, immunoconjugate and/or the ADC is administered as a pharmaceutical composition comprising a pharmaceutically acceptable carrier.

## BRIEF DESCRIPTION OF THE FIGURES

**FIG. 1** is a table summarizing objective response results of non-small cell lung cancer patients treated with huMAb2-3-SPDB-DM4. The table compares objective response between NSCLC patients a hCEACAM5 expression of a percent score greater than or equal to 50 (consisting of 2+ and 3+ intensities) and a hCEACAM5 expression of a percent score of 1-49.

**FIG. 2** is a bar graph of the best relative tumor shrinkage in patients treated with huMAb2-3-SPDB-DM4 according to category of centralized CEACAM5 expression in an archival sample.

**FIG. 3** is a table of the duration of response and time to progression for huMAb2-3 - huMAb2-3-SPDB-DM4 treated NSCLC patients with a hCEACAM5 expression of a percent score greater than or equal to 50 (consisting of 2+ and 3+ intensities).

**FIG. 4** is a table summarizing the best objective response ICI pre-treated and not pre-treated NSCLC patients. The

NSCLC patients are those treated with huMAb2-3-SPDB-DM4 and with a hCEACAM5 expression of a percent score greater than or equal to 50 (consisting of 2+ and 3+ intensities).

## DETAILED DESCRIPTION

The disclosure provides pharmaceutical compositions and methods of using these compositions for the treatment of NSQ-NSCLC, and the improvement of at least one symptom of the disease. These compositions include at least one antibody that specifically binds (CEACAM5), for example the antibody is antibody huMAb2-3. The ADC huMAb2-3-SPDB-DM4 is an immunoconjugate combining huMAb2-3 (anti-CEACAM5) antibody and the maytansinoid derivative 4 (DM4), a potent antimitotic agent that inhibits microtubule assembly. DM4 is covalently bound to huMAb2-3through an optimized linker SPDB [N-succinimidyl 4-(2-pyridyldithio)-butyrate] that is stable in plasma and cleavable inside cells. After binding and internalization in targeted cancer cells, huMAb2-3-SPDB-DM4 is degraded, releasing cytotoxic DM4 metabolites.

As used herein, high CEACAM5 cancer refers to several types including lung, cancer. In some embodiments, the lung cancer is non-squamous non-small cell lung cancer. In certain embodiments, high CEACAM5 expressers have greater than 2+ intensity in at least 50% of expressing tumor cell population. High CEACAM5 expressers, represent ~20% of lung cancer. The ADC described herein comprising a DM4 cytotoxic agent, SPDB linker and humanized antibody huMAb2-3 was administered in a proof of concept study. Data show that the ADC achieved proof of concept in a subset of lung cancer.

The ADC was analyzed in a Phase 1/2 study in heavily pre-treated high CEACAM5 expressers. The ADC demonstrated competitive Overall Response Rate (ORR) and Duration of Response (DoR) for in 3L setting. Most common Adverse Drug Reactions (ADRs) were ocular toxicity (reversible without treatment discontinuation), and minimal hematological/nerve toxicity.

As used herein, "heavily pre-treated" refers to the pre-treatment of a subject for more than 1 month. In other embodiments, a subject who is heavily pre-treated has undergone treatment for more than 2, 3, 4, 5, 6, 7, 8, 9, 10, 11, 12, 13, 14, 15, 16, 17, 18, 19, 20, 21, 22, 23 or 24 months. In certain embodiments, the pre-treatment is the administration of one or more cancer therapeutics.

### Non-small cell lung cancer (NSCLC)

Non-small cell lung cancer is a disease in which malignant (cancer) cells form in the tissues of the lung. Smoking is the major cause of the disease. This is a type of epithelial lung cancer other than small cell lung carcinoma. There are several types of non-small cell lung cancer. Each type of non-small cell lung cancer has different kinds of cancer cells. The cancer cells of each type grow and spread in different ways. The types of non-small cell lung cancer are named for the kinds of cells found in the cancer and how the cells look under a microscope: (1) squamous cell carcinoma: Cancer that begins in squamous cells, which are thin, flat cells that look like fish scales. This is also called epidermoid carcinoma. (2) large cell carcinoma: Cancer that may begin in several types of large cells. And (3) adenocarcinoma: Cancer that begins in the cells that line the alveoli and make substances such as mucus.

Selectively targeting potent cytotoxics to tumor cells using ADCs has now been shown to be an effective strategy for the treatment of cancer, as demonstrated by the recent approvals of brentuximab vedotin for the treatment of Hodgkin lymphoma and trastuzumab emtansine (T-DM1) for the treatment of relapsed metastatic HER2+ breast cancer (Young A. et al., 2012J Clin Oncol. 30(18):2183-9; Verma, S. et al., 2012 N Engl J Med. 19:1783-91). Many other malignant diseases with unmet medical needs could benefit from such therapeutic options. The mechanism of action of ADCs begins with its binding to a specific antigen, sufficiently expressed on the tumor cells in order to achieve a selective and efficient internalization of the drug.

Radical surgery is the standard of care for fit stage I NSCLC patients (e.g. pneumonectomy, lobectomy, segmentectomy or wedge resection, sleeve resection). Adjuvant treatment should be offered only as part of an investigation trial. Stage II and IIIA adjuvant cisplatin-based chemotherapy remains the gold standard for completely resected NSCLC tumors. Other chemotherapeutic agents used in combination with cisplatin, or with each other, may include carboplatin, paclitaxel (Taxol), albumin-bound paclitaxel (nab-paclitaxel, Abraxane), docetaxel (Taxotere), Gemcitabine (Gemzar), vinorelbine (Navelbine), irinotecan (Camptosar), etoposide (VP-16), vinblastine, and pemetrexed (Alimta). Additionally, radiotherapy may be used in patients with N2 lymph nodes. In advanced stage IIIB/IV or inoperable NSCLC pts, treatment may include multiple cycles of cisplatin-based chemotherapy plus a 3rd generation cytotoxic agent or a cytostatic drug (anti-EGFR, anti-VEGFR). (See Zarogoulidis et al., J Thorac Dis. 2013 Sep; 5(Suppl 4): S389-S396.)

Treatments for cancers, including lung cancers can include angiogenesis inhibitors, Epidermal growth factor receptor (EGFR) inhibitors, anaplastic lymphoma kinase (ALK) inhibitors, receptor tyrosine kinase ROS1 inhibitors and immune checkpoint inhibitors. See Yadav, L. et al., J Clin Diagn Res. 2015 Jun; 9(6): XE01-XE05; Kyle, F. et al., 2008 Cancer Imaging. 8(1): 199-2; Chin, L.P. et al., 2012 Thorac Oncol. 7: 1625-1630; Dempke, W.C.M. et al., Lung Cancer,

Volume 67, Issue 3, 257 - 274; Chanprapaph, K. et al., 2014, Dermatol Res Pract. 734249; Jenkins, R. W. et al., 2018 British Journal of Cancer volume 118, pages 9-16, and De la Bellacasa, R. P. et al., 2013 Transl Lung Cancer Res. 2(2): 72-86.

Angiogenesis inhibitors may include, but are not limited to, Axitinib (Inlyta), Bevacizumab (Avastin), Cabozantinib (Cometriq), Everolimus (Afinitor, Zortress), Lenalidomide (Revlimid), Pazopanib (Votrient), Ramucirumab (Cyramza), Regorafenib (Stivarga), Sorafenib (Nexavar), Sunitinib (Sutent), Thalidomide (Synovir, Thalomid), Vandetanib (Caprelsa) and Ziv-aflibercept (Zaltrap).

Epidermal growth factor receptor (EGFR) inhibitors may include, but are not limited to, gefitinib (Iressa), erlotinib (Tarceva), lapatinib (Tykerb), cetuximab (Erbitux), neratinib (Nerlynx), osimertinib (Tagrisso), panitumumab (Vectibix), vandetanib (Caprelsa), necitumumab (Protrazza) and dacomitinib (Vizimpro).

Immune checkpoint inhibitors may include, but are not limited to, Programmed Death 1 (PD-1) receptor (PD-1) binding agents (e.g. pembrolizumab, nivolumab, cimiplimab), Programmed Death-ligand 1 (PD-L1) binding agents (e.g., atezolizumab, avelumab, durvalumab), CTLA-4 binding agents (e.g., ipilimumab), OX40 or OX40L binding agents, Adenosine A2A receptor binding agents, B7-H3 binding agents, B7-H4 binding agents, BTLA binding agents, Indoleamine 2,3-dioxygenase binding agents, Killer-cell Immunoglobulin-like Receptor (KIR) binding agents, Lymphocyte Activation Gene-3 (LAG-3) binding agents, nicotinamide adenine dinucleotide phosphate NADPH oxidase isoform (NOX2) binding agents, T-cell Immunoglobulin domain and Mucin domain 3 (TIM-3) binding agents, V-domain Ig suppressor of T cell activation (VISTA) binding agents, Glucocorticoid-Induced TNFR family Related gene (GITR) binding agents, and Sialic acid-binding immunoglobulin-type lectin 7 (SIGLEC7) binding agents.

## CEA and CEACAM

Carcino-embryonic antigen (CEA) is a glycoprotein involved in cell adhesion. CEA was first identified in 1965 (Gold and Freedman, J Exp Med, 121, 439, 1965) as a protein normally expressed by fetal gut during the first six months of gestation, and found in cancers of the pancreas, liver and colon. The CEA family belongs to the immunoglobulin superfamily. The CEA family, which consists of 18 genes, is sub-divided in two sub-groups of proteins: the carcinoembryonic antigen-related cell adhesion molecule (CEACAM) sub-group and the pregnancy-specific glycoprotein subgroup (Kammerer & Zimmermann, BMC Biology 2010, 8:12).

In humans, the CEACAM sub-group consists of 7 members: CEACAM1, CEACAM3, CEACAM4, CEACAM5, CEACAM6, CEACAM7, CEACAM8. Numerous studies have shown that CEACAM5, identical to the originally identified CEA, is highly expressed on the surface of colorectal, gastric, lung, breast, prostate, ovary, cervix, and bladder tumor cells and weakly expressed in few normal epithelial tissues such as columnar epithelial and goblet cells in colon, mucous neck cells in the stomach and squamous epithelial cells in esophagus and cervix (Hammarström et al, 2002, in "Tumor markers, Physiology, Pathobiology, Technology and Clin-ical Applications" Eds. Diamandis E. P. et al., AACC Press, Washington pp 375). Thus, CEA-CAM5 may constitute a therapeutic target suitable for tumor specific targeting approaches, such as immunoconjugates. The present invention provides antibodies directed against CEACAM5 and shows that they can be conjugated to a cytotoxic agent using a linker *in vivo* and safely administered to subjects having NSQ-NSCLC. Data show that the hCEACAM5 expression in various lung cancer cells was a percent score greater than or equal to about 50, about 50 to about 80, or about 100 (consisting of 2+ and 3+ intensities). The extracellular domains of CEACAM family members are composed of repeated immunoglobulin-like (Ig-like) domains which have been categorized in 3 types, A, B and N, according to sequence homologies. CEACAM5 contains seven such domains, namely N, A1, B1, A2, B2, A3 and B3.

CEACAM5 A1, A2 and A3 domains, on one hand, and B1, B2 and B3 domains, on the other hand, show high sequence homologies, the A domains of human CEACAM5 presenting from 84 to 87% pairwise sequence similarity, and the B domains from 69 to 80%. Furthermore, other human CEACAM members presenting A and/or B domains in their structure, namely CEACAM1, CEACAM6, CEACAM7 and CEACAM8, show homology with human CEACAM5. In particular, the A and B domains of human CEACAM6 protein display sequence homologies with A1 and A3 domains, and any of B1 to B3 domains of human CEACAM5, respectively, which are even higher than observed among the A domains and the B domains of human CEA-CAM5.

Numerous anti-CEA antibodies were generated in view of CEA-targeted diagnostic or therapeutic purposes. Specificity towards related antigens has always been mentioned as a concern in this field, as an example by Sharkey et al. (1990, Cancer Research 50, 2823). Due to the above-mentioned homologies some of previously described antibodies may demonstrate binding to repetitive epitopes of CEACAM5 present in the different immunoglobulin domains show cross-reactivity to other CEACAM members such as CEACAM1, CEACAM6, CEACAM7, or CEA-CAM8, lacking specificity to CEACAM5. The specificity of the anti-CEACAM5 antibody is de-sired in view of CEA-targeted therapies such that it binds to human CEACAM5-expressing tumor cells but does not bind to some normal tissues expressing the others CEACAM members. It is noteworthy that CEACAM1, CEACAM6 and CEACAM8 have been described as expressed by neutrophils of human and non-human primates (Ebrahimmnejad et al, 2000, Exp Cell Res, 260, 365; Zhao

et al, 2004, J Immunol Methods 293, 207; Strickland et al, 2009 J Pathol, 218, 380) where they have been shown to regulate granulopoiesis and to play a role in immune response.

An anti-CEACAM6 antibody drug conjugate has been described, such as the maytansinoid anti-CEACAM6 antibody developed by Genentech (Strickland et al, 2009 J Pathol, 218, 380), which has been shown to induce CEACAM6-dependent haematopoietic toxicity in non-human primates. This toxicity, attributed to accumulation of the antibody drug conjugate in bone marrow and depletion of granulocytes and their cell precursors, was considered by the authors as a major safety concern. So, more precisely, for therapeutic purposes, cross-reactivity of an anti-CEACAM5 antibody with CEACAM1, CEACAM6, CEACAM7, or CEACAM8 may de-crease the therapeutic index of the compound by increased toxicity on normal tissues. Thus, there is a strong advantage in obtaining antibodies specifically directed to CEACAM5 that would not cross-react with other molecules of the CEACAM family, especially for use as an antibody drug conjugate (ADC) or with any other mode of action resulting in killing the target cell.

Moreover, as CEACAM5 is described to be expressed, although at low level, in some normal cell tissues, it is critical to develop anti-CEACAM5 antibodies capable of binding to human CEACAM5 as well as to cynomolgus monkey (*Macacafascicularis*) CEACAM5, as such antibodies may be readily tested in preclinical toxicological studies in cynomolgus monkeys to evaluate their safety profile. Since it has been shown that the efficiency of therapeutic antibodies may be dependent on the localization of the epitope in the target, both in the case of functional antibodies (Doern et al. 2009, J. Biol. Chem 284 10254) and in the case where effector functions are involved (Beers et al. Semin Hematol 47:107-114), a human/monkey cross-reactive antibody has to be shown to bind epitopes in the same repeated Ig-like homologous domain of human and cynomolgus monkey proteins.

Combining the need for species cross-reactivity of such antibodies with the specificity for human and *Macaca fascicularis* CEACAM5, *i.e*., no cross reactivity with other Macaca fascicu-laris and human CEACAM members, adds a further degree of complexity, given the overall sequence homologies between human and *Macaca fascicularis* CEACAM proteins.

Indeed, global pairwise alignment of *Macaca fascicularis* CEACAM5 sequence with human CEACAM5 sequence (AAA51967.1/GI:180223, 702 amino acids) indicated only 78.5% identity. *Macaca fascicularis* CEACAM1, CEACAM5, and CEACAM6 genes were cloned and a global alignment of human and *Macaca fascicularis* A, B and N domains was performed. This alignment predicted that there are very few regions, if any, to localize an ideal epitope that would be common to human and macaque CEACAM5 and not shared with any other family member. For these reasons developing antibodies cross-reactive between human and Macaca fascicu-laris CEACAM5 without cross-reactivity with other human and *Macaca fascicularis* CEACAM members was expected to have a low probability of success. Noteworthy, previously described anti-CEACAM5 antibodies are almost never documented for *Macaca fascicularis* cross-reactivity, with very few exceptions (MT111).

Anti-human CEACAM5 antibodies have already been used in clinical trials, such as Immunomedics labetuzumab (also known as hMN14, Sharkey et al, 1995, Cancer Research 55, 5935). This antibody has been shown not to bind to related antigens, but is not cross-reacting with CEACAM5 from *Macaca fascicularis.* Noteworthy, Micromet's MT111 antibody (also known as MEDI-565 antibody of MedImmune) is a bi-specific antibody binding to human CEA-CAM5 and human CD3 (Peng et al., PLoS ONE 7(5): e3641; WO 2007/071426). MT111 is said to have been created by fusion of a single chain variable fragment (scFv) from an antibody that recognizes human and cynomolgus CEACAM5 with scFv from an antibody that recognize human CD3 (poster of Oberst et al., AACR Annual Meeting April 2009 Denver, CO). It has also been reported that MT111 does not bind other CEACAM family members (Peng et al., PLoS ONE 7(5): e3641). MT111 binds to a conformational epitope in the A2 domain of human CEA-CAM5. This conformational epitope is missing in a splice variant of human CEACAM5, which is expressed concomitantly with full-length CEACAM5 on tumors (Peng et al., PLoS ONE 7(5): e3641). In addition, there is no evidence that MT111 binds to the same epitope in Macaca fascicularis CEACAM5.

In an attempt to produce new antibodies against CEACAM5 surface protein with optimal characteristics for therapeutic purposes, mice were immunized with recombinant proteins and with tumour cells. They have screened hundreds of hybridoma using ELISA on several recombinant proteins of the CEACAM family, and flow cytometry with relevant cell lines, in order to select only immunoglobulins (IgGs) with the advantageous profile. Unexpectedly, they were able to select hybridoma clones and produce corresponding mature IgGs that comprise all of the desired features. They specifically bind to the A3-B3 domain of human CEACAM5 with a high affinity and do not recognize human CEACAM1, CEACAM6, CEACAM7 and CEACAM8 proteins. In a cellular context, these antibodies display high affinity for tumor cells (in the nanomolar range). Moreover, these antibodies also bind to *Macaca fascicularis* CEACAM5 protein with a ratio of affinity monkey/human less than or equal to 10.

By targeting the A3-B3 domain of CEACAM5, these antibodies have increased tumour-targeting potential, as they have the capacity to bind both full-length human CEACAM5 and to its splice variant identified by Peng *et al.* See Finally, CEACAM5 is described in literature as a poorly internalizing surface protein (reviewed in Schmidt et al, 2008, Cancer Immunol. Immunother. 57, 1879), and therefore may not be a favourable target for antibody drug conjugates. In spite of what has been reported in the prior art, the inventors have shown that the antibodies they have produced are

able to internalize the CEACAM5-antibody complex after binding, and to induce cytotoxic activity on tumor cells *in vitro* when combined to a cytotoxic agent. The same antibodies combined to a cytotoxic agent are also able to markedly inhibit tumor growth in mice bearing human primary colon and stomach tumors. See WO2014079886, which is incorporated herein in its entirety.

**Definitions**

As used herein, the term "about" in quantitative terms refers to plus or minus 10% of the value it modifies (rounded up to the nearest whole number if the value is not sub-dividable, such as a number of molecules or nucleotides). For example, the phrase "about 100 mg" would encompass 90 mg to 110 mg, inclusive; the phrase "about 2500 mg" would encompass 2250 mg to 2750 mg. When applied to a percentage, the term "about" refers to plus or minus 10% relative to that percentage. For example, the phrase "about 20%" would encompass 18-22% and "about 80%" would encompass 72-88%, inclusive. Moreover, where "about" is used herein in conjunction with a quantitative term it is understood that in addition to the value plus or minus 10%, the exact value of the quantitative term is also contemplated and described. For example, the term "about 23%" expressly contemplates, describes, and includes exactly 23%.

It is to be noted that the term "a" or "an" entity refers to one or more of that entity; for example, "a symptom," is understood to represent one or more symptoms. As such, the terms "a" (or "an"), "one or more," and "at least one" can be used interchangeably herein.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It is understood that wherever aspects are described herein with the language "comprising," otherwise analogous aspects described in terms of "consisting of' and/or "consisting essentially of' are also provided.

As used herein "CEACAM5" designates the "carcinoembryonic antigen-related cell adhesion molecule 5", also known as "CD66e" (Cluster of Differentiation 66e) or CEA. CEACAM5 is a glycoprotein involved in cell adhesion. CEACAM5 is highly expressed in particular on the surface of colorectal, gastric, lung and uterine tumor cells.

A reference sequence of full length human CEACAM5, including signal peptide (positions 1-34) and pro-peptide (positions 686-702), is available from the GenBank database under accession number AAA51967.1 (SEQ ID NO:52) Five non-synonymous SNPs have been identified with a frequency higher than 2% in caucasian population, four of them being localised in the N domain (at positions 80, 83, 112, 113), the last one in the A2 domain (at position 398) of human CEACAM5 (SEQ ID NO:58). GenBank AAA51967.1 contains the major haplotype (I80, V83, I112, I113 and E398).

A sequence of the extracellular domain of *Macaca fascicularis* CEACAM5, cloned by the inventors, is disclosed in SEQ ID NO: 12. See also WO2014079886, which is incorporated by reference in its entirety.

SEQ ID NO: 12

QLTIESRPFNVAEGKEVLLLAHNVSQNLFGYIWYKGERVDASRRIGSCVIRTQQITPGPA
HSGRETIDFNASLLIQNVTQSDTGSYTIQVIKEDLVNEEATGQFRVYPELPKPYITSNNSN
PIEDKDAVALTCEPETQDTTYLWWVNNQSLPVSPRLELSSDNRTLTVFNIPRNDTTSYKC
ETQNPVSVRRSDPVTLNVLYGPDAPTISPLNTPYRAGEYLNLTCHAASNPTAQYFWFVN
GTFQQSTQELFIPNITVNNSGSYMCQAHNSATGLNRTTVTAITVYAELPKPYITSNNSNPI
EDKDAVTLTCEPETQDTTYLWWVNNQRLSVSSRLELSNDNRTLTVFNIPRNDTTFYECE
TQNPVSVRRSDPVTLNVLYGPDAPTISPLNTPYRAGENLNLSCHAASNPAAQYFWFVNG
TFQQSTQELFIPNITVNNSGSYMCQAHNSATGLNRTTVTAITVYVELPKPYISSNNSNPIE
DKDAVTLTCEPVAENTTYLWWVNNQSLSVSPRLQLSNGNRILTLLSVTRNDTGPYECGI
QNSESAKRSDPVTLNVTYGPDTPIISPPDLSYRSGANLNLSCHSDSNPSPQYSWLINGTLR
QHTQVLFISKITSNNNGAYACFVSNLATGRNNSIVKNISVSSGDSAPGSSGLSA

A "domain" may be any region of a protein, generally defined on the basis of sequence homologies and often related to a specific structural or functional entity. CEACAM family members are known to be composed of Ig-like domains. The term domain is used in this document to designate either individual Ig-like domains, such as "N-domain" or for groups of consecutive domains, such as "A3-B3 domain".

A Domain organisation of human CEACAM5 is as follows (based on GenBank AAA51967.1; SEQ ID NO: 13):

SEQ ID NO: 13

MESPSAPPHRWCIPWQRLLLTASLLTFWNPPTTAKLTIESTPFNVAEGKEVLLLVHNLPQ
HLFGYSWYKGERVDGNRQIIGYVIGTQQATPGPAYSGREIIYPNASLLIQNIIQNDTGFYT
LHVIKSDLVNEEATGQFRVYPELPKPSISSNNSKPVEDKDAVAFTCEPETQDATYLWWV
NNQSLPVSPRLQLSNGNRTLTLFNVTRNDTASYKCETQNPVSARRSDSVILNVLYGPDA
PTISPLNTSYRSGENLNLSCHAASNPPAQYSWFVNGTFQQSTQELFIPNITVNNSGSYTCQ
AHNSDTGLNRTTVTTITVYAEPPKPFITSNNSNPVEDEDAVALTCEPEIQNTTYLWWVNN
QSLPVSPRLQLSNDNRTLTLLSVTRNDVGPYECGIQNELSVDHSDPVILNVLYGPDDPTIS
PSYTYYRPGVNLSLSCHAASNPPAQYSWLIDGNIQQHTQELFISNITEKNSGLYTCQANN
SASGHSRTTVKTITVSAELPKPSISSNNSKPVEDKDAVAFTCEPEAQNTTYLWWVNGQS
LPVSPRLQLSNGNRTLTLFNVTRNDARAYVCGIQNSVSANRSDPVTLDVLYGPDTPIISPP
DSSYLSGANLNLSCHSASNPSPQYSWRINGIPQQHTQVLFIAKITPNNNGTYACFVSNLA
TGRNNSIVKSITVSASGTSPGLSAGATVGIMIGVLVGVALI

Table 1A. Human CEACAM5 domains

| Human CEACAM5 domains | Positions on SEQ ID NO :13 |
|---|---|
| Domain N | 35 - 142 |
| Domain A1 | 143 - 237 |
| Domain B1 | 238 - 320 |
| Domain A2 | 321 - 415 |
| Domain B2 | 416 - 498 |
| Domain A3 | 499 - 593 |
| Domain B3 | 594 - 685 |

Accordingly, the A3-B3 domain of human CEACAM5 consists of amino acids at positions 499-685 of SEQ ID NO:13.

Domain organization of *Macaca fascicularis* CEACAM5 is as follows (based on cloned extracellular domain sequence; SEQ ID NO:12):

Table 1B: *Macaca fascicularis* CEACAM5 domains

| *Macaca fascicularis* CEACAM5 domains | Positions on SEQ ID NO :12 |
|---|---|
| Domain N-A1-B1 | -1 - 286 |
| Domain A2-B2 | -287 - 464 |
| Domain A3-B3 | 465 - 654 |

Accordingly, the A3-B3 domain of *Macaca fascicularis* CEACAM5 consists of amino acids at positions 465-654 of SEQ ID NO:53.

A "coding sequence" or a sequence "encoding" an expression product, such as a RNA, polypeptide, protein, or enzyme, is a nucleotide sequence that, when expressed, results in the production of that RNA, polypeptide, protein, or enzyme, *i.e.,* the nucleotide sequence encodes an amino acid sequence for that polypeptide, protein or enzyme. A coding sequence for a protein may include a start codon (usually ATG) and a stop codon.

As used herein, references to specific proteins (*e.g.*, antibodies) can include a polypeptide having a native amino acid sequence, as well as variants and modified forms regardless of their origin or mode of preparation. A protein which has a native amino acid sequence is a protein having the same amino acid sequence as obtained from nature. Such native sequence proteins can be isolated from nature or can be prepared using standard recombinant and/or synthetic methods. Native sequence proteins specifically encompass naturally occurring truncated or soluble forms, naturally occurring variant forms (*e.g.*, alternatively spliced forms), naturally occurring allelic variants and forms including post-translational modifications. Native sequence proteins include proteins carrying post-translational modifications such as glycosylation, or phosphorylation, or other modifications of some amino acid residues.

The term "gene" means a DNA sequence that codes for, or corresponds to, a particular sequence of amino acids which comprises all or part of one or more proteins or enzymes, and may or may not include regulatory DNA sequences, such as promoter sequences, which determine for example the conditions under which the gene is expressed. Some genes, which are not structural genes, may be transcribed from DNA to RNA, but are not translated into an amino acid sequence. Other genes may function as regulators of structural genes or as regulators of DNA transcription. In particular, the term gene may be intended for the genomic sequence encoding a protein, *i.e.* a sequence comprising regulator, promoter, intron and exon sequences.

A percentage of "sequence identity" may be determined by comparing the two sequences, optimally aligned over a comparison window, wherein the portion of the polynucleotide or polypeptide sequence in the comparison window may comprise additions or deletions (*i.e.,* gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. Optimal alignment of sequences for comparison is conducted by global pairwise alignment, *e.g.* using the algorithm of Needleman and Wunsch J. Mol. Biol. 48:443 (1970). The percentage of sequence identity can be readily determined for instance using the program Needle, with the BLOSUM62 matrix, and the following parameters gap-open=10, gap-extend=0.5.

A "conservative amino acid substitution" is one in which an amino acid residue is substituted by another amino acid residue having a side chain R group with similar chemical properties (*e.g.*, charge, size or hydrophobicity). In general, a conservative amino acid substitution will not substantially change the functional properties of a protein. Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Conservative amino acids substitution groups can also be defined on the basis of amino acid size

The present disclosure includes methods that comprise administering to a subject an antibody, or an antigen-binding fragment thereof, that binds specifically to CEACAM5. As used herein, the term "hCEACAM5" means a human cytokine receptor that specifically binds human CEACAM5. In certain embodiments, the antibody that is administered to the patient binds specifically to at least one domain of hCEACAM5.

Studies were performed for generating, screening and selecting specific mouse anti-CEACAM5 antibodies displaying high affinity for both human and *Macaca fascicularis* CEACAM5 protein, and which do not significantly cross-react with human CEACAM1, CEACAM6, CEACAM7 and CEACAM8 proteins, and with *Macaca fascicularis* CEACAM1, CEACAM6 and CEACAM8 proteins.

The so-called "antibody MAb1" comprises:

- a variable domain of heavy chain consisting of sequence EVMLVESGGGLVKPGGSLKLSCAAS**GFTFSSYA**M-SWVRQTPEKRLEWVATI**SSGGSYI** YYLDSVKGRFTISRDNAKNTLYLQMSSLRSEDTAMYYC**ARPAYYGNPAM-DY**WGQGTS VTVSS (SEQ ID NO:14, with CDRs shown in bold characters) in which FR1-H spans amino acid positions 1 to 25, CDR1-H spans amino acid positions 26 to 33, FR2-H spans amino acid positions 34 to 50, CDR2-H spans amino acid positions 51 to 58, FR3-H spans amino acid positions 59 to 96, CDR3-H spans amino acid positions 97 to 109, and FR4-H spans amino acid positions 110 to 120, and
- a variable domain of light chain consisting of sequence DILMTQSQKFMSTSVGDRVSVTCKAS**QNVGTN**VAWYQQKPGQSPKPLIY**SAS**YRYSGV PDRFTGSGSGTDFTLTISNVQSEDLAEYFC**QQYNSYPLYT**FGGGTKLEIK (SEQ ID NO:15, with CDRs shown in bold characters) in which FR1-L spans amino acid positions 1 to 26,

CDR1-L spans amino acid positions 27 to 32, FR2-L spans amino acid positions 33 to 49, CDR2-L spans amino acid positions 50 to 52, FR3-L spans amino acid positions 53 to 88, CDR3-L spans amino acid positions 89 to 98, and FR4-L spans amino acid positions 99 to 108.

The so-called "antibody MAb2" comprises:

- a variable domain of heavy chain consisting of sequence EVQLQESGGVLVKPGGSLKLSCAAS**GFVFSSYD**MSWVRQTPEKRLEWVAY**ISSGGGIT** YFPDTVQGRFTVSRDNAKNTLYLQMNSLKSEDTAIYYC**AAHYFGSSGPFAY**WGQGTL VTVSA (SEQ ID NO:16, with CDRs shown in bold characters) in which FR1-H spans amino acid positions 1 to 25, CDR1-H spans amino acid positions 26 to 33, FR2-H spans amino acid positions 34 to 50, CDR2-H spans amino acid positions 51 to 58, FR3-H spans amino acid positions 59 to 96, CDR3-H spans amino acid positions 97 to 109, and FR4-H spans amino acid positions 110 to 120, and
- a variable domain of light chain consisting of sequence DIQMTQSPASLSASVGETVTITCRAS**ENIFSY**LA WYQQKQGKSPQLLVYNTKTLAEGVP SRFSGSGSGTQFSLKINSLQPEDFGSYYC**QHHYGTPFT**FGSGTKLEIK (SEQ ID NO:17, with CDRs shown in bold characters) in which FR1-L spans amino acid positions 1 to 26, CDR1-L spans amino acid positions 27 to 32, FR2-L spans amino acid positions 33 to 49, CDR2-L spans amino acid positions 50 to 52, FR3-L spans amino acid positions 53 to 88, CDR3-L spans amino acid positions 89 to 97, and FR4-L spans amino acid positions 98 to 107.

A variant of antibody MAb2 was also generated by introducing a K52R substitution in the CDR2-L. This variant, which is called herein "Mab2$_{K52R}$", has essentially the same affinity for human and *Macaca fascicularis* CEACAM5 as MAb2.

The so-called "antibody MAb3" comprises:

- a variable domain of heavy chain consisting of sequence EVKLVESGGGLVKPGGSLTLPCAAS**GFTFSRYA**MSWVRQTPEKRLEWVAS**ISSGGDT**Y YPDSVKGRFTVSRDNARNILFLQMSSLRSEDTGMYYC**ARVNYYDSSFLDW**WGQGTTL TVSS (SEQ ID NO:18, with CDRs shown in bold characters) in which FR1-H spans amino acid positions 1 to 25, CDR1-H spans amino acid positions 26 to 33, FR2-H spans amino acid positions 34 to 50, CDR2-H spans amino acid positions 51 to 57, FR3-H spans amino acid positions 58 to 95, CDR3-H spans amino acid positions 96 to 108, and FR4-H spans amino acid positions 109 to 119, and
- a variable domain of light chain consisting of sequence DIVMTQSQRFMSTLEGDRVSVTCKAS**QNVGTN**VAWYQQKPGQSPKALIY**SAS**YRYSG VPDRFTGSGSGTDFTLTISNVQSEDLAEYFC**QQYNNYPLYTF**GGGTKLEIK (SEQ ID NO:19, with CDRs shown in bold characters) in which FR1-L spans amino acid positions 1 to 26, CDR1-L spans amino acid positions 27 to 32, FR2-L spans amino acid positions 33 to 49, CDR2-L spans amino acid positions 50 to 52, FR3-L spans amino acid positions 53 to 88, CDR3-L spans amino acid positions 89 to 98, and FR4-L spans amino acid positions 99 to 108.

The so-called "antibody MAb4" comprises:

- a variable domain of heavy chain consisting of sequence EVQLVESGGGLVKPGGSLKLSCAAS**GFTFSSYD**MSWVRQTPEKRLEWVAF**ISSYGGRT** YYADTVKGRFTISRDNAKNTLYLQMSSLKSEDTAMFYC**AAHYFGTSGPFAY**WGQGTL VTVSA (SEQ ID NO:20, with CDRs shown in bold characters) in which FR1-H spans amino acid positions 1 to 25, CDR1-H spans amino acid positions 26 to 33, FR2-H spans amino acid positions 34 to 50, CDR2-H spans amino acid positions 51 to 58, FR3-H spans amino acid positions 59 to 96, CDR3-H spans amino acid positions 97 to 109, and FR4-H spans amino acid positions 110 to 120, and
- a variable domain of light chain consisting of sequence DIQMTQSPASLSASVGETVTITCRAS**ENIYSYF**AWYQQKQGKSPQLLVY**NAK**ILAEGVP SRFSGSGSGTQFSLKINSLQPEDFGTYYC**QHHYGIPFT**FGSGTKLELK (SEQ ID NO:21, with CDRs shown in bold characters) in which FR1-L spans amino acid positions 1 to 26, CDR1-L spans amino acid positions 27 to 32, FR2-L spans amino acid positions 33 to 49, CDR2-L spans amino acid positions 50 to 52, FR3-L spans amino acid positions 53 to 88, CDR3-L spans amino acid positions 89 to 97, and FR4-L spans amino acid positions 98 to 107.

The so-called "antibody MAb5" comprises:

- a variable domain of heavy chain consisting of sequence ELQLVESGGVLVKPGGSLKLSCAAS**GFAFSSYD**MSWVRQTPEKRLEWVTY**INSGGGIT** YYPDTVKGRFTISRDNARNTLYLQMSSLKSEDTAIYYC**TAHYFGSSGPFAY**WGQGTLV TVSA (SEQ ID NO:22, with CDRs shown in bold characters) in which FR1-H spans amino acid positions 1 to 25, CDR1-H spans amino acid positions 26 to 33, FR2-H spans amino acid positions 34 to 50, CDR2-H spans amino acid positions 51 to 58, FR3-H spans amino acid positions 59 to 96, CDR3-H spans amino acid positions 97 to 109, and FR4-H spans amino acid positions 110 to 120, and

- a variable domain of light chain consisting of sequence DIQMTQSPASLSASVGETVTITCRAS**ENIYSY**LAWYQQKQGKSPQLLVY**NAK**TLTEGVP SRFSGSGSGTQFSLKINSLQPEDFGSYYC**QHHYGTPFT**FGSGTKLEIK (SEQ ID NO:23, with CDRs shown in bold characters) in which FR1-L spans amino acid positions 1 to 26, CDR1-L spans amino acid positions 27 to 32, FR2-L spans amino acid positions 33 to 49, CDR2-L spans amino acid positions 50 to 52, FR3-L spans amino acid positions 53 to 88, CDR3-L spans amino acid positions 89 to 97, and FR4-L spans amino acid positions 98 to 107.

Multiple variations and variants of these antibodies are described in WO2014079886, which is incorporated by reference in its entirety. In an embodiment, the antibody of the invention is antibody huMAb2-3 or a variant thereof, *i.e.* an isolated antibody which binds to A3-B3 domain of human and Macaca fascicularis CEACAM5 proteins and which comprises:

a) a heavy chain consisting of sequence SEQ ID NO:8 or a sequence at least 85% identical thereto; or
b) a light chain consisting of sequence SEQ ID NO:9 or a sequence at least 85% identical thereto or a heavy chain and a light chain.

In various embodiments, the invention relates to an antibody which binds to human and *Macaca fascicularis* CEACAM5. In an embodiment the antibody of the invention binds to the A3-B3 domains of human and *Macaca fascicularis* CEACAM5. More specifically, the antibody can bind to the human and *Macaca fascicularis* A3-B3 domains indifferently whether expressed in isolated form, or present in a soluble extracellular domain or membrane-anchored full-length CEACAM5 protein.

The specificity of the antibodies for the A3-B3 domain of human CEACAM5 is advantageous as no SNP with a frequency higher than 2% in Caucasian population was reported in this domain, which minimizes the risk that the antibodies' epitope(s) on CEACAM5 be altered in part of the population.

According to an embodiment, the antibody according to the invention is specific for the surface human and *Macaca fascicularis* CEACAM5 proteins. In an embodiment, the antibody of the invention does not bind to, or does not significantly cross-react with human CEACAM1, human CEACAM6, human CEACAM7, human CEACAM8, *Macaca fascicularis* CEACAM1, *Macaca fascicularis* CEACAM6 and *Macaca fascicularis* CEACAM8 proteins.

In various embodiments, the antibody does not bind to, or does not significantly cross-react with the extracellular domain of the aformentionned human and *Macaca fascicularis* CEACAM proteins.

Human CEACAM1 full-length protein is available in GenBank database under accession number NP_001703.2. The extracellular domain of human CEACAM 1 consists of amino acids at positions 35-428 of SEQ ID NO:11. Human CEACAM6 full-length protein is available in GenBank database under accession number NP_002474.3. The extracellular domain of human CEACAM6 consists of amino acids at positions 35-327 of this protein.

Human CEACAM7 full-length protein is available in GenBank database under accession number NP_008821.1 (SEQ ID NO:72). The extracellular domain of human CEACAM7 consists of amino acids at positions 36-248 of the protein.

Human CEACAM8 full-length protein is available in GenBank database under accession number NP_001807.2 (SEQ ID NO:73). The extracellular domain of human CEACAM8 consists of amino acids at positions 35-332 of the protein.

*M.fascicularis* CEACAM1 extracellular domain consists of amino acids at positions 35-428 of full-length protein, *i.e.* amino acids 1-394 of the protein.

*M.fascicularis* CEACAM6 extracellular domain consists of amino acids at positions 35-327 of full-length protein, *i.e.* amino acids 1-293 the protein.

*M.fascicularis* CEACAM8 extracellular domain consists of amino acids at positions 35-332 of full-length protein, *i.e.* amino acids 1-298 of the protein. The term "antibody", as used herein, refers to immunoglobulin molecules comprising four polypeptide chains, two heavy (H) chains and two light (L) chains inter-connected by disulfide bonds, as well as multimers thereof (*e.g.,* IgM). Each heavy chain comprises a heavy chain variable region (abbreviated herein as HCVR or VH) and a heavy chain constant region. The heavy chain constant region comprises three domains, CH1, CH2 and CH3. Each light chain comprises a light chain variable region (abbreviated herein as LCVR or VL) and a light chain constant region. The light chain constant region comprises one domain (CL1). The VH and VL regions can be further subdivided into regions of hypervariability, termed complementarity determining regions (CDRs), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. In some embodiments, the FRs of the antibody (or antigen-binding portion thereof) may be identical to the human germline sequences, or may be naturally or artificially modified. An amino acid consensus sequence may be defined based on a side-by-side analysis of two or more CDRs.

The term "antibody," as used herein, also includes antigen-binding fragments of full antibody molecules. The

terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, e.g., commercial sources, DNA libraries (including, e.g., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (*e.g.*, an isolated complementarity determining region (CDR) such as a CDR3 peptide, or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (e.g., monovalent nanobodies, and bivalent nanobodies), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR which is adjacent to or in frame with one or more framework sequences. In antigen-binding fragments having a VH domain associated with a VL domain, the VH and VL domains may be situated relative to one another in any suitable arrangement. For example, the variable region may be dimeric and contain VH-VH, VH-VL or VL-VL dimers. Alternatively, the antigen-binding fragment of an antibody may contain a monomeric VH or VL domain.

In certain embodiments, an antigen-binding fragment of an antibody may contain at least one variable domain covalently linked to at least one constant domain. Non-limiting, exemplary configurations of variable and constant domains that may be found within an antigen-binding fragment of an antibody include: (i) VH-CH1; (ii) VH-CH2; (iii) VH-CH3; (iv) VH-CH1-CH2; (v) VH-CH1-CH2-CH3; (vi) VH-CH2-CH3; (vii) VH-CL; (viii) VL-CH1; (ix) VL-CH2; (x) VL-CH3; (xi) VL-CH1-CH2; (xii) VL-CH1-CH2-CH3; (xiii) VL-CH2-CH3; and (xiv) VL-CL. In any configuration of variable and constant domains, including any of the exemplary configurations listed above, the variable and constant domains may be either directly linked to one another or may be linked by a full or partial hinge or linker region. A hinge region may in various embodiments consist of at least 2 (*e.g.*, 5, 10, 15, 20, 40, 60 or more) amino acids which result in a flexible or semi-flexible linkage between adjacent variable and/or constant domains in a single polypeptide molecule. Moreover, an antigen-binding fragment of an antibody may in various embodiments comprise a homo-dimer or hetero-dimer (or other multimer) of any of the variable and constant domain configurations listed above in non-covalent association with one another and/or with one or more monomeric VH or VL domain (e.g., by disulfide bond(s)).

In specific embodiments, the antibody or antibody fragment for use in the method of the invention may be a multispecific antibody, which may be specific for different epitopes of one target polypeptide or may contain antigen-binding domains specific for epitopes of more than one target polypeptide. An exemplary bi-specific antibody format that can be used in the context of the present invention involves the use of a first immunoglobulin (Ig) $C_{H3}$ domain and a second Ig $C_{H3}$ domain, wherein the first and second Ig $C_{H3}$ domains differ from one another by at least one amino acid, and wherein at least one amino acid difference reduces binding of the bispecific antibody to Protein A as compared to a bi-specific antibody lacking the amino acid difference. In one embodiment, the first Ig $C_{H3}$ domain binds Protein A and the second Ig $C_{H3}$ domain contains a mutation that reduces or abolishes Protein A binding such as an H95R modification (by IMGT exon numbering; H435R by EU numbering). The second $C_{H3}$ may further comprise an Y96F modification (by IMGT; Y436F by EU). Further modifications that may be found within the second $C_{H3}$ include: D16E, L18M, N44S, K52N, V57M, and V82I (by IMGT; D356E, L358M, N384S, K392N, V397M, and V422I by EU) in the case of IgG1 antibodies; N44S, K52N, and V82I (IMGT; N384S, K392N, and V422I by EU) in the case of IgG2 antibodies; and Q15R, N44S, K52N, V57M, R69K, E79Q, and V82I (by IMGT; Q355R, N384S, K392N, V397M, R409K, E419Q, and V422I by EU) in the case of IgG4 antibodies. Variations on the bi-specific antibody format described above are contemplated within the scope of the present invention. Any multispecific antibody format, including the exemplary bispecific antibody formats disclosed herein, may in various embodiments be adapted for use in the context of an antigen-binding fragment of an anti-CEACAM5 antibody using routine techniques available in the art.

The CEACAM5 antibodies disclosed herein may comprise one or more amino acid substitutions, insertions and/or deletions in the framework and/or CDR regions of the heavy and light chain variable domains as compared to the corresponding germline sequences. Such mutations can be readily ascertained by comparing the amino acid sequences disclosed herein to germline sequences available from, for example, public antibody sequence databases. The present invention includes antibodies, and antigen-binding fragments thereof, which are derived from any of the amino acid sequences disclosed herein, wherein one or more amino acids within one or more framework and/or CDR regions

are back-mutated to the corresponding germline residue(s) or to a conservative amino acid substitution (natural or non-natural) of the corresponding germline residue(s) (such sequence changes are referred to herein as "germline back-mutations"). A person of ordinary skill in the art, starting with the heavy and light chain variable region sequences disclosed herein, can easily produce numerous antibodies and antigen-binding fragments which comprise one or more individual germline back-mutations or combinations thereof. In certain embodiments, all of the framework residues and/or CDR residues within the VH and/or VL domains are mutated back to the germline sequence. In other embodiments, only certain residues are mutated back to the germline sequence, e.g., only the mutated residues found within the first 8 amino acids of FR1 or within the last 8 amino acids of FR4, or only the mutated residues found within CDR1, CDR2 or CDR3. Furthermore, the antibodies of the present invention may contain any combination of two or more germline back-mutations within the framework and/or CDR regions, *i.e.,* wherein certain individual residues are mutated back to the germline sequence while certain other residues that differ from the germline sequence are maintained. Once obtained, antibodies and antigen-binding fragments that contain one or more germline back-mutations can be easily tested for one or more desired property such as, improved binding specificity, increased binding affinity, improved or enhanced antagonistic or agonistic biological properties (as the case may be), reduced immunogenicity, *etc.* Antibodies and antigen-binding fragments obtained in this general manner are encompassed within the present invention.

The constant region of an antibody is important in the ability of an antibody to fix complement and mediate cell-dependent cytotoxicity. Thus, the isotype of an antibody may be selected on the basis of whether it is desirable for the antibody to mediate cytotoxicity.

The term "human antibody", as used herein, is intended to include antibodies having variable and constant regions derived from human germline immunoglobulin sequences. The human antibodies featured in the disclosure may in various embodiments nonetheless include amino acid residues not encoded by human germline immunoglobulin sequences (*e.g*., mutations introduced by random or site-specific mutagenesis *in vitro* or by somatic mutation *in vivo*), for example in the CDRs and in some embodiments CDR3. However, the term "human antibody", as used herein, is not intended to include antibodies in which CDR sequences derived from the germline of another mammalian species, such as a mouse, have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (*e.g*., a mouse) that is transgenic for human immunoglobulin genes (see *e.g.,* Taylor et al., (1992) Nucl. Acids Res. 20:6287-6295, incorporated herein by reference in its entirety,) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.*

Human antibodies can exist in two forms that are associated with hinge heterogeneity. In an embodiment, an immunoglobulin molecule comprises a stable four chain construct of approximately 150-160 kDa in which the dimers are held together by an interchain heavy chain disulfide bond. In another embodiment, the dimers are not linked via inter-chain disulfide bonds and a molecule of about 75-80 kDa is formed composed of a covalently coupled light and heavy chain (half-antibody). These embodiments/forms have been extremely difficult to separate, even after affinity purification.

The term "humanised antibody" or "humanized antibody" refers to an antibody which is wholly or partially of non-human origin and which has been modified to replace certain amino acids, for instance in the framework regions of the VH and VL domains, in order to avoid or minimize an immune response in humans. The constant domains of a humanized antibody are most of the time human CH and CL domains.

Numerous methods for humanisation/humanization of an antibody sequence are known in the art; see *e.g.* the review by Almagro & Fransson (2008) Front Biosci. 13: 1619-1633. One commonly used method is CDR grafting, or antibody reshaping, which involves grafting of the CDR sequences of a donor antibody, generally a mouse antibody, into the framework scaffold of a human antibody of different specificity. Since CDR grafting may reduce the binding specificity and affinity, and thus the biological activity, of a CDR grafted non-human antibody, back mutations may be introduced at selected positions of the CDR grafted antibody in order to retain the binding specificity and affinity of the parent antibody. Identification of positions for possible back mutations can be performed using information available in the literature and in antibody databases. Amino acid residues that are candidates for back mutations are typically those that are located at the surface of an antibody molecule, while residues that are buried or that have a low degree of surface exposure will not normally be altered. An alternative humanization technique to CDR grafting and back mutation is resurfacing, in which non-surface exposed residues of non-human origin are retained, while surface residues are

altered to human residues. Another alternative technique is known as "guided selection" (Jespers et al. (1994) Biotechnology 12, 899) and can be used to derive from a murine antibody a fully human antibody conserving the epitope and binding characteristics of the parental antibody.

The frequency of appearance of the second form in various intact IgG isotypes is due to, but not limited to, structural differences associated with the hinge region isotype of the antibody. A single amino acid substitution in the hinge region of the human IgG4 hinge can significantly reduce the appearance of the second form (Angal et al., (1993) Molecular Immunology 30:105, incorporated by reference in its entirety) to levels typically observed using a human IgG1 hinge. The instant disclosure encompasses in various embodiments antibodies having one or more mutations in the hinge, CH2 or CH3 region which may be desirable, for example, in production, to improve the yield of the desired antibody form.

An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody." In various embodiments, the isolated antibody also includes an antibody in situ within a recombinant cell. In other embodiments, isolated antibodies are antibodies that have been subjected to at least one purification or isolation step. In various embodiments, an isolated antibody may be substantially free of other cellular material and/or chemicals.

The term "specifically binds," or the like, means that an antibody or antigen-binding fragment thereof forms a complex with an antigen that is relatively stable under physiologic conditions. Methods for determining whether an antibody specifically binds to an antigen are well known in the art and include, for example, equilibrium dialysis, surface plasmon resonance, and the like. For example, an antibody that "specifically binds" CEACAM5, as used herein, includes antibodies that bind CEACAM5 or portion thereof with a KD of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM or about 0.5 nM, as measured in a surface plasmon resonance assay. Specific binding can also be characterized by a dissociation constant of at least about $1x10^{-6}$ M or smaller. In other embodiments, the dissociation constant is at least about $1x10^{-7}$ M, $1x10^{-8}$ M, or $1x10^{-9}$ M. An isolated antibody that specifically binds human CEACAM5 may, however, have cross-reactivity to other antigens, such as CEACAM5 molecules from other (non-human) species.

The term "surface plasmon resonance", as used herein, refers to an optical phenomenon that allows for the analysis of real-time interactions by detection of alterations in protein concentrations within a biosensor matrix, for example using the BIACORE system (Biacore Life Sciences division of GE Healthcare, Piscataway, NJ).

The term "KD", as used herein, is intended to refer to the equilibrium dissociation constant of an antibody-antigen interaction.

"Affinity" is defined, in theory, by the equilibrium association between the whole antibody and the antigen. It can be experimentally assessed by a variety of known methods, such as measuring association and dissociation rates with surface plasmon resonance or measuring the EC50 (or apparent KD) in an immunochemical assay (ELISA, FACS). In these assays, the EC50 is the concentration of the antibody which induces a response halfway between the baseline and maximum after some specified exposure time on a defined concentration of antigen by ELISA (enzyme-linked immuno-sorbent assay) or cell expressing the antigen by FACS (Fluorescence Activated Cell Sorting).

A monoclonal antibody binding to antigen 1(Ag1) is "cross-reactive" to antigen 2 (Ag2) when the EC50s are in a similar range for both antigens. In the present application, a monoclonal antibody binding to Ag1 is cross-reactive to Ag2 when the ratio of affinity of Ag2 to affinity of Ag1 is equal or less than 10 (for instance 5, 2, 1 or 0.5), affinities being measured with the same method for both antigens.

Affinity for human CEACAM5 or for *Macaca fascicularis* CEACAM5 may be determined as the EC50 value in an ELISA using soluble recombinant CEACAM5 as capture antigen.

The antibody of the invention may also have an apparent dissociation constant (apparent KD), as may be determined by FACS analysis on tumor cell line MKN45 (DSMZ, ACC 409) or on xenograft tumor cells deriving from patient (CR-IGR-034P available from Oncodesign Biotechnology, tumor collection CReMEC), which is ≤25nM, for instance ≤20nM, ≤10nM, ≤5nM, ≤3nM or ≤1nM. The apparent KD may be within the range 0.01-20 nM, or may be within the range 0.1-20nM, 0.1-10nM, or 0.1-5nM.

Additionally, antibodies according to the invention have been shown to be able to detect CEACAM5 expression by immunohistochemistry in frozen and formalin-fixed and paraffin embedded (FFPE) tissue sections.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different

segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

The anti- CEACAM5 antibodies useful for the methods described herein may in various embodiments include one or more amino acid substitutions, insertions and/or deletions in the framework and/or CDR regions of the heavy and light chain variable domains as compared to the corresponding germline sequences from which the antibodies were derived. Such mutations can be readily ascertained by comparing the amino acid sequences disclosed herein to germline sequences available from, for example, public antibody sequence databases. The present disclosure includes in various embodiments methods involving the use of antibodies, and antigen-binding fragments thereof, which are derived from any of the amino acid sequences disclosed herein, wherein one or more amino acids within one or more framework and/or CDR regions are mutated to the corresponding residue(s) of the germline sequence from which the antibody was derived, or to the corresponding residue(s) of another human germline sequence, or to a conservative amino acid substitution of the corresponding germline residue(s) (such sequence changes are referred to herein collectively as "germline mutations"). Numerous antibodies and antigen-binding fragments may be constructed which comprise one or more individual germline mutations or combinations thereof. In certain embodiments, all of the framework and/or CDR residues within the VH and/or VL domains are mutated back to the residues found in the original germline sequence from which the antibody was derived. In other embodiments, only certain residues are mutated back to the original germline sequence, e.g., only the mutated residues found within the first 8 amino acids of FR1 or within the last 8 amino acids of FR4, or only the mutated residues found within CDR1, CDR2 or CDR3. In other embodiments, one or more of the framework and/or CDR residue(s) are mutated to the corresponding residue(s) of a different germline sequence (*i.e.*, a germline sequence that is different from the germline sequence from which the antibody was originally derived). Furthermore, the antibodies may contain any combination of two or more germline mutations within the framework and/or CDR regions, *e.g.*, wherein certain individual residues are mutated to the corresponding residue of a certain germline sequence while certain other residues that differ from the original germline sequence are maintained or are mutated to the corresponding residue of a different germline sequence. Once obtained, antibodies and antigen-binding fragments that contain one or more germline mutations can be easily tested for one or more desired property such as, improved binding specificity, increased binding affinity, improved or enhanced antagonistic or agonistic biological properties (as the case may be), reduced immunogenicity, etc. The use of antibodies and antigen-binding fragments obtained in this general manner are encompassed within the present disclosure.

The present disclosure also includes methods involving the use of anti-CEACAM5 antibodies comprising variants of any of the HCVR, LCVR, and/or CDR amino acid sequences disclosed herein having one or more conservative substitutions. For example, the present disclosure includes the use of anti- CEACAM5 antibodies having HCVR, LCVR, and/or CDR amino acid sequences with, *e.g.,* 10 or fewer, 8 or fewer, 6 or fewer, 4 or fewer, etc. conservative amino acid substitutions relative to any of the HCVR, LCVR, and/or CDR amino acid sequences disclosed herein.

According to the present disclosure, the anti-CEACAM5 antibody, or antigen-binding fragment thereof, in various embodiments comprises a heavy chain variable region (HCVR), light chain variable region (LCVR), and/or complementarity determining regions (CDRs) comprising any of the amino acid sequences of the anti-CEACAM5 antibodies described in Intl. Patent Pub. No. WO 2014/079886 A1, incorporated herein by reference in its entirety.

Amino acid sequence modification(s) of the antibodies described herein are contemplated. For example, it may be desirable to improve the binding affinity and/or other biological properties of the antibody. It is known that when a humanised antibody is produced by simply grafting only CDRs in VH and VL of an antibody derived from a non-human animal in FRs of the VH and VL of a human antibody, the antigen binding activity may be reduced in comparison with that of the original antibody derived from a non-human animal. It is considered that several amino acid residues of the VH and VL of the non-human antibody, not only in CDRs but also in FRs, may be directly or indirectly associated with the antigen binding activity. Hence, substitution of these amino acid residues with different amino acid residues derived from FRs of the VH and VL of the human antibody would reduce the binding activity. In order to solve the problem, in human antibodies grafted with non-human CDRs, attempts have to be made to identify, among amino acid sequences of the FR of the VH and VL of human antibodies, an amino acid residue which is directly associated with binding of the antibody, or which interacts with an amino acid residue of a CDR, or which maintains the three-dimensional structure of the antibody and which is directly associated with binding to the antigen. The reduced antigen binding activity could be increased by replacing the identified amino acids with amino acid residues of the original antibody derived from a non-human animal.

Modifications and changes may be made in the structure of the antibodies of the present invention, and in the DNA sequences encoding them, and still result in a functional antibody or polypeptide with desirable characteristics.

A further object of the present invention also encompasses function-conservative variants of the polypeptides of the present invention. For example, certain amino acids may be substituted by other amino acids in a protein structure without appreciable loss of activity. Since the interactive capacity and nature of a protein define its biological functional activity, certain amino acid substitutions can be made in a protein sequence, and of course in its DNA encoding sequence,

while nevertheless obtaining a protein with like properties. It is thus contemplated that various changes may be made in the antibodies sequences of the invention, or corresponding DNA sequences which encode said polypeptides, without appreciable loss of their biological activity. It is known in the art that certain amino acids may be substituted by other amino acids having a similar hydropathic index or score and still result in a protein with similar biological activity, *i.e.* still obtain a biological functionally equivalent protein. It is also possible to use well-established technologies, such as alanine-scanning approaches, to identify, in an antibody or polypeptide of the invention, all the amino acids that can be substituted without significant loss of binding to the antigen. Such residues can be qualified as neutral, since they are not involved in antigen binding or in maintaining the structure of the antibody. One or more of these neutral positions can be substituted by alanine or by another amino acid can without changing the main characteristics of the antibody or polypeptide of the invention.

Neutral positions can be seen as positions where any amino acid substitution could be incorporated to the antibodies. Indeed, in the principle of alanine-scanning, alanine is chosen since it this residue does not carry specific structural or chemical features. It is generally admitted that if an alanine can be substituted for a specific amino acid without changing the properties of a protein, many other, if not all amino acid substitutions are likely to be also neutral. In the opposite case where alanine is the wild-type amino acid, if a specific substitution can be shown as neutral, it is likely that other substitutions would also be neutral. As outlined above, amino acid substitutions are generally therefore based on the relative similarity of the amino acid side-chain substituents, for example, their hydrophobicity, hydrophilicity, charge, size, and the like. Exemplary substitutions which take any of the foregoing characteristics into consideration are well known to those of skill in the art and include: arginine and lysine; glutamate and aspartate; serine and threonine; glutamine and asparagine; and valine, leucine and isoleucine.

It may be also desirable to modify the antibody of the invention with respect to effector function, *e.g.* so as to enhance antigen-dependent cell-mediated cytotoxicity (ADCC) and/or complement dependent cytotoxicity (CDC) of the antibody. This may be achieved by introducing one or more amino acid substitutions in an Fc region of the antibody. Alternatively or additionally, cysteine residue(s) may be introduced in the Fc region, thereby allowing inter-chain disulfide bond formation in this region. The homodimeric antibody thus generated may have improved internalization capability and/or increased complement-mediated cell killing and/or antibody-dependent cellular cytotoxicity (ADCC) (Caron PC. *et al.* 1992; and Shopes B. 1992).

Another type of amino acid modification of the antibody of the invention may be useful for altering the original glycosylation pattern of the antibody, *i.e.* by deleting one or more carbohydrate moieties found in the antibody, and/or adding one or more glycosylation sites that are not present in the antibody. The presence of either of the tripeptide sequences asparagine-X-serine, and asparagine-X-threonine, where X is any amino acid except proline, creates a potential glycosylation site. Addition or deletion of glycosylation sites to the antibody is conveniently accomplished by altering the amino acid sequence such that it contains one or more of the above-described tripeptide sequences (for N-linked glycosylation sites).

Another type of modification involves the removal of sequences identified, either in silico or experimentally, as potentially resulting in degradation products or heterogeneity of antibody preparations. As examples, deamidation of asparagine and glutamine residues can occur depending on factors such as pH and surface exposure. Asparagine residues are particularly susceptible to deamidation, primarily when present in the sequence Asn-Gly, and to a lesser extent in other dipeptide sequences such as Asn-Ala. When such a deamidation site, in particular Asn-Gly, is present in an antibody or polypeptuide of the invention, it may therefore be desirable to remove the site, typically by conservative substitution to remove one of the implicated residues. Such substitutions in a sequence to remove one or more of the implicated residues are also intended to be encompassed by the present invention.

Another type of covalent modification involves chemically or enzymatically coupling glycosides to the antibody. These procedures are advantageous in that they do not require production of the antibody in a host cell that has glycosylation capabilities for N-or O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, orhydroxyproline, (e) aromatic residues such as those of phenylalanine, tyrosine, or tryptophan, or (f) the amide group of glutamine. For example, such methods are described in WO87/05330.

Removal of any carbohydrate moieties present on the antibody may be accomplished chemically or enzymatically. Chemical deglycosylation requires exposure of the antibody to the compound trifluoromethanesulfonic acid, or an equivalent compound. This treatment results in the cleavage of most or all sugars except the linking sugar (N-acetylglucosamine or N-acetylgalactosamine), while leaving the antibody intact. Chemical deglycosylation is described by Sojahr H. *et al.* (1987) and by Edge, AS. *et al.* (1981). Enzymatic cleavage of carbohydrate moieties on antibodies can be achieved by the use of a variety of endo-and exo-glycosidases as described by Thotakura, NR. *et al.* (1987).

Another type of covalent modification of the antibody comprises linking the antibody to one of a variety of non-proteinaceous polymers, *e.g.*, polyethylene glycol, polypropylene glycol, or polyoxyalkylenes, in the manner set forth in US Patent Nos. 4,640, 835; 4,496, 689; 4,301, 144; 4,670, 417; 4,791, 192 or 4,179,337.

In an embodiment, the antibody of the invention is antibody huMAb2-3 or a variant thereof. The different amino acid sequences for the huMAb2-3 antibody are shown below in SEQ ID NOs: 1-9.

The heavy chain variable domain amino acid sequence of SEQ ID NO: 1 is

EVQLQESGPGLVKPGGSLSLSCAASGFVFSSYDMSWVRQTPERGLEWVAYISSGGGITY
APSTVKGRFTVSRDNAKNTLYLQMNSLTSEDTAVYYCAAHYFGSSGPFAYWGQGTLVT
VSS.

The light chain variable domain amino acid sequence of SEQ ID NO: 2 is

DIQMTQSPASLSASVGDRVTITCRASENIFSYLAWYQQKPGKSPKLLVYNTRTLA
EGVPSRFSGSGSGTDFSLTISSLQPEDFATYYCQHHYGTPFTFGSGTKLEIK.

The CDR sequences of SEQ ID NOs: 1 and 2 are listed below and encompass SEQ ID NOs: 3-7.

The amino acid sequence of SEQ ID NO: 3 is GFVFSSYD.

The amino acid sequence of SEQ ID NO: 4 is ISSGGGIT.

The amino acid sequence of SEQ ID NO: 5 is AAHYFGSSGPFAY.

The amino acid sequence of SEQ ID NO: 6 is ENIFSY.

The amino acid sequence of light chain CDR2 is NTR.

The amino acid sequence of SEQ ID NO: 7 is QHHYGTPFT.

The heavy chain amino acid sequence of SEQ ID NO: 8 is

EVQLQESGPGLVKPGGSLSLSCAASGFVFSSYDMSWVRQTPERGLEWVAYISSGGGITY
APSTVKGRFTVSRDNAKNTLYLQMNSLTSEDTAVYYCAAHYFGSSGPFAYWGQGTLVT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLP
PSRDELTKNQVSLTCLVKGFYPSDIAVEWESNGQPENNYKTTPPVLDSDGSFFLYSKLTV
DKSRWQQGNVFSCSVMHEALHNHYTQKSLSLSPG. .

The light chain amino acid sequence of SEQ ID NO: 9 is

DIQMTQSPSSVSASVGDRVTITCRASQGISSWLAWYQQKPGKAPKLLIYGASSLE
SGVPSRFSGSGSGTDFTLTISSLQPEDFASYYCQQANSFPYTFGQGTKLEIKRTVAAPSVFI
FPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC.

According to the present disclosure, the antibodies disclosed herein bind to an epitope of of the A3-B3 domain of human CEACAM5 protein. The amino acid sequences of the A3-B3 domain epitopes bound by antibodies of the disclosure include SGANLNL (SEQ ID NO: 10), and INGIPQQHTQVLF (SEQ ID NO: 11).

The term "bioequivalent" as used herein, refers to a molecule having similar bioavailability (rate and extent of availability) after administration at the same molar dose and under similar conditions (e.g., same route of administration), such that the effect, with respect to both efficacy and safety, can be expected to be essentially same as the comparator molecule. Two pharmaceutical compositions comprising an anti-CEACAM5 antibody are bioequivalent if they are pharmaceutically equivalent, meaning they contain the same amount of active ingredient, in the same dosage form, for the same route of administration and meeting the same or comparable standards.

The disclosure in certain embodiments relates to methods comprising administering to the subject an antibody which comprises the heavy chain variable region comprising sequence SEQ ID NO: 1 and the light chain variable region comprising sequence SEQ ID NO: 2.

The disclosure provides pharmaceutical compositions comprising such antibody, and methods of using these compositions.

The antibody in various embodiments comprises the CDRs in the heavy chain variable region/domain of SEQ ID NO: 1 and the CDRs in light chain variable region/domain of SEQ ID NO: 2. The antibody in various embodiments comprises the heavy chain variable region comprising sequence SEQ ID NO: 1 and the light chain variable region comprising sequence SEQ ID NO: 2 is an antibody that specifically binds CEACAM5. See international publication number WO 2014/079886 A1, incorporated herein by reference in its entirety. In one embodiment, the antibody comprises the heavy chain variable region comprising sequence SEQ ID NO: 8 and the light chain variable region comprising sequence SEQ ID NO: 9.

## Immunoconjugates

The present invention also includes cytotoxic conjugates, or immunoconjugates, or antibody-drug conjugates, or conjugates. As used herein, all these terms have the same meaning and are interchangeable.

Accordingly, the invention relates to "immunoconjugates" comprising an antibody of the invention linked or conjugated to at least one growth inhibitory agent, such as a cytotoxic agent or a radioactive isotope.

A "growth inhibitory agent", or "anti-proliferative agent", which can be used indifferently, refers to a compound or composition which inhibits growth of a cell, especially tumour cell, either *in vitro* or *in vivo.*

The term "cytotoxic agent" as used herein refers to a substance that inhibits or prevents the function of cells and/or causes destruction of cells. The term "cytotoxic agent" is intended to include chemotherapeutic agents, enzymes, antibiotics, and toxins such as small molecule toxins or enzymatically active toxins of bacterial, fungal, plant or animal origin, including fragments and/or variants thereof, and the various antitumor or anticancer agents disclosed below. In some embodiments, the cytotoxic agent is a taxoid, vincas, a maytansinoid or maytansinoid analog such as DM1 or DM4, a small drug, a tomaymycin or pyrrolobenzodiazepine derivative, a cryptophycin derivative, a leptomycin derivative, an auristatin or dolastatin analog, a prodrug, topoisomerase II inhibitors, a DNA alkylating agent, an anti-tubulin agent, a CC-1065 or CC-1065 analog.

As used herein "maytansinoids" denotes maytansinoids and maytansinoid analogs. Maytansinoids are drugs that inhibit microtubule formation and that are highly toxic to mammalian cells.

Examples of suitable maytansinoids include maytansinol and maytansinol analogs.

Examples of suitable maytansinol analogues include those having a modified aromatic ring and those having modifications at other positions. Such suitable maytansinoids are disclosed in U.S. Patent Nos. 4,424,219; 4,256,746; 4,294,757; 4,307,016; 4,313,946; 4,315,929; 4,331,598; 4,361,650; 4,362,663; 4,364,866; 4,450,254; 4,322,348; 4,371,533; 6,333,410; 5,475,092; 5,585,499; and 5,846,545.

Specific examples of suitable analogues of maytansinol having a modified aromatic ring include:

(1) C-19-dechloro (U.S. Pat. No. 4,256,746) (prepared by LAH reduction of ansamytocin P2);
(2) C-20-hydroxy (or C-20-demethyl) +/-C-19-dechloro (U.S. Pat. Nos. 4,361,650 and 4,307,016) (prepared by demethylation using *Streptomyces* or *Actinomyces* or dechlorination using LAH); and
(3) C-20-demethoxy, C-20-acyloxy (-OCOR), +/-dechloro (U.S. Pat. No 4,294,757) (prepared by acylation using acyl chlorides).

Specific examples of suitable analogues of maytansinol having modifications of other positions include:

(1) C-9-SH (U.S. Pat. No. 4,424,219) (prepared by the reaction of maytansinol with $H_2S$ or $P_2S_5$);
(2) C-14-alkoxymethyl (demethoxy/$CH_2OR$) (U.S. Pat. No. 4,331,598);
(3) C-14-hydroxymethyl or acyloxymethyl ($CH_2OH$ or $CH_2OAc$) (U.S. Pat. No. 4,450,254) (prepared from *Nocardia*);
(4) C-15-hydroxy/acyloxy (U.S. Pat. No. 4,364,866) (prepared by the conversion of maytansinol by *Streptomyces*);
(5) C-15-methoxy (U.S. Pat. Nos. 4,313,946 and 4,315,929) (isolated from *Trewia nudiflora*);
(6) C-18-*N*-demethyl (U.S. Pat. Nos. 4,362,663 and 4,322,348) (prepared by the demethylation of maytansinol by *Streptomyces*); and
(7) 4,5-deoxy (U.S. Pat. No 4,371,533) (prepared by the titanium trichloride/LAH reduction of maytansinol).

In an embodiment of the invention, the cytotoxic conjugates of the present invention utilize the thiol-containing

maytansinoid (DM1), formally termed *N*$^{2'}$-deacetyl-*N*$^{2'}$-(3-mercapto-1-oxopropyl)-maytansine, as the cytotoxic agent. DM1 is represented by the following structural formula (I):

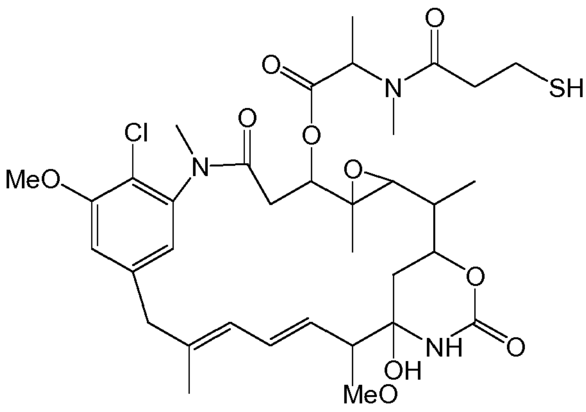

(I).

In another embodiment, the cytotoxic conjugates of the present invention utilize the thiol-containing maytansinoid DM4, formally termed *N*$^{2'}$-deacetyl-*N*-$^{2'}$(4-methyl-4-mercapto-1-oxopentyl)-maytansine, as the cytotoxic agent. DM4 is represented by the following structural formula (II):

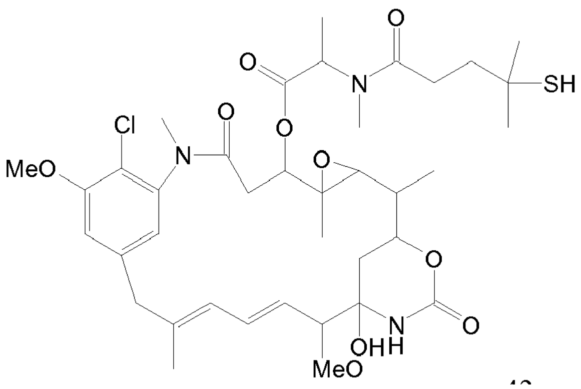

(II)

In further embodiments of the invention, other maytansines, including thiol and disulfide-containing maytansinoids bearing a mono or di-alkyl substitution on the carbon atom bearing the sulfur atom, may be used. These include a maytansinoid having, at C-3, C-14 hydroxymethyl, C-15 hydroxy, or C-20 desmethyl, an acylated amino acid side chain with an acyl group bearing a hindered sulfhydryl group, wherein the carbon atom of the acyl group bearing the thiol functionality has one or two substituents, said substituents being $CH_3$, $C_2H_5$, linear or branched alkyl or alkenyl having from 1 to 10 reagents and any aggregate which may be present in the solution.

Examples of these cytotoxic agents and of methods of conjugation are further given in the application WO2008/010101 which is incorporated by reference.

The term "radioactive isotope" is intended to include radioactive isotopes suitable for treating cancer, such as $At^{211}$, $Bi^{212}$, $Er^{169}$, $I^{131}$, $I^{125}$, $Y^{90}$, $In^{111}$, $P^{32}$, $Re^{186}$, $Re^{188}$, $Sm^{153}$, $Sr^{89}$, and radioactive isotopes of Lu. Such radioisotopes generally emit mainly beta-radiation.In an embodiment the radioactive isotope is alpha-emitter isotope, more precisely Thorium 227 which emits alpha-radiation. The immunoconjugates according to the present invention can be prepared as described in the application WO2004/091668.

In some embodiments, the antibodies of the present invention are covalently attached, directly or via a cleavable or non-cleavable linker, to at least one growth inhibitory agent.

"Linker", as used herein, means a chemical moiety comprising a covalent bond or a chain of atoms that covalently attaches a polypeptide to a drug moiety.

The conjugates may be prepared by *in vitro* methods. In order to link a drug or prodrug to the antibody, a linking group is used. Suitable linking groups are well known in the art and include disulfide groups, thioether groups, acid labile groups, photolabile groups, peptidase labile groups and esterase labile groups. Conjugation of an antibody of the invention with cytotoxic agents or growth inhibitory agents may be made using a variety of bifunctional protein coupling agents including but not limited to N-succinimidyl pyridyldithiobutyrate (SPDB), butanoic acid 4-[(5-nitro-2-pyridinyl)dithio]-2,5-dioxo-1-pyrrolidinyl ester (nitro-SPDB), 4-(Pyridin-2-yldisulfanyl)-2-sulfo-butyric acid (sulfo-SPDB), N-succinimidyl (2-pyridyldithio) propionate (SPDP), succinimidyl (N-maleimidomethyl) cyclohexane-1-carboxylate (SMCC), iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis (p-azidobenzoyl)-hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4- dinitrobenzene). For example, a ricin immunotoxin can be prepared as described in Vitetta et al (1987). Carbon labeled 1-isothiocyanatobenzyl methyldiethylene triaminepentaacetic acid (MX-DTPA) is an exemplary chelating agent for conjugation of radionucleotide to the antibody (WO 94/11026).

The linker may be a "cleavable linker" facilitating release of the cytotoxic agent or growth inhibitory agent in the cell. For example, an acid-labile linker, a peptidase-sensitive linker, an esterase labile linker, a photolabile linker or a disulfide-containing linker (See *e.g.* U.S. Patent No. 5,208,020) may be used. The linker may be also a "non-cleavable linker" (for example SMCC linker) that might lead to better tolerance in some cases.

Alternatively, a fusion protein comprising the antibody of the invention and a cytotoxic or growth inhibitory polypeptide may be made, by recombinant techniques or peptide synthesis. The length of DNA may comprise respective regions encoding the two portions of the conjugate either adjacent one another or separated by a region encoding a linker peptide which does not destroy the desired properties of the conjugate.

The antibodies of the present invention may also be used in Dependent Enzyme Mediated Prodrug Therapy by conjugating the polypeptide to a prodrug-activating enzyme which converts a prodrug (*e.g.* a peptidyl chemotherapeutic agent, see WO81/01145) to an active anti-cancer drug (See, for example, WO 88/07378 and U.S. Patent No. 4,975,278). The enzyme component of the immunoconjugate useful for ADEPT includes any enzyme capable of acting on a prodrug in such a way so as to convert it into its more active, cytotoxic form. Enzymes that are useful in the method of this invention include, but are not limited to, alkaline phosphatase useful for converting phosphate-containing prodrugs into free drugs; arylsulfatase useful for converting sulfate-containing prodrugs into free drugs; cytosine deaminase useful for converting non-toxic fluorocytosine into the anticancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as O-galactosidase and neuraminidase useful for converting glycosylated prodrugs into free drugs; P-lactamase useful for converting drugs derivatized with P- lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. The enzymes can be covalently bound to the polypeptides of the invention by techniques well known in the art such as the use of the heterobifunctional crosslinking reagents discussed above.

According to an embodiment, in the conjugate of the invention, the growth inhibitory agent is a maytansinoid, in an embodiment DM1 or DM4.

In said conjugate, the antibody is conjugated to said at least one growth inhibitory agent by a linking group. In an embodiment said linking group is a cleavable or a non-cleavable linker, such as SPDB, sulfo-SPDB, or SMCC.

The conjugate may be selected from the group consisting of:

an antibody-SPDB-DM4 conjugate of fomula (III)

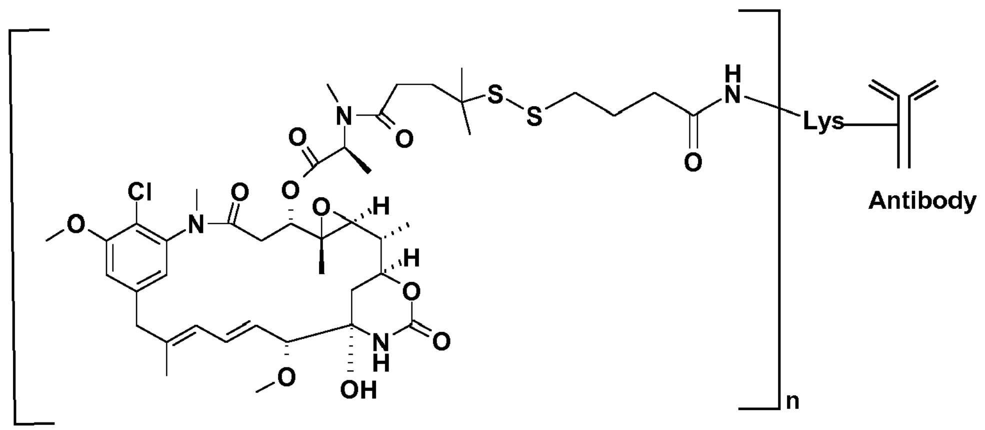

(III);

Ab-SPDB-DM4

an antibody-sulfo-SPDB-DM4 conjugate of fomula (IV)

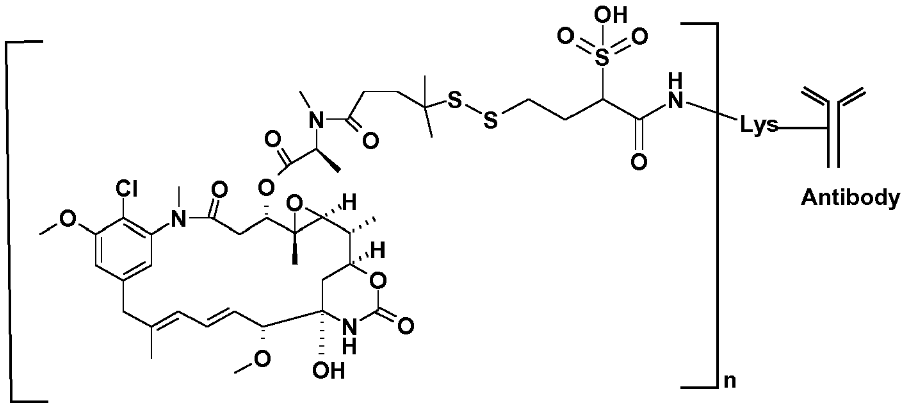

(IV);

**Ab-SulfoSPDB-DM4**

and

an antibody-SMCC-DM1 conjugate of fomula (V)

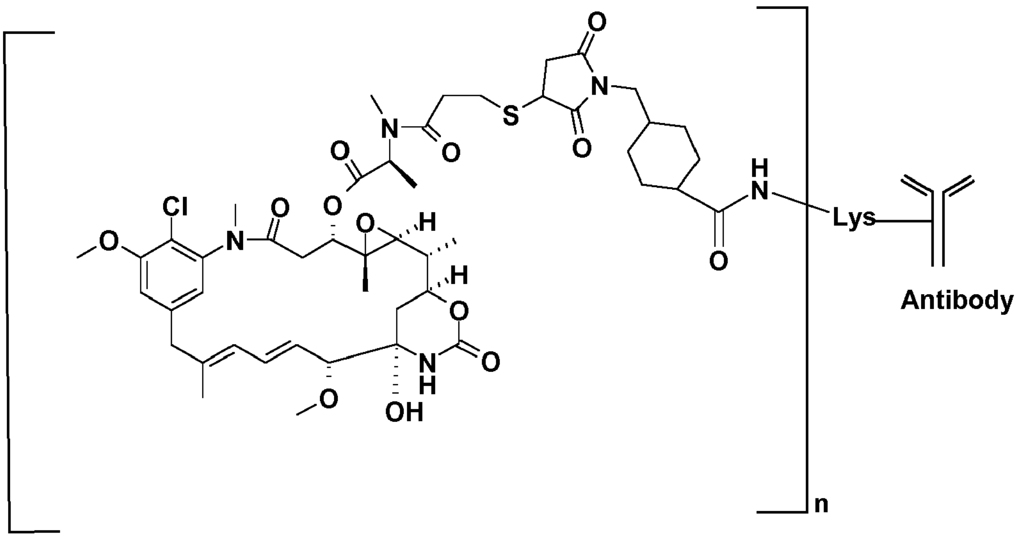

(V).

**Ab-SMCC-DM1**

In an embodiment the conjugate is a conjugate of formula (III), (IV) or (V) as defined above, in which the antibody is an antibody described herein.

In general, the conjugate can be obtained by a process comprising the steps of:

(i) bringing into contact an optionally-buffered aqueous solution of a cell-binding agent (*e.g.* an antibody according to the invention) with solutions of a linker and a cytotoxic compound;

(ii) then optionally separating the conjugate which was formed in (i) from the unreacted cell-binding agent.

The aqueous solution of cell-binding agent can be buffered with buffers such as, *e.g.* potassium phosphate, acetate, citrate or N-2-Hydroxyethylpiperazine-N'-2-ethanesulfonic acid (Hepes buffer). The buffer depends upon the nature of the cell-binding agent. The cytotoxic compound is in solution in an organic polar solvent, *e.g.* dimethyl sulfoxide (DMSO) or dimethylacetamide (DMA).

The reaction temperature is usually comprised between 20 and 40°C. The reaction time can vary from 1 to 24 hours. The reaction between the cell-binding agent and the cytotoxic agent can be monitored by size exclusion chromatography (SEC) with a refractometric and/or UV detector. If the conjugate yield is too low, the reaction time can be extended.

A number of different chromatography methods can be used by the person skilled in the art in order to perform the separation of step (ii): the conjugate can be purified *e.g.* by SEC, adsorption chromatography (such as ion exchange

chromatography, IEC), hydrophobic interaction chromatograhy (HIC), affinity chromatography, mixed-support chromatography such as hydroxyapatite chromatography, or high performance liquid chromatography (HPLC). Purification by dialysis or diafiltration can also be used.

As used herein, the term "aggregates" means the associations which can be formed between two or more cell-binding agents, said agents being modified or not by conjugation. The aggregates can be formed under the influence of a great number of parameters, such as a high concentration of cell-binding agent in the solution, the pH of the solution, high shearing forces, the number of bonded dimers and their hydrophobic character, the temperature (see Wang & Gosh, 2008, J. Membrane Sci., 318: 311-316, and references cited therein); note that the relative influence of some of these parameters is not clearly established. In the case of proteins and antibodies, the person skilled in the art will refer to Cromwell et al. (2006, AAPS Jounal, 8(3): E572-E579). The content in aggregates can be determined with techniques well known to the skilled person, such as SEC (see Walter et al., 1993, Anal. Biochem., 212(2): 469-480).

After step (i) or (ii), the conjugate-containing solution can be submitted to an additional step (iii) of chromatography, ultrafiltration and/or diafiltration.

The conjugate is recovered at the end of these steps in an aqueous solution.

According to an embodiment, the conjugate according to the invention is characterised by a "drug-to-antibody ratio" (or "DAR") ranging from 1 to 10, for instance from 2 to 5, in particular from 3 to 4. This is generally the case of conjugates including maytansinoid molecules.

This DAR number can vary with the nature of the antibody and of the drug (*i.e.* the growth-inhibitory agent) used along with the experimental conditions used for the conjugation (like the ratio growth-inhibitory agent/antibody, the reaction time, the nature of the solvent and of the cosolvent if any). Thus the contact between the antibody and the growth-inhibitory agent leads to a mixture comprising several conjugates differing from one another by different drug-to-antibody ratios; optionally the naked antibody; optionally aggregates. The DAR that is determined is thus a mean value.

A method which can be used to determine the DAR consists in measuring spectrophotometrically the ratio of the absorbance at of a solution of substantially purified conjugate at $\lambda_D$ and 280 nm. 280 nm is a wavelength generally used for measuring protein concentration, such as antibody concentration. The wavelength $\lambda_D$ is selected so as to allow discriminating the drug from the antibody, *i.e.* as readily known to the skilled person, $\lambda_D$ is a wavelength at which the drug has a high absorbance and $\lambda_D$ is sufficiently remote from 280 nm to avoid substantial overlap in the absorbance peaks of the drug and antibody. $\lambda_D$ may be selected as being 252 nm in the case of maytansinoid molecules. A method of DAR calculation may be derived from Antony S. Dimitrov (ed), LLC, 2009, Therapeutic Antibodies and Protocols, vol 525, 445, Springer Science:

The absorbances for the conjugate at $\lambda_D$ ($A_{\lambda D}$) and at 280 nm ($A_{280}$) are measured either on the monomeric peak of the size exclusion chromatography (SEC) analysis (allowing to calculate the "DAR(SEC)" parameter) or using a classic spectrophotometer apparatus (allowing to calculate the "DAR(UV)" parameter). The absorbances can be expressed as follows:

$$A_{\lambda D} = (c_D \text{ x } \varepsilon_{D\lambda D}) + (c_A \text{ x } \varepsilon_{A\lambda D})$$

$$A_{280} = (c_D \text{ x } \varepsilon_{D280}) + (c_A \text{ x } \varepsilon_{A280})$$

wherein :

$c_D$ and $c_A$ are respectively the concentrations in the solution of the drug and of the antibody
$\varepsilon_{D\lambda D}$ and $\varepsilon_{D280}$ are respectively the molar extinction coefficients of the drug at $\lambda_D$ and 280 nm
$\varepsilon_{A\lambda D}$ and $\varepsilon_{A280}$ are respectively the molar extinction coefficients of the antibody at $\lambda_D$ and 280 nm.

Resolution of these two equations with two unknowns leads to the following equations:

$$c_D = [(\varepsilon_{A280} \text{ x } A_{\lambda D}) - (\varepsilon_{A\lambda D} \text{ x } A_{280})] / [(\varepsilon_{D\lambda D} \text{ x } \varepsilon_{A280}) - (\varepsilon_{A\lambda D} \text{ x } \varepsilon_{D280})]$$

$$c_A = [A_{280} - (c_D \text{ x } \varepsilon_{D280})] / \varepsilon_{A280}$$

The average DAR is then calculated from the ratio of the drug concentration to that of the antibody: DAR = $c_D$ / $c_A$.

## Pharmaceutical compositions

The antibodies or immunoconjugates of the invention may be combined with pharmaceutically acceptable excipients, and optionally sustained-release matrices, such as biodegradable polymers, to form therapeutic compositions.

Thus, another object of the invention relates to a pharmaceutical composition comprising an antibody or an immunoconjugate of the invention and a pharmaceutically acceptable carrier or excipient.

The invention also relates to a polypeptide or an immunoconjugate according to the invention, for use as a medicament.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

As used herein, "pharmaceutically-acceptable carriers" includes any and all solvents, dispersion media, coatings, antibacterial and antifungal agents, and the like that are physiologically compatible. Examples of suitable carriers, diluents and/or excipients include one or more of water, amino acids, saline, phosphate buffered saline, buffer phosphate, acetate, citrate, succinate; amino acids and derivates such as histidine, arginine, glycine, proline, glycylglycine; inorganic salts NaCl, calcium chloride; sugars or polyalcohols such as dextrose, glycerol, ethanol, sucrose, trehalose, mannitol; surfactants such as Polysorbate 80, polysorbate 20, poloxamer 188; and the like, as well as combination thereof. In many cases, it will be preferable to include isotonic agents, such as sugars, polyalcohols, or sodium chloride in the composition, and formulation may also contain an antioxidant such as tryptamine and a stabilizing agent such as Tween 20.

The form of the pharmaceutical compositions, the route of administration, the dosage and the regimen naturally depend upon the condition to be treated, the severity of the illness, the age, weight, and gender of the patient, etc.

The pharmaceutical compositions of the invention can be formulated for a topical, oral, parenteral, intranasal, intravenous, intramuscular, subcutaneous or intraocular administration and the like.

In an embodiment, the pharmaceutical compositions contain vehicles which are pharmaceutically acceptable for a formulation capable of being injected. These may be isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical composition can be administrated through drug combination devices.

The doses used for the administration can be adapted as a function of various parameters, and for instance as a function of the mode of administration used, of the relevant pathology, or alternatively of the desired duration of treatment.

To prepare pharmaceutical compositions, an effective amount of the antibody or immunoconjugate of the invention may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and injectable with the appropriate device or system for delivery without degradation. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant. Dispersions can also be prepared in glycerol, liquid polyethylene glycols, and mixtures thereof and in oils. Under ordinary conditions of storage and use, these preparations contain a preservative to prevent the growth of microorganisms.

A polypeptide, antibody or immunoconjugate of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, glycine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the

use in the compositions of agents delaying absorption, for example, aluminum monostearate and gelatin.

Sterile injectable solutions are prepared by incorporating the active compounds in the required amount in the appropriate solvent with any of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more concentrated, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The antibody or immunoconjugate of the invention may be formulated within a therapeutic mixture to comprise about 0.01 to 100 milligrams, per dose or so.

In addition to the antibody or immunoconjugate formulated for parenteral administration, such as intravenous or intramuscular injection, other pharmaceutically acceptable forms include, e.g., tablets or other solids for oral administration; time release capsules; and any other form currently used.

In certain embodiments, the use of liposomes and/or nanoparticles is contemplated for the introduction of polypeptides into host cells. The formation and use of liposomes and/or nanoparticles are known to those of skill in the art.

Nanocapsules can generally entrap compounds in a stable and reproducible way. To avoid side effects due to intracellular polymeric overloading, such ultrafine particles (sized around 0.1 μm) are generally designed using polymers able to be degraded *in vivo.* Biodegradable polyalkyl-cyanoacrylate nanoparticles, or biodegradable polylactide or polylactide co glycolide nanoparticules that meet these requirements are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 μm. Sonication of MLVs results in the formation of small unilamellar vesicles (SUVs) with diameters in the range of 200 to 500 Å, containing an aqueous solution in the core. The physical characteristics of liposomes depend on pH, ionic strength and the presence of divalent cations.

**Methods of Administration and Formulations**

The methods described herein comprise administering a therapeutically effective amount of an anti-CEACAM5 antibody to a subject. As used herein, an "effective amount" or "therapeutically effective amount" is a dose of the therapeutic that results in treatment of lung cancer (*e.g.*, NSQ NSCLC). As used herein, "treating" refers to causing a detectable improvement in one or more symptoms associated with lung cancer or causing a biological effect *(e.g.,* a decrease in the level of a particular biomarker) that is correlated with the underlying pathologic mechanism(s) giving rise to the condition or symptom(s). For example, a dose of anti-CEACAM5 antibody which causes an improvement in any of the following symptoms or conditions associated with lung cancer is deemed a "therapeutically effective amount": In another example, a treatment has not been effective when a dose of anti-CEACAM5 antibody does not result in a detectable improvement in one or more parameters or symptoms associated with cancer (*e.g.*, lung cancer) or which does not cause a biological effect that is correlated with the underlying pathologic mechanism(s) giving rise to the condition or symptom(s) of cancer.

According to some of these embodiments, the anti-CEACAM5 antibody is administered intravenously.

In accordance with the methods of the present invention, a therapeutically effective amount of anti-CEACAM5 antibody that is administered to the subject will vary depending upon the age and the size (*e.g.,* body weight or body surface area) of the subject as well as the route of administration and other factors well known to those of ordinary skill

in the art.

In certain embodiments, the dose of the antibody varies depending on the body surface area of the subject. In certain embodiments, the dose of anti-CEACAM5 antibody administered to the subject is from about 1 $mg/m^2$ to about 500 $mg/m^2$. In some embodiments, the dose of the antibody administered to the subject is from about 5 mg to about 300 $mg/m^2$. In various embodiments, the dose of the antibody administered to the subject is from about 5 to about 250 $mg/m^2$. In various embodiments, the dose is about 5, 10, 20, 30, 40, 60, 80, 100, 120, 150, 180, or 210 $mg/m^2$ based on the body surface area of the subject. In various embodiments, the antibody is administered at a dose of about 2.5 $mg/m^2$ to about 5 $mg/m^2$. For example, the antibody is administered for a period of time (*e.g.,* 30 minutes and one hour) at a dose of about 2.5 $mg/m^2$ to about 5 $mg/m^2$. The dose includes 2.5 $mg/m^2$ of the antibody, 5 $mg/m^2$ of the antibody and all doses in between 2.5 $mg/m^2$ and 5 $mg/m^2$, for example 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, and 4.9 $mg/m^2$.

For example, the present invention includes (but is not limited to) methods wherein about 1 $mg/m^2$, about 5 $mg/m^2$, 10 $mg/m^2$, about 15 $mg/m^2$, about 20 $mg/m^2$, about 25 $mg/m^2$, about 30 $mg/m^2$, about 35 $mg/m^2$, about 40 $mg/m^2$, about 45 $mg/m^2$, about 50 $mg/m^2$, about 55 $mg/m^2$, about 60 $mg/m^2$, about 65 $mg/m^2$, about 70 $mg/m^2$, about 75 $mg/m^2$, about 80 $mg/m^2$, about 85 $mg/m^2$, about 90 $mg/m^2$, about 95 $mg/m^2$, about 100 $mg/m^2$, about 105 $mg/m^2$, about 110 $mg/m^2$, about 115 $mg/m^2$, about 120 $mg/m^2$, about 125 $mg/m^2$, about 130 $mg/m^2$, about 135 $mg/m^2$, about 140 $mg/m^2$, about 145 $mg/m^2$, about 150 $mg/m^2$, about 155 $mg/m^2$, about 160 $mg/m^2$, about 165 $mg/m^2$, about 170 $mg/m^2$, about 175 $mg/m^2$, about 180 $mg/m^2$, about 185 $mg/m^2$, about 190 $mg/m^2$, about 195 $mg/m^2$, about 200 $mg/m^2$, about 205 $mg/m^2$, about 210 $mg/m^2$, about 215 $mg/m^2$, about 220 $mg/m^2$, about 225 $mg/m^2$, about 230 $mg/m^2$, about 235 $mg/m^2$, about 240 $mg/m^2$, about 245 $mg/m^2$, about 250 $mg/m^2$, about 255 $mg/m^2$, about 260 $mg/m^2$, about 265 $mg/m^2$, about 270 $mg/m^2$, about 275 $mg/m^2$, about 280 $mg/m^2$, about 285 $mg/m^2$, about 290 $mg/m^2$, about 295 $mg/m^2$, about 300 $mg/m^2$, about 325 $mg/m^2$, about 350 $mg/m^2$, about 375 $mg/m^2$, about 400 $mg/m^2$, about 425 $mg/m^2$, about 450 $mg/m^2$, about 475 $mg/m^2$, or about 500 $mg/m^2$ of anti- CEACAM5 antibody is administered to the patient per week or once every two weeks. In various embodiments, the antibody is administered at about 5, 10, 20, 30, 40, 60, 80, 100, 120, 150, 180, or 210 mg/m2 every two week based on the body surface area of the subject.

As used herein, "administered from about 1 to about 500 mg/kg" means that the referred to substance is administered at any value within the stated range including the endpoints of the range. For example, "the dose of anti-CEACAM5 antibody administered to the patient is from 1 $mg/m^2$ to 500 $mg/m^2$," includes administration of 1 $mg/m^2$ of the anti-CEACAM5 antibody, 500 $mg/m^2$ of the anti-CEACAM5 antibody and all doses in between. In an embodiment, the CEACAM5 antibody is administered at a dose of about 5, 10, 20, 30, 40, 60, 80, 100, 120, 150, 180, or 210 $mg/m^2$ over a period of time, for example once every 14 days (*i.e.,* every two weeks) or 3 weeks. In various embodiments of the method, the antibody is used at a dose listed in Example 1.

In various embodiments, the dose is administered at a constant rate. Alternatively, the dose is administered at a variable rate. In various embodiments, the dose is administered at a constant rate of about 0.5, 1, 1.5, 2, 2.5, 3, 3.5, 4, 2.5 or 5 mg/min. In various embodiments, the antibody is administered at a rate for the first 30 minutes or for the first 1 hour. In various embodiments, after about 30 minutes or 1 hour, the rate of administration of the antibody is changed. For example, the rate is decreased. In various embodiments, the rate is increased. In various embodiments, the antibody is administered at a rate of 2.5 mg/min for the first 30 minutes or 1 hour. In various embodiments, after about 30 minutes or 1 hour, the rate of administration of the antibody is increased to 5 mg/min.

The methods of the present invention include administering multiple doses of an anti-CEACAM5 antibody to a patient over a specified time course. For example, the anti-CEACAM5 antibody can be administered about 1 to 5 times per day, about 1 to 5 times per week, about 1 to 5 times every two weeks, about 1 to 5 times per month or about 1 to 5 times per year. In certain embodiments, the methods of the invention include administering a first dose of anti-CEACAM5 antibody to a patient at a first time point, followed by administering at least a second dose of anti-CEACAM5 antibody to the patient at a second time point. The first and second doses, in certain embodiments, may contain the same amount of anti-CEACAM5 antibody. The time between the first and second doses may be from about a few hours to several weeks. For example, the second time point (*i.e.,* the time when the second dose is administered) can be from about 1 hour to about 7 weeks after the first time point (*i.e.,* the time when the first dose is administered). According to certain exemplary embodiments of the present invention, the second time point can be about 1 hour, about 4 hours, about 6 hours, about 8 hours, about 10 hours, about 12 hours, about 24 hours, about 2 days, about 3 days, about 4 days, about 5 days, about 6 days, about 7 days, about 2 weeks, about 3 weeks, about 4 weeks, about 6 weeks, about 8 weeks, about 10 weeks, about 12 weeks, about 14 weeks or longer after the first time point. In certain embodiments, the second time point is about 1 week or about 2 weeks. Third and subsequent doses may be similarly administered throughout the course of treatment of the patient. The invention provides methods of using therapeutic compositions comprising anti-CEACAM5 antibodies or antigen-binding fragments thereof and, optionally, one or more additional therapeutic agents. The therapeutic compositions of the invention will be administered with suitable carriers, excipients, and other agents that are incorporated into formulations to provide improved transfer, delivery, tolerance, and the like. A multitude of appropriate formulations can be found in the formulary known to all pharmaceutical chemists: Remington's Pharmaceutical Sciences, Mack Publishing Company, Easton, PA, incorporated herein by reference in its entirety. These formulations include, for example, powders, pastes, ointments, jellies, waxes, oils, lipids, lipid (cationic or anionic) containing vesicles (such as LIPOFECTIN), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311, incorporated herein by reference in its entirety.

Various delivery systems are known and can be used to administer the pharmaceutical composition of the invention, *e.g.*, encapsulation in liposomes, microparticles, microcapsules, receptor mediated endocytosis (see, *e.g.,* Wu et al. (1987) J. Biol. Chem. 262:4429-4432, incorporated herein by reference in its entirety). Methods of introduction include, but are not limited to, intradermal, intramuscular, intraperitoneal, intravenous, subcutaneous, intranasal, epidural, and oral routes. The composition may be administered by any convenient route, for example by infusion or bolus injection, by absorption through epithelial or mucocutaneous linings (*e.g.,* oral mucosa, rectal and intestinal mucosa, *etc.*) and may be administered together with other biologically active agents. Administration can be systemic or local. The CEACAM5 antibody can be administered subcutaneously.

The pharmaceutical composition can also be delivered in a vesicle, such as a liposome (see Langer (1990) Science 249:1527-1533, incorporated herein by reference in its entirety). In certain situations, the pharmaceutical composition can be delivered in a controlled release system, for example, with the use of a pump or polymeric materials. In another embodiment, a controlled release system can be placed in proximity of the composition's target, thus requiring only a fraction of the systemic dose.

The injectable preparations may include dosage forms for intravenous, subcutaneous, intracutaneous and intramuscular injections, local injection, drip infusions, *etc.* These injectable preparations may be prepared by methods publicly known. For example, the injectable preparations may be prepared, *e.g.,* by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, *etc.,* which may be used in combination with an appropriate solubilizing agent such as an alcohol (*e.g.*, ethanol), a polyalcohol (*e.g.*, propylene glycol, polyethylene glycol), a nonionic surfactant [*e.g.,* polysorbate 80, HCO-50 (polyoxyethylene (50 mol) adduct of hydrogenated castor oil)], *etc.*). As the oily medium, there are employed, *e.g.,* sesame oil, soybean oil, *etc.,* which may be used in combination with a solubilizing agent such as benzyl benzoate, benzyl alcohol, *etc.* The injection thus prepared can be filled in an appropriate ampoule.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, *etc.*

In accordance with the methods disclosed herein, the anti-CEACAM5 antibody (or pharmaceutical formulation comprising the antibody) can be administered to the patient using any acceptable device or mechanism. For example, the administration can be accomplished using a syringe and needle or with a reusable pen and/or autoinjector delivery device. The methods of the present invention include the use of numerous reusable pen and/or autoinjector delivery devices to administer an anti-CEACAM5 antibody (or pharmaceutical formulation comprising the antibody). Examples of such devices include, but are not limited to AUTOPEN (Owen Mumford, Inc., Woodstock, UK), DISETRONIC pen (Disetronic Medical Systems, Bergdorf, Switzerland), HUMALOG MIX 75/25 pen, HUMALOG pen, HUMALIN 70/30 pen (Eli Lilly and Co., Indianapolis, IN), NOVOPEN I, II and III (Novo Nordisk, Copenhagen, Denmark), NOVOPEN JUNIOR (Novo Nordisk, Copenhagen, Denmark), BD pen (Becton Dickinson, Franklin Lakes, NJ), OPTIPEN, OPTIPEN PRO, OPTIPEN STARLET, and OPTICLIK (Sanofi-Aventis, Frankfurt, Germany). Examples of disposable pen and/or autoinjector delivery devices having applications in subcutaneous delivery of a pharmaceutical composition of the present invention include, but are not limited to the SOLOSTAR pen (Sanofi-Aventis), the FLEXPEN (Novo Nordisk), and the KWIKPEN (Eli Lilly), the SURECLICK Autoinjector (Amgen, Thousand Oaks, CA), the PENLET (Haselmeier, Stuttgart, Germany), the EPIPEN (Dey, L.P.), and the HUMIRA Pen (AbbVie Inc., North Chicago, IL), to name only a few.

In one embodiment, the antibody is administered with a prefilled syringe. In another embodiment, the antibody is administered with a prefilled syringe containing a safety system. For example, the safety system prevents an accidental needle-stick injury. In various embodiments, the antibody is administered with a prefilled syringe containing an ERIS safety system (West Pharmaceutical Services Inc.). See also U.S. patent numbers 5,215,534 and 9,248,242, incorporated herein by reference in their entireties.

In another embodiment, the antibody is administered with an auto-injector. In various embodiments, the antibody is administered with an auto-injector featuring the PUSHCLICK technology (SHL Group). In various embodiments, the autoinjector is a device comprising a syringe that allows for administration of a dose of the composition and/or antibody to a subject. See also U.S. patent numbers 9,427,531 and 9,566,395, incorporated herein by reference in their entireties.

The use of a microinfusor to deliver an anti-CEACAM5 antibody (or pharmaceutical formulation comprising the antibody) to a patient is also contemplated herein. As used herein, the term "microinfusor" means a subcutaneous delivery device designed to slowly administer large volumes (*e.g.*, up to about 2.5 mL or more) of a therapeutic formulation

over a prolonged period of time (*e.g.,* about 10, 15, 20, 25, 30 or more minutes). See, *e.g.,* U.S. 6,629,949; US 6,659,982; and Meehan et al., J. Controlled Release 46:107-116 (1996), incorporated herein by reference in their entireties. Microinfusors are particularly useful for the delivery of large doses of therapeutic proteins contained within high concentration and/or viscous solutions.

All publications mentioned herein are incorporated herein by reference in their entirety for all purposes.

## EXAMPLES

### Example 1: TED13751 - A first-in-human study for the evaluation of the safety pharmacokinetics and antitumor activity of huMAb2-3-SPDB-DM4 in patients with advanced solid tumors - Study Design

A first-in-human (FIH) clinical study for the evaluation of the safety and pharmacokinetic of the ADC identified as huMAb2-3-SPDB-DM4 administered as a single agent every 2 weeks (q2w; 14 days) through the IV route, was conducted in adults with advanced, unresectable or metastatic solid tumors.

The study was divided in two parts: the Escalation Phase and the Expansion Phase.

During the Escalation Phase, the population to be treated was enriched, but not restricted, for patients with tumor types known to express CEACAM5; confirmation of the CEACAM5 expression was retrospectively using IHC on the most recent archival tissue samples and in a central laboratory. Expression of circulating CEACAM5 was also used for enrichment.

During the Expansion Phase, the population to be treated was restricted to patients with NSQ-NSCLC having CEACAM5 expression of 22+ in intensity involving 250% of the tumor cell population was documented during pre-screening on the most recent archival tissue sample and using local IHC evaluation for patients potentially eligible for study treatment in the gastric adenocarcinoma cohort.

There were 2 independent NSQ-NSCLC expansion phase cohorts: The first one **(Lung cohort),** included patients with CEACAM5 expression of 22+ in intensity involving at least 50% of the tumor cell population. The second cohort **(Lung bis cohort)** included patients that pre-screened positive at the intensity 22+ between 21% to <50% of the tumor cell population. For investigational centers that did not have the CEACAM5 assay-based on huMAb2-3-SPDB-DM4 monoclonal antibody - implemented on their local IHC platform a pre-screening assessment of tumor CEACAM5 expression was conducted centrally. Full screening was done for archival cases meeting the above definition.

The level of CEACAM5 expression was documented essentially retrospectively and centrally on both archival and fresh (baseline sample) tumor tissues.

Confirmation of CEACAM5 tumor expression was done retrospectively in a central laboratory on fresh tumor tissue collected at baseline (mandatory biopsy in the Expansion Phase only, on NSQ-NSCLC. If sufficient archived tumor material was available, a central evaluation was also done retrospectively to gain knowledge on the variability in evaluation of the expression. The results of the retrospective analyses had no impact on patient's treatment. It was for the better interpretation of the overall response, and as the baseline for comparison with CEACAM5 expression upon progression (exploration for loss of CEACAM5 as mechanism of acquired resistance).

A maximum of 60 patients were included in the NSQ-NSCLC (Lung) cohort and a maximum of 28 patients were included in the NSQ-NSCLC (Lung bis) cohort. Only patients with measurable malignant disease were eligible. NSQ-NSCLC patients with malignant disease meeting strict CEACAM5 expression criteria during the pre-screening procedure were further screened for his or her eligibility to be treated at the MTD without the loading dose.

The safety of the first 6 patients enrolled in the expansion phase was reviewed by the Study Committee, when the 6th patient had been treated for two cycles, before enrolling the next patients. The MTD (without the loading dose) was confirmed if a third or less of the treated patients (enrolled in the 3 cohorts) had experienced DLT at the end of Cycle 2 at the planned dose of huMAb2-3-SPDB-DM4. At the same time-point a preliminary evaluation was on presence or absence of benefit from primary corneal toxicity prophylaxis in preventing corneal toxicity. In addition, occurrence of cumulative toxicity, if any, was assessed. The antitumor activity of the drug was evaluated, according to RECIST 1.1.

The duration of the study for an individual patient included a period for inclusion of up to 4 weeks (baseline period), a treatment period of at least 1 cycle (2 weeks), an end-of-treatment (EOT) visit around 30 days following the last IMP administration, and at least one follow-up visit (around 30 days after EOT visit) for immunogenicity evaluation.

## INCLUSION CRITERIA

I1. Locally advanced or metastatic solid malignant tumor disease for which, in the judgement of the Investigator, no standard alternative therapy is available and meeting the following inclusion criterias.

I2. At least 6 x 5 μm slides plus an additional number of slides that can be either 3 x 10 μm (best), or 6 x 5 μm, or

equivalent to keep the same total amount of material needed, from FFPE archival tissue should be available for local testing at the site and/or shipment to the Sponsor, or laboratory designated by the Sponsor, evaluation of tumor CEACAM5 expression (retrospectively in the Escalation Phase and prospectively in the Expansion Phase) and exploration of other predictive biomarkers of response. If less material was available, patient could still be eligible after discussion with the Sponsor who assessed and confirmed that there was sufficient relevant material for key evaluations.

I3. For participants to the Escalation Phase cohorts (Main and bis): Inclusion was enriched for (although not restricted to) tumors expressing or likely to be expressing CEACAM5 which included malignant diseases with high prevalence of CEACAM5 expression *i.e.,* NSQ-NSCLC. For participants to the Expansion Phase cohorts inclusion was restricted to patients with either NSQ-NSCLC sub-types or other lung cancer subtypes. There were two independent cohorts in NSQ-NSCLC expansion phase, based on local or central CEACAM5 expression assessment on archival tumor tissue: The first one (Lung) that started before amendment #4, included patients with CEACAM5 expression at 22+ in intensity involving at least 50% of the tumor cell population. The second independent cohort (Lung bis) included patients that pre-screened positive at the intensity of 22+ in 21% to <50% of the tumor cell population.

I4. At least one measurable lesion by RECIST v1.1 in the Expansion Phase only.

I5. At least one lesion amenable to biopsy (Expansion cohort only). Patient must consent to a baseline biopsy for retrospective confirmation of tumor CEACAM5 expression before treatment initiation, except if NSCLC or SCLC without lesion amenable to biopsy).

## INVESTIGATIONAL PRODUCT

Pharmaceutical form

The huMAb2-3-SPDB-DM4 ADC was supplied as a 25 mL extractable volume of concentrate for solution for infusion of 125 mg (5 mg/mL) contained in a 30 mL type I glass vial.

Dose of drug per administration

The starting DL is 5 mg/m2 to be administered every 2 weeks.

The patient's body surface area (BSA) was calculated using their height and actual body weight. For patients with a BSA >2.2 m2, the dose will be calculated on the basis of 2.2 m2 BSA.

Pre-medication with Histamine H1 antagonist (diphenylhydramine 50 mg PO or equivalent [*e.g.*, dexchlorpheniramine] given approximately 1 hour before huMAb2-3-SPDB-DM4 administration) is required for all patients.

Using an infusion controlled pump, huMAb2-3-SPDB-DM4 will be administered by IV infusion at a rate of 2.5 mg/min for the first 30 minutes and then increased to 5 mg/min in the absence of hypersensitivity reactions.

The exact dose and time of IMP administered (day/month/year, h:min) will be documented in the eCRF.

### Duration of treatment: 14 days

The huMAb2-3-SPDB-DM4 was administered on Day 1, and repeated every 14 days; this period of 14 days constituted one treatment cycle (1 cycle). Patients may continue treatment until disease progression, unacceptable toxicity, or willingness to stop.

### Dilution and infusion method

No bacteriostatic agent was present in the product; therefore, adherence to aseptic technique was required. Prior to dosing, each patient's dose needs to be individually prepared by the study pharmacist starting from pre-filled bags of diluent (0.9% sodium chloride).

Once the solution is prepared, the dose was administered to the patient within 7.5 hours from the bag preparation to the end of the dose infusion.

Two types of administration were used:

- Infusion by syringe driver for low doses (up to 30 mg/m2).
- Infusion by pump for other doses.

An IV tubing administration set with a 0.2 micron filter unit attached to it was used for infusion. The study drug was not administered with any other IV fluids. However, infusion tubing was optionally primed with normal saline or huMAb2-3-SPDB-DM4. For infused volumes ≤25 mL, a flush and destruction of 25 mL of huMAb2-3-SPDB-DM4 needed

to be ensured before infusion of the dose. At the end of the infusion by pump, the IV line was flushed with normal saline as needed to ensure delivery of the full dose. At the end of the infusion by syringe driver, the remaining quantity of huMAb2-3-SPDB-DM4 in the syringe was destroyed.

## TUMOR RESPONSE EVALUATION

To assess objective response or future progression, it was necessary to estimate the overall tumour burden at baseline and use this as a comparator for subsequent measurements. Only patients with measurable disease at baseline were included in protocols where objective tumour response was the primary endpoint. Measurable disease was defined by the presence of at least one measurable lesion. In studies where the primary endpoint was tumour progression (either time to progression or proportion with progression at a fixed date), the protocol specificied if entry is restricted to those with measurable disease or whether patients having non-measurable disease only are also eligible. See Table 2 and Table 3.

### Response Criteria

**Table 2**
**Evaluation of target lesions**

| | |
|---|---|
| Complete Response (CR) | Disappearance of all target lesions. Any pathological lymph nodes (whether target or non-target) must have reduction in short axis to <10 mm. |
| Partial Response (PR): | At least a 30% decrease in the sum of diameters of target lesions, taking as reference the baseline sum diameters. |
| Progressive Disease (PD): | At least a 20% increase in the sum of diameters of target lesions, taking as reference the smallest sum on study (this includes the baseline sum if that is the smallest on study). In addition to the relative increase of 20%, the sum must also demonstrate an absolute increase of at least 5 mm. (Note: the appearance of one or more new lesions is also considered progression). |
| Stable Disease (SD): | Neither sufficient shrinkage to qualify for PR nor sufficient increase to qualify for PD, taking as reference the smallest sum diameters while on study |

**Table 3**
**Evaluation of non-target lesions**

| | |
|---|---|
| | Disappearance of all non-target lesions and normalisation of tumour marker level. All lymph nodes must be non-pathological in size (<10 mm short axis). |
| Incomplete | Persistence of one or more non-target lesion(s) and/or maintenance of tumour marker level |
| Response/Stable Disease (SD): | above the normal limits. |
| Progressive Disease (PD): | Unequivocal progression (see comments below) of existing non-target lesions. (Note: the appearance of one or more new lesions is also considered progression). |

Though a clear progression of "non-target" lesions only is exceptional, in such circumstances, the opinion of the treating physician. Should prevail and the progression status should be confirmed later on by the review panel (or study chair).

## EVALUATION OF BEST OVERALL RESPONSE

The following table provides a summary of the overall response status calculation at each time point for patients who have measurable disease at baseline.

**Table 4**

| Target lesions | Non-target lesions | New lesions | Overall response |
|---|---|---|---|
| CR | CR | No | CR |

(continued)

| Target lesions | Non-target lesions | New lesions | Overall response |
|---|---|---|---|
| CR | Non-CR/non-PD | No | PR |
| CR | Not evaluated | No | PR |
| PR | Non-PD or not all evaluated | No | PR |
| SD | Non-PD or not all evaluated | No | SD |
| Not all evaluated | Non PD | No | NE |
| PD | Any | Yes or No | PD |
| Any | PD | Yes or No | PD |
| Any | Any | Yes | PD |

**Table 5**

| Overall response First time point | Overall response Subsequent time point | BEST overall response |
|---|---|---|
| CR | CR | CR |
| CR | PR | SD, PD or PR*a* |
| CR | SD | SD provided minimum criteria for SD duration met, otherwise, |
| PD | | |
| CR | PD | SD provided minimum criteria for SD duration met, otherwise, |
| PD | | |
| CR | NE | SD provided minimum criteria for SD duration met, otherwise |
| NE | | |
| PR | CR | PR |
| PR | PR | PR |
| PR | SD | SD |
| PR | PD | SD provided minimum criteria for SD duration met, otherwise, |
| PD | | |
| PR | NE | SD provided minimum criteria for SD duration met, otherwise |
| NE | | |
| NE | NE | NE |

*a* If a CR is truly met at first time point, then any disease seen at a subsequent time point, even disease meeting PR criteria relative to baseline, makes the disease PD at that point (since disease must have reappeared after CR). Best response would depend on whether minimum duration for SD was met. However, sometimes 'CR' may be claimed when subsequent scans suggest small lesions were likely still present and in fact the patient had PR, not CR at the first time point. Under these circumstances, the original CR should be changed to PR and the best response is PR.

The best overall response was determined once all the data for the patient was known. Best response determination in trials where confirmation of complete or partial response was not required: Best response in these trials is defined as the best response across all time points (for example, a patient who has SD at first assessment, PR at second assessment, and PD on last assessment has a best overall response of PR). When SD is believed to be best response, it must also meet the protocol specified minimum time from baseline. If the minimum time is not met when SD is otherwise the best time point response, the patient's best response depends on the subsequent assessments. For example, a patient who has SD at first assessment, PD at second and does not meet minimum duration for SD, will have a best response of PD. The same patient lost to follow-up after the first SD assessment would be considered non-evaluable.

Best response determination in trials where confirmation of complete or partial response was required: Complete or partial responses may be claimed only if the criteria for each are met at a subsequent time point as specified in the protocol (generally 4 weeks later). In this circumstance, the best overall response was interpreted as in Table 5.

**Example 2: TED13751 - Expansion Phase - LUNG HIGH Cohort - Primary Phase Results**

A discussed in Example 1, there were two independent cohorts in the non-sqNSCLC expansion phase, based on local or central CEACAM5 expression assessment on archival tumor tissue: the first cohort included patients with CEACAM5 expression at ≥2+ in intensity involving at least 50% of the tumor cell population. The second independent cohort (Lung bis) included patients that pre-screened positive at the intensity of ≥2+ in 21% to <50% of the tumor cell population. Both cohort were selected for treatment with 100mg/$m^2$ of huMAb2-3-SPDB-DM4.

For the non-sqNSCLC (**Lung**) cohort with CEACAM5 expression ≥50% of tumor cell population at ≥2+ intensity, a minimum of 30 patients pre-treated with anti-PD1/anti-PDL1 was included to evaluate the antitumor activity of huMAb2-3-SPDB-DM4 in patients pre-treated with an anti-PDLl and to ensure a minimum of power for subgroup analysis in this sub-population. For the non-sqNSCLC **(Lung bis)** cohort with CEACAM5 expression between ≥1% to <50% at ≥2+ intensity, the relationship between CEACAM5 expression level and efficacy outcome will be assessed by adding a cohort of 28 treated patients with mild CEACAM5 membrane staining (at least 1% positive tumor cells and <50% of tumor cells at intensity >2+).

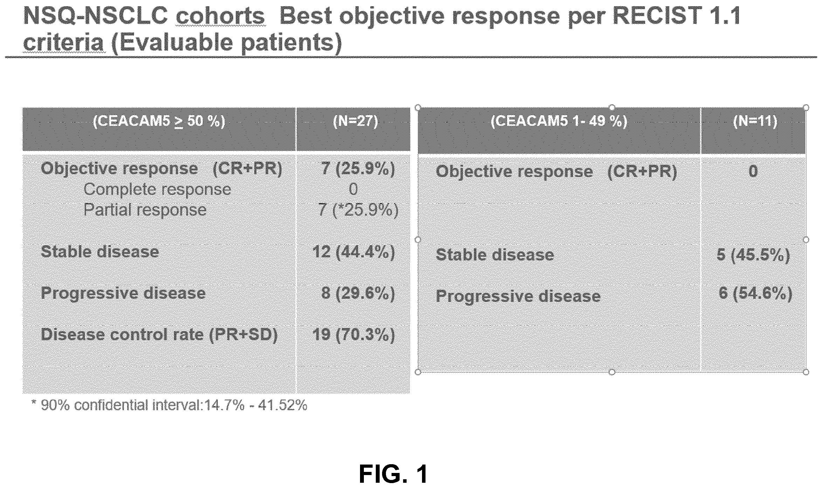

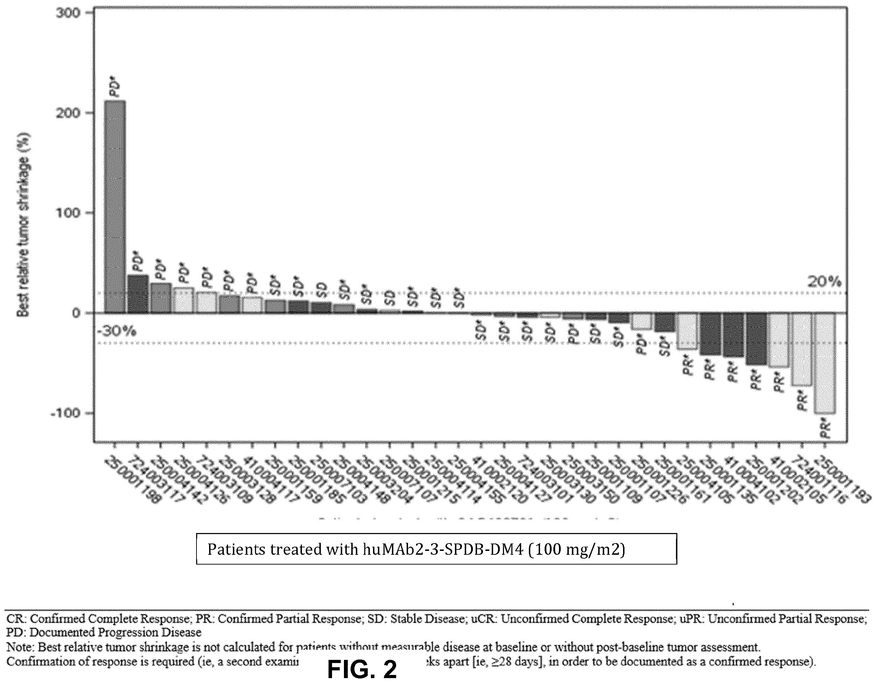

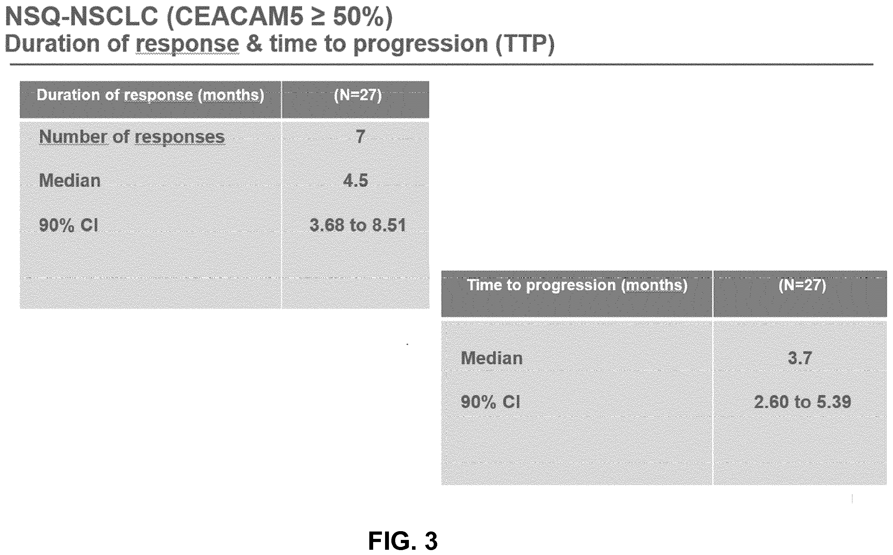

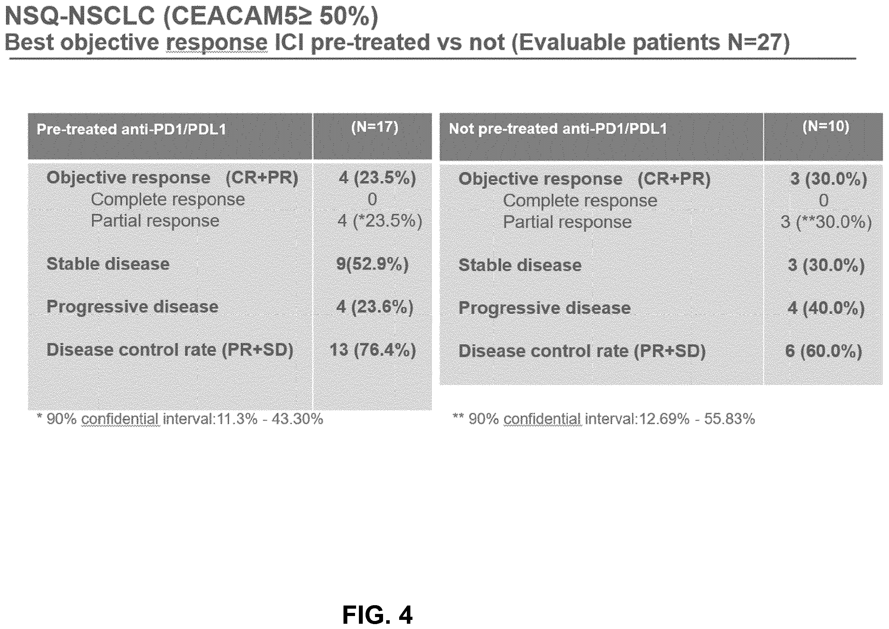

As shown in **FIG. 1** an objective response of 25.9% was observed in the high CEACAM5-expression (Lung) cohort, whereas no objective response was observed in the low expression (Lung-bis) cohort. Further, as depicted in **FIG. 2,** objective response results of non-small cell lung cancer patients treated with huMAb2-3-SPDB-DM4. Further, the best relative tumor shrinkage was observed in patients with CEACAM5 expression of 50-80% or greater than 80%. As depicted in **FIG. 3,** the duration of response (DoR) and time to progression (TTP) was also improved in the high expressing (Lung) cohort. Moreover, of these high expressing patients, a similar object response was obtained in those pre-treated with anti-PD1/PDL1 and those not pre-treated with anti-PD1/PDL1 (**FIG. 4**).

In conclusion, these data demonstrate that proof of concept was achieved in a subset of NSCLC lung cancers treated with huMAb2-3-SPDB-DM4. In particular, these data support the conclusion that huMAb2-3-SPDB-DM4 is effective in treating NSQ-NSCLC, a subtype that represents approximately 60% lung cancers. Moreover, these data support the conclusion that huMAb2-3-SPDB-DM4 is particularly effective in treating high CEACAM5 expressing NSQ-NSCLC, a tumor types that represents approximately 20% of all lung cancers.

## Claims

1. An antibody for use in treating high carcinoembryonic antigen-related cell adhesion molecule 5 cancer in a subject in need thereof, wherein the antibody specifically binds human carcinoembryonic antigen-related cell adhesion molecule 5 (hCEACAM5) wherein the antibody comprises a heavy chain variable region (VH) and a light chain variable region (VL), wherein the VH comprises the three complementarity determining regions HCDR1, HCDR2 and HCDR3 and wherein the VL comprises the three CDRs LCDR1, LCDR2 and LCDR3, wherein the HCDR1 comprises the amino acid sequence of SEQ ID NO: 3 (**GFVFSSYD**); the HCDR2 comprises the amino acid sequence of SEQ ID NO: 4 (**ISSGGGIT**)**;** the HCDR3 comprises the amino acid sequence of SEQ ID NO: 5 (**AAHYFGSSGPFAY**); the LCDR1 comprises the amino acid sequence of SEQ ID NO: 6 (**ENIFSY**); the LCDR2 comprises the amino acid sequence of **NTR**; and the LCDR3 comprises the amino acid sequence of SEQ ID NO: 7 (**QHHYGTPFT**).

2. An antibody for use in treating non squamous non-small cell lung cancer (NSQ NSCLC) in a subject in need thereof, wherein the antibody specifically hCEACAM5 wherein the antibody comprises a VH and a VL, wherein the VH comprises the three complementarity determining regions HCDR1, HCDR2 and HCDR3) and wherein the VL comprises the three CDRs LCDR1, LCDR2 and LCDR3, wherein the HCDR1 comprises the amino acid sequence of SEQ ID NO: 3 **(GFVFSSYD)**; the HCDR2 comprises the amino acid sequence of SEQ ID NO: 4 **(ISSGGGIT)**; the HCDR3 comprises the amino acid sequence of SEQ ID NO: 5 **(AAHYFGSSGPFAY)**; the LCDR1 comprises the amino acid sequence of SEQ ID NO: 6 **(ENIFSY)**; the LCDR2 comprises the amino acid sequence of **NTR**; and the LCDR3 comprises the amino acid sequence of SEQ ID NO: 7 **(QHHYGTPFT).**

3. An antibody for use in treating a subject that has been pre-treated with a cancer therapeutic , wherein the antibody specifically binds hCEACAM5 wherein the antibody comprises a VH and a VL, wherein the VH comprises the three complementarity determining regions HCDR1, HCDR2 and HCDR3) and wherein the VL comprises the three CDRs LCDR1, LCDR2 and LCDR3, wherein the HCDR1 comprises the amino acid sequence of SEQ ID NO: 3 **(GFVFSSYD)**; the HCDR2 comprises the amino acid sequence of SEQ ID NO: 4 **(ISSGGGIT)**; the HCDR3 comprises the amino acid sequence of SEQ ID NO: 5 (**AAHYFGSSGPFAY**); the LCDR1 comprises the amino acid sequence of SEQ ID NO: 6 (**ENIFSY**); the LCDR2 comprises the amino acid sequence of **NTR**; and the LCDR3 comprises the amino acid sequence of SEQ ID NO: 7 **(QHHYGTPFT).**

4. The antibody for use according to claim 3, wherein the subject is a high carcinoembryonic antigen-related cell adhesion molecule expresser.

5. The antibody for use according to any of claim 3, wherein the subject was pre-treated with an agent or drug for treatment of non-small cell lung cancer.

6. The antibody for use according to claim 5, wherein the agent or drug is selected from the group consisting of: a chemotherapy agent, an angiogenesis inhibitor, an epidermal growth factor receptor (EGFR) inhibitor, an anaplastic lymphoma kinase (ALK) inhibitor, a receptor tyrosine kinase (ROS1) inhibitor, and an immune checkpoint inhibitor.

7. The antibody for use according to claim 6, wherein the immune checkpoint inhibitor is a PD-1 inhibitor and/or a PD-L1 inhibitor.

8. The antibody for use according to any of claims 1-3, wherein the VH comprises SEQ ID NO: 1

(EVQLQESGPGLVKPGGSLSLSCAAS**GFVFSSYD**MSWVRQTPERGLEWVAY**ISSGGGIT**YAPSTVKGRFTVSRDNAKNTLYLQMNSLTSEDTAVYYC**AAHYFGSSGPFAY**WGQGTLVTVSS).

**9.** The antibody for use according to claim 8, wherein the heavy chain comprises SEQ ID NO: 8

(EVQLQESGPGLVKPGGSLSLSCAASGFVFSSYDMSWVRQTPERGLEWVAYISSGGGITYAPSTVKGRFTVSRDNAKNTLYLQMNSLTSEDTAVYYCAAHYFGSSGPFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEL

LGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG).

**10.** The antibody for use according to any of claims 1-3, wherein the VL comprises SEQ ID NO: 2

(DIQMTQSPASLSASVGDRVTITCRAS**ENIFSY**LAWYQQKPGKSPKLLVY**NTR**TLAEGVPSRFSGSGSGTDFSLTISSLQPEDFATYYC**QHHYGTPFT**FGSGTKLEIK).

**11.** The antibody for use according to claim 10, wherein the light chain comprises SEQ ID NO: 9

(DIQMTQSPASLSASVGDRVTITCRASENIFSYLAWYQQKPGKSPKLLVYNTRTLAEGVPSRFSGSGSGTDFSLTISSLQPEDFATYYCQHHYGTPFTFGSGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC).

**12.** The antibody for use according to any one of claims to 1-11 conjugated or linked to at least one growth inhibitory agent.

**13.** The antibody for use according to claim 12, wherein said growth inhibitory agent is a cytotoxic agent.

**14.** The antibody for use according to claim 12 or 13, wherein said growth inhibitory agent is selected from the group consisting of chemotherapeutic agents, enzymes, antibiotics, and toxins such as small molecule toxins or enzymatically active toxins, taxoids, vincas, taxanes, maytansinoid or maytansinoid analogs, tomaymycin or pyrrolobenzodiazepine derivatives, cryptophycin derivatives, leptomycin derivatives, auristatin or dolastatin analogs, prodrugs, topoisomerase II inhibitors, DNA alkylating agents, anti-tubulin agents, and CC-1065 or CC-1065 analogs.

**15.** The antibody for use according to claim 13 or 14, wherein said growth inhibitory agent is (N2'-deacetyl-N2'-(3-mercapto-1-oxopropyl)-maytansine) DM1 or N2'-deacetyl-N-2'(4-methyl-4-mercapto-1-oxopentyl)-maytansine (DM4).

**16.** The antibody for use according to claim 15, wherein said growth inhibitory agent is DM4.

17. The antibody for use according to any one of claims 12-16, wherein the antibody is covalently attached via a cleavable or non-cleavable linker to the at least one growth inhibitory agent.

18. The antibody for use according to claim 17, wherein said linker is selected from the group consisting of N-succinimidyl pyridyldithiobutyrate (SPDB), 4-(Pyridin-2-yldisulfanyl)-2-sulfo-butyric acid (sulfo-SPDB), and succinimidyl (N-maleimidomethyl) cyclohexane-1-carboxylate (SMCC).

19. The antibody for use according to claim 18, wherein said linker is SPDB.

20. The antibody for use according to any of claims 1-19, wherein the antibody is huMAb2-3.

21. The antibody for use according to any of claims 1-20, wherein the subject has a hCEACAM5 expression of a percent score greater than or equal to 50 (consisting of 2+ and 3+ intensities) in the tumor cells populations, for example, the a percent score greater than or equal to about 50, about 50 to about 80, or 100 (consisting of 2+ and 3+ intensities).

22. The antibody for use according to any of claims 1-21, wherein the antibody is administered by intravenously.

23. The antibody for use according to claim 22, wherein the antibody is administered at a rate of 2.5 mg/min for the first 30 minutes or 1 hour.

24. The antibody for use according to claim 23, wherein, after the 30 minutes, the rate is increased to 5 mg/min.

25. The antibody for use according to claim 23 or 24, wherein the antibody is administered at a dose level of 5, 10, 20, 30, 40, 60, 80, 100, 120, 150, 180, or 210 $mg/m^2$ based on the body surface area of the subject.

26. The antibody for use according to any of claims 1-25, wherein the antibody is administered at every 14 days, or every 3 weeks.

27. The antibody for use according to any of claims 1-26, wherein, prior to administering the antibody, the subject is administered a pre-medication with histamine H1 antagonist.

28. The antibody for use according to claim 27, wherein the histamine H1 antagonist is diphenylhydramine.

29. The antibody for use according to claim 27, wherein the histamine H1 antagonist is dexchlorpheniramine.

30. The antibody for use according to any of the preceding claims, wherein progression of at least one symptom of cancer is reduced, slowed, halted, or otherwise ameliorated.

31. The antibody for use according to claim 30, wherein the tumor growth rate is reduced after treatment with the antibody.

32. The antibody for use according to claim 30, wherein the tumor size is reduced after treatment with the antibody.

33. The antibody for use according to claim 30, wherein the expression pattern, intensity and proportion of expressing cells indicates a reduction, slowing or halting of the cancer.

34. A pharmaceutical composition comprising the antibody according to any one of claims 1-31, and a pharmaceutically acceptable carrier.

35. A method for treating high carcinoembryonic antigen-related cell adhesion molecule 5 cancer in a subject in need thereof, the method comprising administering an antibody that specifically binds hCEACAM5 wherein the antibody comprises a VH and a VL, wherein the VH comprises the three complementarity determining regions HCDR1, HCDR2 and HCDR3) and wherein the VL comprises the three CDRs LCDR1, LCDR2 and LCDR3, wherein the HCDR1 comprises the amino acid sequence of SEQ ID NO: 3 **(GFVFSSYD)**; the HCDR2 comprises the amino acid sequence of SEQ ID NO: 4 **(ISSGGGIT)**; the HCDR3 comprises the amino acid sequence of SEQ ID NO: 5 **(AAHYFGSSGPFAY)**; the LCDR1 comprises the amino acid sequence of SEQ ID NO: 6 **(ENIFSY)**; the LCDR2 comprises the amino acid sequence of **NTR**; and the LCDR3 comprises the amino acid sequence of SEQ ID NO: 7 **(QHHYGTPFT).**

**36.** A method for treating non squamous non-small cell lung cancer (NSQ NSCLC) in a subject in need thereof, the method comprising administering an antibody that specifically binds hCEACAM5 wherein the antibody comprises a VH and a VL, wherein the VH comprises the three complementarity determining regions HCDR1, HCDR2 and HCDR3) and wherein the VL comprises the three CDRs LCDR1, LCDR2 and LCDR3, wherein the HCDR1 comprises the amino acid sequence of SEQ ID NO: 3 **(GFVFSSYD)**; the HCDR2 comprises the amino acid sequence of SEQ ID NO: 4 **(ISSGGGIT)**; the HCDR3 comprises the amino acid sequence of SEQ ID NO: 5 **(AAHYFGSSGPFAY)**; the LCDR1 comprises the amino acid sequence of SEQ ID NO: 6 **(ENIFSY)**; the LCDR2 comprises the amino acid sequence of **NTR**; and the LCDR3 comprises the amino acid sequence of SEQ ID NO: 7 **(QHHYGTPFT).**

**37.** A method for treating a subject that has been pre-treated with a cancer therapeutic, the method comprising administering an antibody that specifically binds hCEACAM5 wherein the antibody comprises a VH and a VL, wherein the VH comprises the three complementarity determining regions HCDR1, HCDR2 and HCDR3) and wherein the VL comprises the three CDRs LCDR1, LCDR2 and LCDR3, wherein the HCDR1 comprises the amino acid sequence of SEQ ID NO: 3 **(GFVFSSYD)**; the HCDR2 comprises the amino acid sequence of SEQ ID NO: 4 **(ISSGGGIT)**; the HCDR3 comprises the amino acid sequence of SEQ ID NO: 5 **(AAHYFGSSGPFAY)**; the LCDR1 comprises the amino acid sequence of SEQ ID NO: 6 **(ENIFSY)**; the LCDR2 comprises the amino acid sequence of **NTR**; and the LCDR3 comprises the amino acid sequence of SEQ ID NO: 7 **(QHHYGTPFT).**

**38.** The method according to claim 37, wherein the subject is a high carcinoembryonic antigen-related cell adhesion molecule expresser.

**39.** The method according to any of claim 37, wherein the subject was previously treated with an agent or drug for treatment of non-small cell lung cancer.

**40.** The method according to claim 39, wherein the agent or drug is selected from the group consisting of: a chemotherapy agent, an angiogenesis inhibitor, an epidermal growth factor receptor (EGFR) inhibitor, an aplastic lymphoma kinase (ALK) inhibitor, a receptor tyrosine kinase (ROS1) inhibitor, and an immune checkpoint inhibitor.

**41.** The method according to claim 40, wherein the immune checkpoint inhibitor is a PD-1 inhibitor and/or a PD-L1 inhibitor.

**42.** The method according to any of claims 35-37, wherein the VH comprises SEQ ID NO: 1

(EVQLQESGPGLVKPGGSLSLSCAAS**GFVFSSYD**MSWVRQTPERGLEWVAY**ISSGGGIT**
YAPSTVKGRFTVSRDNAKNTLYLQMNSLTSEDTAVYYC**AAHYFGSSGPFAY**WGQGTL
VTVSS) .

**43.** The method according to claim 42, wherein the heavy chain comprises SEQ ID NO: 8

(EVQLQESGPGLVKPGGSLSLSCAASGFVFSSYDMSWVRQTPERGLEWVAYISSGGGITY
APSTVKGRFTVSRDNAKNTLYLQMNSLTSEDTAVYYCAAHYFGSSGPFAYWGQGTLVT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEL

LGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLP
PSRDELTKNQVSLTCLVKGFYPSDIAVEWESNGQPENNYKTTPPVLDSDGSFFLYSKLTV
DKSRWQQGNVFSCSVMHEALHNHYTQKSLSLSPG).

**44.** The method according to any of claims 35-37, wherein the VL comprises SEQ ID NO: 2

(DIQMTQSPASLSASVGDRVTITCRAS**ENIFSY**LAWYQQKPGKSPKLLVY**NTR**TLAEGVP SRFSGSGSGTDFSLTISSLQPEDFATYYC**QHHYGTPFT**FGSGTKLEIK).

**45.** The method according to claim 44, wherein the light chain comprises SEQ ID NO: 9

(DIQMTQSPASLSASVGDRVTITCRASENIFSYLAWYQQKPGKSPKLLVYNTRTLAEGVP SRFSGSGSGTDFSLTISSLQPEDFATYYCQHHYGTPFTFGSGTKLEIKRTVAAPSVFIFPPS DEQLKSGTASVVCLLNNFYPREAKVQWKVDNALQSGNSQESVTEQDSKDSTYSLSSTL TLSKADYEKHKVYACEVTHQGLSSPVTKSFNRGEC).

**46.** The method according to any one of claims to 35-45, wherein the antibody is conjugated or linked to at least one growth inhibitory agent.

**47.** The method according to claim 46, wherein said growth inhibitory agent is a cytotoxic agent.

**48.** The method according to claim 46 or 47, wherein said growth inhibitory agent is selected from the group consisting of chemotherapeutic agents, enzymes, antibiotics, and toxins such as small molecule toxins or enzymatically active toxins, taxoids, vincas, taxanes, maytansinoid or maytansinoid analogs, tomaymycin or pyrrolobenzodiazepine derivatives, cryptophycin derivatives, leptomycin derivatives, auristatin or dolastatin analogs, prodrugs, topoisomerase II inhibitors, DNA alkylating agenst, anti-tubulin agents, and CC-1065 or CC-1065 analogs.

**49.** The method according to claim 47 or 48, wherein said growth inhibitory agent is (N2'-deacetyl-N2'-(3-mercapto-1-oxopropyl)-maytansine) DM1 orN2'-deacetyl-N-2'(4-methyl-4-mercapto-1-oxopentyl)-maytansine (DM4).

**50.** The method according to claim 49, wherein said growth inhibitory agent is DM4.

**51.** The method according to any one of claims 46-50, wherein the antibody is covalently attached via a cleavable or non-cleavable linker to the at least one growth inhibitory agent.

**52.** The method according to claim 51, wherein said linker is selected from the group consisting of N-succinimidyl pyridyldithiobutyrate (SPDB), 4-(Pyridin-2-yldisulfanyl)-2-sulfobutyric acid (sulfo-SPDB), and succinimidyl (N-maleimidomethyl) cyclohexane-1-carboxylate (SMCC).

**53.** The method according to claim 51, wherein said linker is SPDB.

**54.** The method according to any of claims 35-53, wherein the antibody is huMAb2-3.

**55.** The method according to any of claims 35-54, wherein the subject has a a hCEACAM5 expression of a percent score greater than or equal to 50 (consisting of 2+ and 3+ intensities) in the tumor cells populations, for example, a percent score greater than or equal to about 50, about 50 to about 80, or 100 (consisting of 2+ and 3+ intensities).

**56.** The method according to any of claims 35-55, wherein the antibody is administered by intravenously.

**57.** The method according to claim 56, wherein the antibody is administered at a rate of 2.5 mg/min for the first 30 minutes or 1 hour.

**58.** The method according to claim 57, wherein, after 30 minutes, the rate is increased to 5 mg/min.

**59.** The method according to claim 57 or 58, wherein the antibody is administered at a dose level of 5, 10, 20, 30, 40, 60, 80, 100, 120, 150, 180, or 210 mg/$m^2$ based on the body surface area of the subject.

**60.** The method according to any of claims 35-59, wherein the antibody is administered at every 14 days, or every 3 weeks.

**61.** The method according to any of claims 35-60, wherein, prior to administering the antibody, the subject is administered a pre-medication with histamine H1 antagonist.

**62.** The method according to claim 61, wherein the histamine H1 antagonist is diphenylhydramine or dexchlorpheniramine.

**63.** The method of any of claims 35-62, wherein progression of at least one symptom of cancer is reduced, slowed, halted, or otherwise ameliorated.

**64.** The method according to claim 63, wherein the tumor growth rate or tumor size is reduced after treatment with the antibody.

**65.** The method according to claim 63, wherein the expression pattern, intensity and proportion of expressing cells indicates a reduction, slowing or halting of the cancer.

**66.** The method of any of claims 35-65, wherein the antibody is administered as a pharmaceutical composition comprising a pharmaceutically acceptable carrier.

## NSQ-NSCLC cohorts Best objective response per RECIST 1.1 criteria (Evaluable patients)

| (CEACAM5 ≥ 50 %) | (N=27) |
|---|---|
| **Objective response (CR+PR)** | **7 (25.9%)** |
| Complete response | 0 |
| Partial response | 7 (*25.9%) |
| **Stable disease** | **12 (44.4%)** |
| **Progressive disease** | **8 (29.6%)** |
| **Disease control rate (PR+SD)** | **19 (70.3%)** |

* 90% confidential interval:14.7% - 41.52%

| (CEACAM5 1- 49 %) | (N=11) |
|---|---|
| **Objective response (CR+PR)** | **0** |
| **Stable disease** | **5 (45.5%)** |
| **Progressive disease** | **6 (54.6%)** |

**FIG. 1**

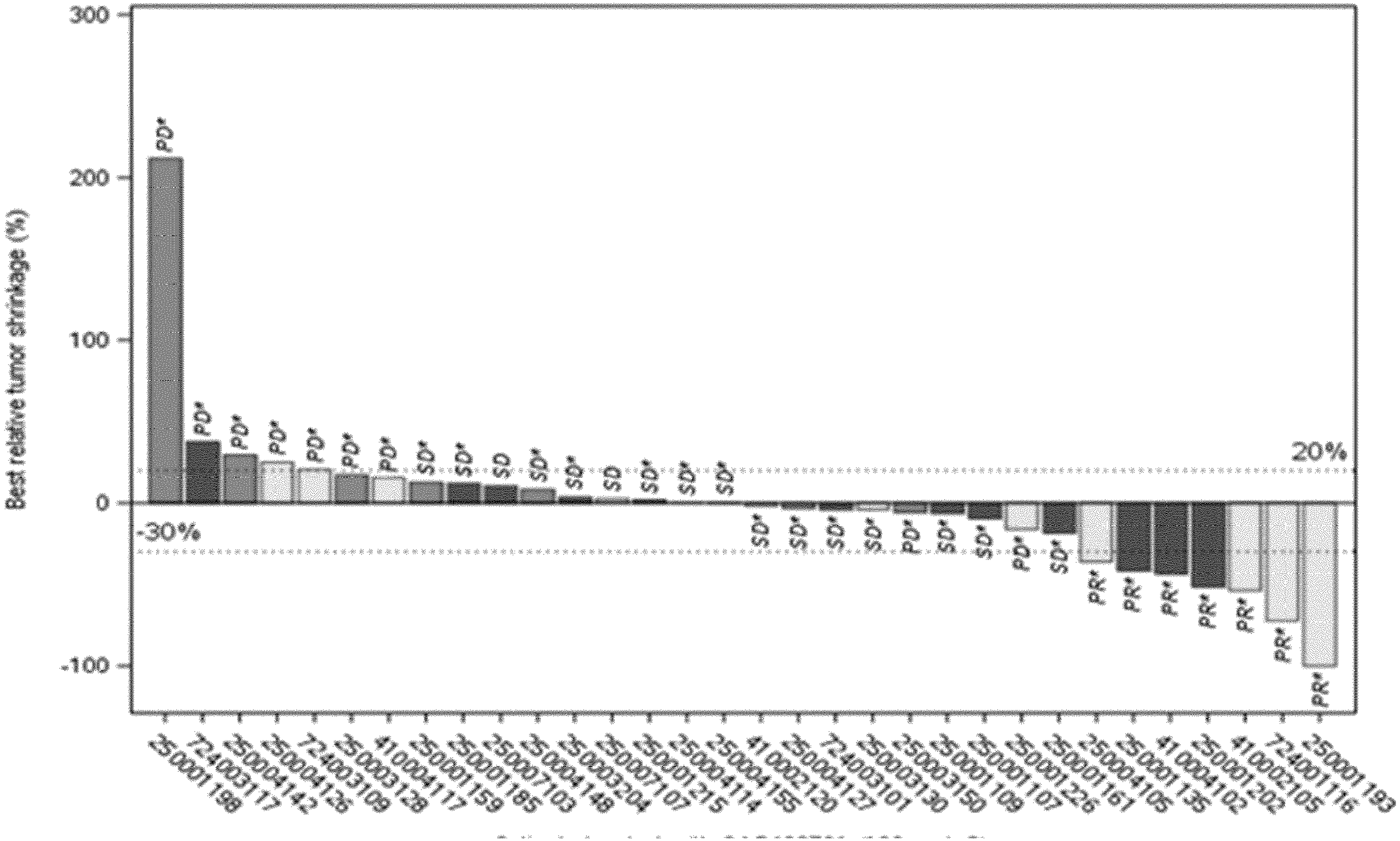


Patients treated with huMAb2-3-SPDB-DM4 (100 mg/m2)

CR: Confirmed Complete Response; PR: Confirmed Partial Response; SD: Stable Disease; uCR: Unconfirmed Complete Response; uPR: Unconfirmed Partial Response; PD: Documented Progression Disease

Note: Best relative tumor shrinkage is not calculated for patients without measurable disease at baseline or without post-baseline tumor assessment.

Confirmation of response is required (ie, a second exami ... ks apart [ie, ≥28 days], in order to be documented as a confirmed response).

**FIG. 2**

## NSQ-NSCLC (CEACAM5 ≥ 50%)
### Duration of response & time to progression (TTP)

| Duration of response (months) | (N=27) |
|---|---|
| Number of responses | 7 |
| Median | 4.5 |
| 90% CI | 3.68 to 8.51 |

| Time to progression (months) | (N=27) |
|---|---|
| Median | 3.7 |
| 90% CI | 2.60 to 5.39 |

FIG. 3

**NSQ-NSCLC (CEACAM5≥ 50%)**
**Best objective response ICI pre-treated vs not (Evaluable patients N=27)**

| Pre-treated anti-PD1/PDL1 | (N=17) |
|---|---|
| **Objective response (CR+PR)** | **4 (23.5%)** |
| Complete response | 0 |
| Partial response | 4 (*23.5%) |
| **Stable disease** | **9(52.9%)** |
| **Progressive disease** | **4 (23.6%)** |
| **Disease control rate (PR+SD)** | **13 (76.4%)** |

* 90% confidential interval:11.3% - 43.30%

| Not pre-treated anti-PD1/PDL1 | (N=10) |
|---|---|
| **Objective response (CR+PR)** | **3 (30.0%)** |
| Complete response | 0 |
| Partial response | 3 (**30.0%) |
| **Stable disease** | **3 (30.0%)** |
| **Progressive disease** | **4 (40.0%)** |
| **Disease control rate (PR+SD)** | **6 (60.0%)** |

** 90% confidential interval:12.69% - 55.83%

**FIG. 4**

Europäisches Patentamt
European Patent Office
Office européen des brevets

# EUROPEAN SEARCH REPORT

Application Number
EP 19 30 5173

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br>Y | WO 2014/079886 A1 (SANOFI SA [FR]) 30 May 2014 (2014-05-30)<br>* page 61, line 33-page 62, line 4; page 62, lines 17-22; page 69, lines 16-18; page 112, lines 13-14; claims 18, 25-28, 34-35; figure 18; table 22; sequences 87, 88 *<br>----- | 1-5,8-15<br>6,7 | INV.<br>A61K47/68<br>A61P35/00<br>C07K16/30 |
| X<br>Y | WO 2018/227023 A1 (SILVERBACK THERAPEUTICS INC [US]) 13 December 2018 (2018-12-13)<br>* page 60, embodiment r); table at page 104 in the row "Humanized Ab2-3"; paragraphs [0170] and [0524]; claims 11-33; sequences 831, 832 *<br>----- | 1,3-6, 8-14<br>6,7 | |

TECHNICAL FIELDS SEARCHED (IPC)

A61K

The present search report has been drawn up for all claims

2

| Place of search | Date of completion of the search | Examiner |
|---|---|---|
| The Hague | 31 October 2019 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

# ANNEX TO THE EUROPEAN SEARCH REPORT ON EUROPEAN PATENT APPLICATION NO. EP 19 30 5173

This annex lists the patent family members relating to the patent documents cited in the above-mentioned European search report.
The members are as contained in the European Patent Office EDP file on
The European Patent Office is in no way liable for these particulars which are merely given for the purpose of information.

31-10-2019

| Patent document cited in search report | Publication date | Patent family member(s) | Publication date |
|---|---|---|---|
| WO 2014079886 A1 | 30-05-2014 | AR 093557 A1 | 10-06-2015 |
| | | AU 2013349733 A1 | 11-06-2015 |
| | | AU 2018267636 A1 | 13-12-2018 |
| | | CA 2889962 A1 | 30-05-2014 |
| | | CL 2015001321 A1 | 17-07-2015 |
| | | CN 104918958 A | 16-09-2015 |
| | | CN 109180815 A | 11-01-2019 |
| | | CR 20150258 A | 11-06-2015 |
| | | CY 1119219 T1 | 14-02-2018 |
| | | DK 2922875 T3 | 06-06-2017 |
| | | DO P2015000110 A | 16-08-2015 |
| | | EA 201590986 A1 | 31-05-2016 |
| | | EP 2922875 A1 | 30-09-2015 |
| | | EP 3199552 A1 | 02-08-2017 |
| | | ES 2625742 T3 | 20-07-2017 |
| | | HK 1215444 A1 | 26-08-2016 |
| | | HR P20170743 T1 | 28-07-2017 |
| | | HU E033369 T2 | 28-11-2017 |
| | | IL 238559 A | 31-10-2018 |
| | | JP 6324399 B2 | 16-05-2018 |
| | | JP 2016506370 A | 03-03-2016 |
| | | KR 20150085828 A | 24-07-2015 |
| | | LT 2922875 T | 12-06-2017 |
| | | MA 38194 A1 | 28-02-2018 |
| | | MX 358321 B | 14-08-2018 |
| | | PE 11802015 A1 | 12-08-2015 |
| | | PH 12015501092 A1 | 27-07-2015 |
| | | PL 2922875 T3 | 31-08-2017 |
| | | PT 2922875 T | 31-05-2017 |
| | | SG 11201503285T A | 28-05-2015 |
| | | TN 2015000177 A1 | 03-10-2016 |
| | | TW 201427997 A | 16-07-2014 |
| | | US 2016108131 A1 | 21-04-2016 |
| | | US 2018022817 A1 | 25-01-2018 |
| | | UY 35147 A | 30-06-2014 |
| | | WO 2014079886 A1 | 30-05-2014 |
| | | ZA 201502959 B | 27-01-2016 |
| WO 2018227023 A1 | 13-12-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007071426 A
- WO 2014079886 A
- WO 2014079886 A1
- WO 8705330 A
- US 4640835 A
- US 4496689 A
- US 4301144 A
- US 4670417 A
- US 4791192 A
- US 4179337 A
- US 4424219 A
- US 4256746 A
- US 4294757 A
- US 4307016 A
- US 4313946 A
- US 4315929 A
- US 4331598 A
- US 4361650 A
- US 4362663 A
- US 4364866 A
- US 4450254 A
- US 4322348 A
- US 4371533 A
- US 6333410 B
- US 5475092 A
- US 5585499 A
- US 5846545 A
- WO 2008010101 A
- WO 2004091668 A
- WO 9411026 A
- US 5208020 A
- WO 8101145 A
- WO 8807378 A
- US 4975278 A
- US 5215534 A
- US 9248242 B
- US 9427531 B
- US 9566395 B
- US 6629949 B
- US 6659982 B


### Non-patent literature cited in the description


- **YOUNES A ; GOPAL AK ; SMITH SE ; ANSELL SM ; ROSENBLATT JD ; SAVAGE KJ et al.** Results of a pivotal phase II study of brentuximab vedotin for patients with relapsed or refractory Hodgkin's lymphoma. *J Clin Oncol.,* 2012, vol. 30 (18), 2183-9
- **VERMA S ; MILES D ; GIANNI L ; KROP IE ; WELSLAU M ; BASELGA J et al.** Trastuzumab emtansine for HER2-positive advanced breast cancer. *N Engl J Med.,* 2012, vol. 19, 1783-91
- **YOUNG A. ; 2012 et al.** *J Clin Oncol.,* vol. 30 (18), 2183-9
- **VERMA, S. et al.** *N Engl J Med.,* 2012, vol. 19, 1783-91
- **ZAROGOULIDIS et al.** *J Thorac Dis.,* September 2013, vol. 5 (4), S389-S396
- **YADAV, L. et al.** *J Clin Diagn Res.,* June 2015, vol. 9 (6), XE01-XE05
- **KYLE, F. et al.** *Cancer Imaging.,* 2008, vol. 8 (1), 199-2
- **CHIN, L.P. et al.** *Thorac Oncol.,* 2012, vol. 7, 1625-1630
- **DEMPKE, W.C.M. et al.** *Lung Cancer,* vol. 67 (3), 257-274
- **CHANPRAPAPH, K. et al.** *Dermatol Res Pract.,* 2014, vol. 734249
- **JENKINS, R. W. et al.** *British Journal of Cancer,* 2018, vol. 118, 9-16
- **DE LA BELLACASA, R. P. et al.** *Transl Lung Cancer Res.,* 2013, vol. 2 (2), 72-86
- **GOLD ; FREEDMAN.** *J Exp Med,* 1965, vol. 121, 439
- **KAMMERER ; ZIMMERMANN.** *BMC Biology,* 2010, vol. 8, 12
- **HAMMARSTRÖM et al.** Tumor markers, Physiology, Pathobiology, Technology and Clin-ical Applications. AACC Press, 2002, 375
- **SHARKEY et al.** *Cancer Research,* 1990, vol. 50, 2823
- **EBRAHIMMNEJAD et al.** *Exp Cell Res,* 2000, vol. 260, 365
- **ZHAO et al.** *J Immunol Methods,* 2004, vol. 293, 207
- **STRICKLAND et al.** *J Pathol,* 2009, vol. 218, 380
- **DOERN et al.** *J. Biol. Chem,* 2009, vol. 284, 10254
- **BEERS et al.** *Semin Hematol,* vol. 47, 107-114

- **SHARKEY et al.** *Cancer Research,* 1995, vol. 55, 5935
- **PENG et al.** *PLoS ONE,* vol. 7 (5), e3641
- **OBERST et al.** *AACR Annual Meeting,* April 2009
- **SCHMIDT et al.** *Cancer Immunol. Immunother,* 2008, vol. 57, 1879
- **NEEDLEMAN ; WUNSCH J.** *Mol. Biol.,* 1970, vol. 48, 443
- **TAYLOR et al.** *Nucl. Acids Res.,* 1992, vol. 20, 6287-6295
- **ALMAGRO ; FRANSSON.** *Front Biosci.,* 2008, vol. 13, 1619-1633
- **JESPERS et al.** *Biotechnology,* 1994, vol. 12, 899
- **ANGAL et al.** *Molecular Immunology,* 1993, vol. 30, 105
- **WANG ; GOSH.** *J. Membrane Sci.,* 2008, vol. 318, 311-316
- **CROMWELL et al.** *AAPS Jounal,* 2006, vol. 8 (3), E572-E579
- **WALTER et al.** *Anal. Biochem.,* 1993, vol. 212 (2), 469-480
- Therapeutic Antibodies and Protocols. Therapeutic Antibodies and Protocols. Springer Science, 2009, vol. 525, 445
- Remington’s Pharmaceutical Sciences. 1035-1038, 1570-1580
- Remington’s Pharmaceutical Sciences. Mack Publishing Company
- **POWELL et al.** Compendium of excipients for parenteral formulations’’ PDA. *J Pharm Sci Technol,* 1998, vol. 52, 238-311
- **WU et al.** *J. Biol. Chem.,* 1987, vol. 262, 4429-4432
- **LANGER.** *Science,* 1990, vol. 249, 1527-1533
- **MEEHAN et al.** *J. Controlled Release,* 1996, vol. 46, 107-116

SEQUENCE LISTING

<110>  SANOFI

<120>  USE OF ANTI-CEACAM5 IMMUNOCONJUGATES FOR TREATING LUNG CANCER

<130>  FR2019/003 EP

<160>  23

<170>  PatentIn version 3.5

<210>  1
<211>  120
<212>  PRT
<213>  Artificial Sequence

<220>
<223>  VH1a of humanized  MAb2 antibody

<400>  1

```
Glu Val Gln Leu Gln Glu Ser Gly Pro Gly Leu Val Lys Pro Gly Gly
1               5                   10                  15

Ser Leu Ser Leu Ser Cys Ala Ala Ser Gly Phe Val Phe Ser Ser Tyr
            20                  25                  30

Asp Met Ser Trp Val Arg Gln Thr Pro Glu Arg Gly Leu Glu Trp Val
        35                  40                  45

Ala Tyr Ile Ser Ser Gly Gly Gly Ile Thr Tyr Ala Pro Ser Thr Val
    50                  55                  60

Lys Gly Arg Phe Thr Val Ser Arg Asp Asn Ala Lys Asn Thr Leu Tyr
65                  70                  75                  80

Leu Gln Met Asn Ser Leu Thr Ser Glu Asp Thr Ala Val Tyr Tyr Cys
                85                  90                  95

Ala Ala His Tyr Phe Gly Ser Ser Gly Pro Phe Ala Tyr Trp Gly Gln
            100                 105                 110

Gly Thr Leu Val Thr Val Ser Ser
        115                 120
```

<210>  2
<211>  107
<212>  PRT
<213>  Artificial sequence

<220>
<223>  VL1c of humanized MAb2 antibody

<400>  2

Asp Ile Gln Met Thr Gln Ser Pro Ala Ser Leu Ser Ala Ser Val Gly
1 5 10 15

Asp Arg Val Thr Ile Thr Cys Arg Ala Ser Glu Asn Ile Phe Ser Tyr
20 25 30

Leu Ala Trp Tyr Gln Gln Lys Pro Gly Lys Ser Pro Lys Leu Leu Val
35 40 45

Tyr Asn Thr Arg Thr Leu Ala Glu Gly Val Pro Ser Arg Phe Ser Gly
50 55 60

Ser Gly Ser Gly Thr Asp Phe Ser Leu Thr Ile Ser Ser Leu Gln Pro
65 70 75 80

Glu Asp Phe Ala Thr Tyr Tyr Cys Gln His His Tyr Gly Thr Pro Phe
85 90 95

Thr Phe Gly Ser Gly Thr Lys Leu Glu Ile Lys
100 105

<210> 3
<211> 8
<212> PRT
<213> artificial sequence

<220>
<223> HCDR1

<400> 3

Gly Phe Val Phe Ser Ser Tyr Asp
1 5

<210> 4
<211> 8
<212> PRT
<213> artificial sequence

<220>
<223> HCDR2

<400> 4

Ile Ser Ser Gly Gly Gly Ile Thr
1 5

<210> 5
<211> 13
<212> PRT
<213> Artificial Sequence

<220>

<223> HCDR3

<400> 5

Ala Ala His Tyr Phe Gly Ser Ser Gly Pro Phe Ala Tyr
1 5 10

<210> 6
<211> 6
<212> PRT
<213> Artificial Sequence

<220>
<223> LCDR1

<400> 6

Glu Asn Ile Phe Ser Tyr
1 5

<210> 7
<211> 9
<212> PRT
<213> Artificial Sequence

<220>
<223> LCDR3

<400> 7

Gln His His Tyr Gly Thr Pro Phe Thr
1 5

<210> 8
<211> 449
<212> PRT
<213> Artificial Sequence

<220>
<223> heavy chain

<400> 8

Glu Val Gln Leu Gln Glu Ser Gly Pro Gly Leu Val Lys Pro Gly Gly
1 5 10 15

Ser Leu Ser Leu Ser Cys Ala Ala Ser Gly Phe Val Phe Ser Ser Tyr
20 25 30

Asp Met Ser Trp Val Arg Gln Thr Pro Glu Arg Gly Leu Glu Trp Val
35 40 45

Ala Tyr Ile Ser Ser Gly Gly Gly Ile Thr Tyr Ala Pro Ser Thr Val
50 55 60

Lys Gly Arg Phe Thr Val Ser Arg Asp Asn Ala Lys Asn Thr Leu Tyr

```
65                  70                  75                  80

Leu Gln Met Asn Ser Leu Thr Ser Glu Asp Thr Ala Val Tyr Tyr Cys
                85                  90                  95

Ala Ala His Tyr Phe Gly Ser Ser Gly Pro Phe Ala Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320
```

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Asp Glu Leu Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 9
<211> 214
<212> PRT
<213> Artificial Sequence

<220>
<223> light chain

<400> 9

Asp Ile Gln Met Thr Gln Ser Pro Ser Ser Val Ser Ala Ser Val Gly
1 5 10 15

Asp Arg Val Thr Ile Thr Cys Arg Ala Ser Gln Gly Ile Ser Ser Trp
20 25 30

Leu Ala Trp Tyr Gln Gln Lys Pro Gly Lys Ala Pro Lys Leu Leu Ile
35 40 45

Tyr Gly Ala Ser Ser Leu Glu Ser Gly Val Pro Ser Arg Phe Ser Gly
50 55 60

Ser Gly Ser Gly Thr Asp Phe Thr Leu Thr Ile Ser Ser Leu Gln Pro

65 70 75 80

Glu Asp Phe Ala Ser Tyr Tyr Cys Gln Gln Ala Asn Ser Phe Pro Tyr
85 90 95

Thr Phe Gly Gln Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 10
<211> 7
<212> PRT
<213> Artificial Sequence

<220>
<223> A3-B3 domain of human CEACAM5 protein

<400> 10

Ser Gly Ala Asn Leu Asn Leu
1 5

<210> 11
<211> 13
<212> PRT
<213> Artificial Sequence

<220>
<223> A3-B3 domain epitopes

<400> 11

Ile Asn Gly Ile Pro Gln Gln His Thr Gln Val Leu Phe
1 5 10

<210> 12
<211> 654
<212> PRT
<213> Artificial Sequence

<220>
<223> extracellular domain of Macaca fascicularis CEACAM5

<400> 12

Gln Leu Thr Ile Glu Ser Arg Pro Phe Asn Val Ala Glu Gly Lys Glu
1 5 10 15

Val Leu Leu Leu Ala His Asn Val Ser Gln Asn Leu Phe Gly Tyr Ile
20 25 30

Trp Tyr Lys Gly Glu Arg Val Asp Ala Ser Arg Arg Ile Gly Ser Cys
35 40 45

Val Ile Arg Thr Gln Gln Ile Thr Pro Gly Pro Ala His Ser Gly Arg
50 55 60

Glu Thr Ile Asp Phe Asn Ala Ser Leu Leu Ile Gln Asn Val Thr Gln
65 70 75 80

Ser Asp Thr Gly Ser Tyr Thr Ile Gln Val Ile Lys Glu Asp Leu Val
85 90 95

Asn Glu Glu Ala Thr Gly Gln Phe Arg Val Tyr Pro Glu Leu Pro Lys
100 105 110

Pro Tyr Ile Thr Ser Asn Asn Ser Asn Pro Ile Glu Asp Lys Asp Ala
115 120 125

Val Ala Leu Thr Cys Glu Pro Glu Thr Gln Asp Thr Thr Tyr Leu Trp
130 135 140

Trp Val Asn Asn Gln Ser Leu Pro Val Ser Pro Arg Leu Glu Leu Ser
145 150 155 160

Ser Asp Asn Arg Thr Leu Thr Val Phe Asn Ile Pro Arg Asn Asp Thr
165 170 175

Thr Ser Tyr Lys Cys Glu Thr Gln Asn Pro Val Ser Val Arg Arg Ser
180 185 190

Asp Pro Val Thr Leu Asn Val Leu Tyr Gly Pro Asp Ala Pro Thr Ile

195 200 205

Ser Pro Leu Asn Thr Pro Tyr Arg Ala Gly Glu Tyr Leu Asn Leu Thr
210 215 220

Cys His Ala Ala Ser Asn Pro Thr Ala Gln Tyr Phe Trp Phe Val Asn
225 230 235 240

Gly Thr Phe Gln Gln Ser Thr Gln Glu Leu Phe Ile Pro Asn Ile Thr
245 250 255

Val Asn Asn Ser Gly Ser Tyr Met Cys Gln Ala His Asn Ser Ala Thr
260 265 270

Gly Leu Asn Arg Thr Thr Val Thr Ala Ile Thr Val Tyr Ala Glu Leu
275 280 285

Pro Lys Pro Tyr Ile Thr Ser Asn Asn Ser Asn Pro Ile Glu Asp Lys
290 295 300

Asp Ala Val Thr Leu Thr Cys Glu Pro Glu Thr Gln Asp Thr Thr Tyr
305 310 315 320

Leu Trp Trp Val Asn Asn Gln Arg Leu Ser Val Ser Ser Arg Leu Glu
325 330 335

Leu Ser Asn Asp Asn Arg Thr Leu Thr Val Phe Asn Ile Pro Arg Asn
340 345 350

Asp Thr Thr Phe Tyr Glu Cys Glu Thr Gln Asn Pro Val Ser Val Arg
355 360 365

Arg Ser Asp Pro Val Thr Leu Asn Val Leu Tyr Gly Pro Asp Ala Pro
370 375 380

Thr Ile Ser Pro Leu Asn Thr Pro Tyr Arg Ala Gly Glu Asn Leu Asn
385 390 395 400

Leu Ser Cys His Ala Ala Ser Asn Pro Ala Ala Gln Tyr Phe Trp Phe
405 410 415

Val Asn Gly Thr Phe Gln Gln Ser Thr Gln Glu Leu Phe Ile Pro Asn
420 425 430

Ile Thr Val Asn Asn Ser Gly Ser Tyr Met Cys Gln Ala His Asn Ser
435 440 445

Ala Thr Gly Leu Asn Arg Thr Thr Val Thr Ala Ile Thr Val Tyr Val
    450                 455                 460

Glu Leu Pro Lys Pro Tyr Ile Ser Ser Asn Asn Ser Asn Pro Ile Glu
465                 470                 475                 480

Asp Lys Asp Ala Val Thr Leu Thr Cys Glu Pro Val Ala Glu Asn Thr
                485                 490                 495

Thr Tyr Leu Trp Trp Val Asn Asn Gln Ser Leu Ser Val Ser Pro Arg
            500                 505                 510

Leu Gln Leu Ser Asn Gly Asn Arg Ile Leu Thr Leu Leu Ser Val Thr
        515                 520                 525

Arg Asn Asp Thr Gly Pro Tyr Glu Cys Gly Ile Gln Asn Ser Glu Ser
    530                 535                 540

Ala Lys Arg Ser Asp Pro Val Thr Leu Asn Val Thr Tyr Gly Pro Asp
545                 550                 555                 560

Thr Pro Ile Ile Ser Pro Pro Asp Leu Ser Tyr Arg Ser Gly Ala Asn
                565                 570                 575

Leu Asn Leu Ser Cys His Ser Asp Ser Asn Pro Ser Pro Gln Tyr Ser
            580                 585                 590

Trp Leu Ile Asn Gly Thr Leu Arg Gln His Thr Gln Val Leu Phe Ile
        595                 600                 605

Ser Lys Ile Thr Ser Asn Asn Asn Gly Ala Tyr Ala Cys Phe Val Ser
    610                 615                 620

Asn Leu Ala Thr Gly Arg Asn Asn Ser Ile Val Lys Asn Ile Ser Val
625                 630                 635                 640

Ser Ser Gly Asp Ser Ala Pro Gly Ser Ser Gly Leu Ser Ala
                645                 650

<210> 13
<211> 702
<212> PRT
<213> Artificial Sequence

<220>
<223> human CEACAM5

<400> 13

Met Glu Ser Pro Ser Ala Pro Pro His Arg Trp Cys Ile Pro Trp Gln

1 5 10 15

Arg Leu Leu Leu Thr Ala Ser Leu Leu Thr Phe Trp Asn Pro Pro Thr
20 25 30

Thr Ala Lys Leu Thr Ile Glu Ser Thr Pro Phe Asn Val Ala Glu Gly
35 40 45

Lys Glu Val Leu Leu Leu Val His Asn Leu Pro Gln His Leu Phe Gly
50 55 60

Tyr Ser Trp Tyr Lys Gly Glu Arg Val Asp Gly Asn Arg Gln Ile Ile
65 70 75 80

Gly Tyr Val Ile Gly Thr Gln Gln Ala Thr Pro Gly Pro Ala Tyr Ser
85 90 95

Gly Arg Glu Ile Ile Tyr Pro Asn Ala Ser Leu Leu Ile Gln Asn Ile
100 105 110

Ile Gln Asn Asp Thr Gly Phe Tyr Thr Leu His Val Ile Lys Ser Asp
115 120 125

Leu Val Asn Glu Glu Ala Thr Gly Gln Phe Arg Val Tyr Pro Glu Leu
130 135 140

Pro Lys Pro Ser Ile Ser Ser Asn Asn Ser Lys Pro Val Glu Asp Lys
145 150 155 160

Asp Ala Val Ala Phe Thr Cys Glu Pro Glu Thr Gln Asp Ala Thr Tyr
165 170 175

Leu Trp Trp Val Asn Asn Gln Ser Leu Pro Val Ser Pro Arg Leu Gln
180 185 190

Leu Ser Asn Gly Asn Arg Thr Leu Thr Leu Phe Asn Val Thr Arg Asn
195 200 205

Asp Thr Ala Ser Tyr Lys Cys Glu Thr Gln Asn Pro Val Ser Ala Arg
210 215 220

Arg Ser Asp Ser Val Ile Leu Asn Val Leu Tyr Gly Pro Asp Ala Pro
225 230 235 240

Thr Ile Ser Pro Leu Asn Thr Ser Tyr Arg Ser Gly Glu Asn Leu Asn
245 250 255

Leu Ser Cys His Ala Ala Ser Asn Pro Pro Ala Gln Tyr Ser Trp Phe
260 265 270

Val Asn Gly Thr Phe Gln Gln Ser Thr Gln Glu Leu Phe Ile Pro Asn
275 280 285

Ile Thr Val Asn Asn Ser Gly Ser Tyr Thr Cys Gln Ala His Asn Ser
290 295 300

Asp Thr Gly Leu Asn Arg Thr Thr Val Thr Thr Ile Thr Val Tyr Ala
305 310 315 320

Glu Pro Pro Lys Pro Phe Ile Thr Ser Asn Asn Ser Asn Pro Val Glu
325 330 335

Asp Glu Asp Ala Val Ala Leu Thr Cys Glu Pro Glu Ile Gln Asn Thr
340 345 350

Thr Tyr Leu Trp Trp Val Asn Asn Gln Ser Leu Pro Val Ser Pro Arg
355 360 365

Leu Gln Leu Ser Asn Asp Asn Arg Thr Leu Thr Leu Leu Ser Val Thr
370 375 380

Arg Asn Asp Val Gly Pro Tyr Glu Cys Gly Ile Gln Asn Glu Leu Ser
385 390 395 400

Val Asp His Ser Asp Pro Val Ile Leu Asn Val Leu Tyr Gly Pro Asp
405 410 415

Asp Pro Thr Ile Ser Pro Ser Tyr Thr Tyr Tyr Arg Pro Gly Val Asn
420 425 430

Leu Ser Leu Ser Cys His Ala Ala Ser Asn Pro Pro Ala Gln Tyr Ser
435 440 445

Trp Leu Ile Asp Gly Asn Ile Gln Gln His Thr Gln Glu Leu Phe Ile
450 455 460

Ser Asn Ile Thr Glu Lys Asn Ser Gly Leu Tyr Thr Cys Gln Ala Asn
465 470 475 480

Asn Ser Ala Ser Gly His Ser Arg Thr Thr Val Lys Thr Ile Thr Val
485 490 495

Ser Ala Glu Leu Pro Lys Pro Ser Ile Ser Ser Asn Asn Ser Lys Pro
500 505 510

Val Glu Asp Lys Asp Ala Val Ala Phe Thr Cys Glu Pro Glu Ala Gln
        515                 520                 525

Asn Thr Thr Tyr Leu Trp Trp Val Asn Gly Gln Ser Leu Pro Val Ser
    530                 535                 540

Pro Arg Leu Gln Leu Ser Asn Gly Asn Arg Thr Leu Thr Leu Phe Asn
545                 550                 555                 560

Val Thr Arg Asn Asp Ala Arg Ala Tyr Val Cys Gly Ile Gln Asn Ser
                565                 570                 575

Val Ser Ala Asn Arg Ser Asp Pro Val Thr Leu Asp Val Leu Tyr Gly
            580                 585                 590

Pro Asp Thr Pro Ile Ile Ser Pro Pro Asp Ser Ser Tyr Leu Ser Gly
        595                 600                 605

Ala Asn Leu Asn Leu Ser Cys His Ser Ala Ser Asn Pro Ser Pro Gln
    610                 615                 620

Tyr Ser Trp Arg Ile Asn Gly Ile Pro Gln Gln His Thr Gln Val Leu
625                 630                 635                 640

Phe Ile Ala Lys Ile Thr Pro Asn Asn Asn Gly Thr Tyr Ala Cys Phe
                645                 650                 655

Val Ser Asn Leu Ala Thr Gly Arg Asn Asn Ser Ile Val Lys Ser Ile
            660                 665                 670

Thr Val Ser Ala Ser Gly Thr Ser Pro Gly Leu Ser Ala Gly Ala Thr
        675                 680                 685

Val Gly Ile Met Ile Gly Val Leu Val Gly Val Ala Leu Ile
    690                 695                 700

<210> 14
<211> 120
<212> PRT
<213> Artificial Sequence

<220>
<223> MAb1 variable domain of heavy chain

<400> 14

Glu Val Met Leu Val Glu Ser Gly Gly Gly Leu Val Lys Pro Gly Gly
1               5                   10                  15

Ser Leu Lys Leu Ser Cys Ala Ala Ser Gly Phe Thr Phe Ser Ser Tyr
20 25 30

Ala Met Ser Trp Val Arg Gln Thr Pro Glu Lys Arg Leu Glu Trp Val
35 40 45

Ala Thr Ile Ser Ser Gly Gly Ser Tyr Ile Tyr Tyr Leu Asp Ser Val
50 55 60

Lys Gly Arg Phe Thr Ile Ser Arg Asp Asn Ala Lys Asn Thr Leu Tyr
65 70 75 80

Leu Gln Met Ser Ser Leu Arg Ser Glu Asp Thr Ala Met Tyr Tyr Cys
85 90 95

Ala Arg Pro Ala Tyr Tyr Gly Asn Pro Ala Met Asp Tyr Trp Gly Gln
100 105 110

Gly Thr Ser Val Thr Val Ser Ser
115 120

<210> 15
<211> 108
<212> PRT
<213> Artificial Sequence

<220>
<223> MAb1 variable domain of light chain

<400> 15

Asp Ile Leu Met Thr Gln Ser Gln Lys Phe Met Ser Thr Ser Val Gly
1 5 10 15

Asp Arg Val Ser Val Thr Cys Lys Ala Ser Gln Asn Val Gly Thr Asn
20 25 30

Val Ala Trp Tyr Gln Gln Lys Pro Gly Gln Ser Pro Lys Pro Leu Ile
35 40 45

Tyr Ser Ala Ser Tyr Arg Tyr Ser Gly Val Pro Asp Arg Phe Thr Gly
50 55 60

Ser Gly Ser Gly Thr Asp Phe Thr Leu Thr Ile Ser Asn Val Gln Ser
65 70 75 80

Glu Asp Leu Ala Glu Tyr Phe Cys Gln Gln Tyr Asn Ser Tyr Pro Leu
85 90 95

Tyr Thr Phe Gly Gly Gly Thr Lys Leu Glu Ile Lys

100 105

<210> 16
<211> 120
<212> PRT
<213> Artificial Sequence

<220>
<223> MAb2 variable domain of heavy chain

<400> 16

Glu Val Gln Leu Gln Glu Ser Gly Gly Val Leu Val Lys Pro Gly Gly
1 5 10 15

Ser Leu Lys Leu Ser Cys Ala Ala Ser Gly Phe Val Phe Ser Ser Tyr
20 25 30

Asp Met Ser Trp Val Arg Gln Thr Pro Glu Lys Arg Leu Glu Trp Val
35 40 45

Ala Tyr Ile Ser Ser Gly Gly Gly Ile Thr Tyr Phe Pro Asp Thr Val
50 55 60

Gln Gly Arg Phe Thr Val Ser Arg Asp Asn Ala Lys Asn Thr Leu Tyr
65 70 75 80

Leu Gln Met Asn Ser Leu Lys Ser Glu Asp Thr Ala Ile Tyr Tyr Cys
85 90 95

Ala Ala His Tyr Phe Gly Ser Ser Gly Pro Phe Ala Tyr Trp Gly Gln
100 105 110

Gly Thr Leu Val Thr Val Ser Ala
115 120

<210> 17
<211> 107
<212> PRT
<213> Artificial Sequence

<220>
<223> MAb2 variable domain of light chain

<400> 17

Asp Ile Gln Met Thr Gln Ser Pro Ala Ser Leu Ser Ala Ser Val Gly
1 5 10 15

Glu Thr Val Thr Ile Thr Cys Arg Ala Ser Glu Asn Ile Phe Ser Tyr
20 25 30

```
Leu Ala Trp Tyr Gln Gln Lys Gln Gly Lys Ser Pro Gln Leu Leu Val
        35                  40                  45

Tyr Asn Thr Lys Thr Leu Ala Glu Gly Val Pro Ser Arg Phe Ser Gly
    50                  55                  60

Ser Gly Ser Gly Thr Gln Phe Ser Leu Lys Ile Asn Ser Leu Gln Pro
65                  70                  75                  80

Glu Asp Phe Gly Ser Tyr Tyr Cys Gln His His Tyr Gly Thr Pro Phe
                85                  90                  95

Thr Phe Gly Ser Gly Thr Lys Leu Glu Ile Lys
            100                 105


<210>  18
<211>  119
<212>  PRT
<213>  Artificial Sequence

<220>
<223>  MAb3 variable domain of heavy chain

<400>  18

Glu Val Lys Leu Val Glu Ser Gly Gly Gly Leu Val Lys Pro Gly Gly
1               5                   10                  15

Ser Leu Thr Leu Pro Cys Ala Ala Ser Gly Phe Thr Phe Ser Arg Tyr
            20                  25                  30

Ala Met Ser Trp Val Arg Gln Thr Pro Glu Lys Arg Leu Glu Trp Val
        35                  40                  45

Ala Ser Ile Ser Ser Gly Gly Asp Thr Tyr Tyr Pro Asp Ser Val Lys
    50                  55                  60

Gly Arg Phe Thr Val Ser Arg Asp Asn Ala Arg Asn Ile Leu Phe Leu
65                  70                  75                  80

Gln Met Ser Ser Leu Arg Ser Glu Asp Thr Gly Met Tyr Tyr Cys Ala
                85                  90                  95

Arg Val Asn Tyr Tyr Asp Ser Ser Phe Leu Asp Trp Trp Gly Gln Gly
            100                 105                 110

Thr Thr Leu Thr Val Ser Ser
        115


<210>  19
```

<211> 108
<212> PRT
<213> Artificial Sequence

<220>
<223> MAb3 variable domain of light chain

<400> 19

Asp Ile Val Met Thr Gln Ser Gln Arg Phe Met Ser Thr Leu Glu Gly
1 5 10 15

Asp Arg Val Ser Val Thr Cys Lys Ala Ser Gln Asn Val Gly Thr Asn
20 25 30

Val Ala Trp Tyr Gln Gln Lys Pro Gly Gln Ser Pro Lys Ala Leu Ile
35 40 45

Tyr Ser Ala Ser Tyr Arg Tyr Ser Gly Val Pro Asp Arg Phe Thr Gly
50 55 60

Ser Gly Ser Gly Thr Asp Phe Thr Leu Thr Ile Ser Asn Val Gln Ser
65 70 75 80

Glu Asp Leu Ala Glu Tyr Phe Cys Gln Gln Tyr Asn Asn Tyr Pro Leu
85 90 95

Tyr Thr Phe Gly Gly Gly Thr Lys Leu Glu Ile Lys
100 105

<210> 20
<211> 120
<212> PRT
<213> Artificial Sequence

<220>
<223> MAb4 variable domain of heavy chain

<400> 20

Glu Val Gln Leu Val Glu Ser Gly Gly Gly Leu Val Lys Pro Gly Gly
1 5 10 15

Ser Leu Lys Leu Ser Cys Ala Ala Ser Gly Phe Thr Phe Ser Ser Tyr
20 25 30

Asp Met Ser Trp Val Arg Gln Thr Pro Glu Lys Arg Leu Glu Trp Val
35 40 45

Ala Phe Ile Ser Ser Tyr Gly Gly Arg Thr Tyr Tyr Ala Asp Thr Val
50 55 60

Lys Gly Arg Phe Thr Ile Ser Arg Asp Asn Ala Lys Asn Thr Leu Tyr
65 70 75 80

Leu Gln Met Ser Ser Leu Lys Ser Glu Asp Thr Ala Met Phe Tyr Cys
85 90 95

Ala Ala His Tyr Phe Gly Thr Ser Gly Pro Phe Ala Tyr Trp Gly Gln
100 105 110

Gly Thr Leu Val Thr Val Ser Ala
115 120

<210> 21
<211> 107
<212> PRT
<213> Artificial Sequence

<220>
<223> MAb4 variable domain of light chain

<400> 21

Asp Ile Gln Met Thr Gln Ser Pro Ala Ser Leu Ser Ala Ser Val Gly
1 5 10 15

Glu Thr Val Thr Ile Thr Cys Arg Ala Ser Glu Asn Ile Tyr Ser Tyr
20 25 30

Phe Ala Trp Tyr Gln Gln Lys Gln Gly Lys Ser Pro Gln Leu Leu Val
35 40 45

Tyr Asn Ala Lys Ile Leu Ala Glu Gly Val Pro Ser Arg Phe Ser Gly
50 55 60

Ser Gly Ser Gly Thr Gln Phe Ser Leu Lys Ile Asn Ser Leu Gln Pro
65 70 75 80

Glu Asp Phe Gly Thr Tyr Tyr Cys Gln His His Tyr Gly Ile Pro Phe
85 90 95

Thr Phe Gly Ser Gly Thr Lys Leu Glu Leu Lys
100 105

<210> 22
<211> 120
<212> PRT
<213> Artificial Sequence

<220>
<223> MAb5 variable domain of heavy chain

<400> 22

```
Glu Leu Gln Leu Val Glu Ser Gly Gly Val Leu Val Lys Pro Gly Gly
1               5                   10                  15

Ser Leu Lys Leu Ser Cys Ala Ala Ser Gly Phe Ala Phe Ser Ser Tyr
            20                  25                  30

Asp Met Ser Trp Val Arg Gln Thr Pro Glu Lys Arg Leu Glu Trp Val
        35                  40                  45

Thr Tyr Ile Asn Ser Gly Gly Gly Ile Thr Tyr Tyr Pro Asp Thr Val
    50                  55                  60

Lys Gly Arg Phe Thr Ile Ser Arg Asp Asn Ala Arg Asn Thr Leu Tyr
65                  70                  75                  80

Leu Gln Met Ser Ser Leu Lys Ser Glu Asp Thr Ala Ile Tyr Tyr Cys
                85                  90                  95

Thr Ala His Tyr Phe Gly Ser Ser Gly Pro Phe Ala Tyr Trp Gly Gln
            100                 105                 110

Gly Thr Leu Val Thr Val Ser Ala
        115                 120


<210>  23
<211>  107
<212>  PRT
<213>  Artificial Sequence

<220>
<223>  MAb5 variable domain of light chain

<400>  23

Asp Ile Gln Met Thr Gln Ser Pro Ala Ser Leu Ser Ala Ser Val Gly
1               5                   10                  15

Glu Thr Val Thr Ile Thr Cys Arg Ala Ser Glu Asn Ile Tyr Ser Tyr
            20                  25                  30

Leu Ala Trp Tyr Gln Gln Lys Gln Gly Lys Ser Pro Gln Leu Leu Val
        35                  40                  45

Tyr Asn Ala Lys Thr Leu Thr Glu Gly Val Pro Ser Arg Phe Ser Gly
    50                  55                  60

Ser Gly Ser Gly Thr Gln Phe Ser Leu Lys Ile Asn Ser Leu Gln Pro
65                  70                  75                  80
```

```
Glu Asp Phe Gly Ser Tyr Tyr Cys Gln His His Tyr Gly Thr Pro Phe
                85                  90                  95

Thr Phe Gly Ser Gly Thr Lys Leu Glu Ile Lys
            100                 105
```